(19) Europäisches Patentamt
European Patent Office
Office européen des brevets

(11) EP 1 392 823 B1

(12) **EUROPEAN PATENT SPECIFICATION**

(45) Date of publication and mention of the grant of the patent:
**30.11.2011 Bulletin 2011/48**

(21) Application number: **02736480.1**

(22) Date of filing: **24.01.2002**

(51) Int Cl.:
*C12N 9/02* (2006.01)

(86) International application number:
**PCT/US2002/001924**

(87) International publication number:
**WO 2002/081668 (17.10.2002 Gazette 2002/42)**

(54) **DESATURASE GENES AND USES THEREOF**

MODIFIZIERTES INSULIN MIT REDUZIERTER IMMUNOGENITÄT

GENES DESATURASE ET UTILISATIONS DE CES DERNIERS

(84) Designated Contracting States:
**AT BE CH CY DE DK ES FI FR GB GR IE IT LI LU MC NL PT SE TR**

(30) Priority: **25.01.2001 US 769863**
**22.01.2002 US 54534**

(43) Date of publication of application:
**03.03.2004 Bulletin 2004/10**

(60) Divisional application:
**10184220.1 / 2 325 300**
**10185334.9 / 2 333 052**

(73) Proprietor: **ABBOTT LABORATORIES**
**Abbott Park IL 60064-3500 (US)**

(72) Inventors:
- **MUKERJI, Pradip**
  **Gahanna, OH 43230 (US)**
- **HUANG, Yung-Sheng**
  **Columbus, OH 43220 (US)**
- **DAS, Tapas**
  **Worthington, OH 43085 (US)**
- **THURMOND, Jennifer**
  **Columbus, OH 43231 (US)**
- **PEREIRA, Suzette, L.**
  **Westerville, OH 43081 (US)**

(74) Representative: **Modiano, Micaela Nadia**
**Modiano Josif Pisanty & Staub Ltd**
**Thierschstrasse 11**
**80538 München (DE)**

(56) References cited:
**EP-A- 1 035 207** **WO-A-00/20603**
**WO-A-00/75341** **WO-A-02/26946**
**WO-A-93/06712** **WO-A-99/61602**
**US-A- 5 972 664**

- **QIU XIAO ET AL: "Identification of a DELTA4 fatty acid desaturase from Thraustochytrium sp. involved in the biosynthesis of docosahexanoic acid by heterologous expression in Saccharomyces cerevisiae and Brassica juncea" JOURNAL OF BIOLOGICAL CHEMISTRY, AMERICAN SOCIETY OF BIOLOGICAL CHEMISTS, BALTIMORE, MD, US, vol. 276, no. 34, 24 August 2001 (2001-08-24), pages 31561-31566, XP002207404 ISSN: 0021-9258**
- **SAITO M. ET AL.: "Identification delta-5 fatty acid desaturase from the cellular slime mold Dictyostelium discoideum" EUR. J. BIOCHEM., vol. 265, 1999, pages 809-814, XP002215761**
- **LEONARD A E ET AL: "cDNA cloning and characterization of human DELTA5-desaturase involved in the biosynthesis of arachidonic acid" BIOCHEMICAL JOURNAL, THE BIOCHEMICAL SOCIETY, LONDON, GB, vol. 347, no. 3, 1 May 2000 (2000-05-01), pages 719-724, XP002223219 ISSN: 0264-6021**
- **CHO H P ET AL: "Cloning, Expression, and Fatty Acid Regulation of the Human Delta-5 Desaturase" JOURNAL OF BIOLOGICAL CHEMISTRY, THE AMERICAN SOCIETY OF BIOLOGICAL CHEMISTS, INC.,, US, vol. 274, no. 52, 24 December 1999 (1999-12-24), pages 37335-37339, XP002140847 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SAKURADANI E ET AL: "DELTA6-FATTY ACID DESATURASE FROM AN ARACHIDONIC ACID-PRODUCING MORTIERELLA FUNGUS GENE CLONING AND ITS HETEROLOGOUS EXPRESSION IN A FUNGUS, ASPERGILLUS" GENE: AN INTERNATIONAL JOURNAL ON GENES AND GENOMES, ELSEVIER SCIENCE PUBLISHERS, BARKING, GB, vol. 238, no. 2, 1999, pages 445-453, XP002929099 ISSN: 0378-1119**
- **HUANG Y S ET AL: "CLONING OF 12- AND 6-DESATURASES FROM MORTIERELLA ALPINA AND RECOMBINANT PRODUCTION OF GAMMA-LINOLENIC ACID IN SACCHAROMYCES CEREVISIAE" LIPIDS, CHAMPAIGN, IL, US, vol. 34, no. 7, July 1999 (1999-07), pages 649-659, XP000908862 ISSN: 0024-4201**
- **LOPEZ ALONSO D ET AL: "Plants as 'chemical factories' for the production of polyunsaturated fatty acids" BIOTECHNOLOGY ADVANCES, ELSEVIER PUBLISHING, BARKING, GB, vol. 18, no. 6, October 2000 (2000-10), pages 481-497, XP002208312 ISSN: 0734-9750**

SEQUENCE LISTING

<110> Abbott Laboratories
Mukerji, Pradip
Huang, Yung-Sheng
Das, Tapas
Thurmond, Jennifer M.
Pereira, Suzette L.

<120> DESATURASE GENES AND USES THEREOF

<130> 6763.PC.O1

<140> Not Yet Assigned
<141> 2002-01-23

<150> US Not Yet Assigned
<151> 2002-01-22

<150> US 09/769,863
<151> 2001-01-25

<160> 55

<170> FastSEQ for Windows Version 4.0

<210> 1
<211> 42
<212> DNA

<213> Artificial Sequence

<220>
<223> Primer R0834

<221> misc_feature
<222> (3)...(3)
<223> b = g or c or t/u at position 3

<221> misc_feature
<222> (6)...(6)
<223> y = t/u or c at position 6

<221> misc_feature
<222> (9)...(9)
<223> y = t/u or c at position 9

<221> misc_feature
<222> (12)... (12)
<223> b = g or c or t/u at position 12

<221> misc_difference
<222> (18)...(18)
<223> r = g or a at position 18

<221> misc_feature
<222> (24)...(24)
<223> b = g or c or t/u at position 24

<221> misc_feature
<222> (30)...(30)
<223> b = g or c or t/u at position 30

<221> misc_feature
<222> (33)...(33)
<223> y = t/u or c at position 33

<221> misc_feature
<222> (36)...(36)
<223> y = t/u or c at position 36

<221> misc_feature
<222> (39)...(39)
<223> h = a or c or t/u at position 39

<221> misc_feature
<222> (42)...(42)
<223> h = a or c or t/u at position 42

<400> 1
gtbtaygayg tbaccgartg ggtbaagcgy cayccbgghg gh 42

<210> 2
<211> 45
<212> DNA
<213> Artificial Sequence

<220>

<223> Forward Primer R0835

<221> misc_feature
<222> (3)...(3)
<223> h = a or c or t/u at position 3

<221> misc_feature
<222> (6)...(6)
<223> y = t/u or c at position 6

<221> misc_feature
<222> (12)...(12)
<223> y = t/u or c at position 12

<221> misc_feature
<222> (27)...(27)
<223> y = t/u or c at position 27

<221> misc_feature
<222> (33)...(33)
<223> y = tu or c at position 33

<221> misc_feature
<222> (39)...(39)
<223> b = g or c or t/u at position 39

<221> misc_feature
<222> (41)...(41)
<223> y = t/u or c at position 41

<221> misc_feature
<222> (45)...(45)
<223> y = t/u or c at position 45

<400> 2
gghgcytccg cyaactggtg gaagcaycag cayaacgtbc aycay 45

<210> 3
<211> 45
<212> DNA
<213> Artificial Sequence

<220>
<223> Reverse Primer RO836

<221> misc_feature
<222> (1)...(1)
<223> r = g or a at position 1

<221> misc_feature
<222> (4)...(4)
<223> r = g or a at position 4

<221> misc_feature
<222> (7)...(7)
<223> v = a or g or c at position 7

<221> misc_feature

<222> (13)...(13)
<223> r = g or a at position 13

<221> misc_feature
<222> (19)...(19)
<223> r = g or a at position 19

<221> misc_feature
<222> (34)...(34)
<223> r = g or a at position 34

<221> misc_feature
<222> (40)...(40)
<223> r = g or a at position 40

<221> misc_feature
<222> (43)...(43)
<223> d = a or g or t/u at position 43

<400> 3
rtgrtgvacg ttrtgctgrt gcttccacca gttrgcggar gcdcc 45

<210> 4
<211> 36
<212> DNA
<213> Artificial Sequence

<220>
<223> Reverse Primer R0838

<221> misc_feature
<222> (6)...(6)
<223> r = g or a at position 6

<221> misc_feature
<222> (12)...(12)
<223> r = g or a at position 12

<221> misc_feature
<222> (15)...(15)
<223> y = t/u or c at position 15

<221> misc_feature
<222> (18)...(18)
<223> r = g or a at position 18

<221> misc_feature
<222> (21)...(21)
<223> r = g or a at position 21

<221> misc_feature
<222> (24)...(24)
<223> s = g or c at position 24

<221> misc_feature
<222> (27)...(27)
<223> r = g or a at position 27

<221> misc_feature
<222> (30)...(30)
<223> v = a or g or c at position 30

<400> 4
ttgatrgtct arctygtrgt rgasaarggv tggtac 36

<210> 5
<211> 24
<212> DNA
<213> Artificial Sequence

<220>
<223> Primer R0753

<221> misc_feature
<222> (10)...(10)
<223> n = a or g or c or t/u, unknown, or other at position 10

<221> misc_feature
<222> (13)...(13)
<223> r = g or a at position 13

<221> misc_feature
<222> (16)...(16)
<223> n = a or g or c or t/u, unknown, or other at position 16

<221> misc_feature
<222> (18)...(19)
<223> r = g or a at positions 18-19

<221> misc_feature
<222> (22)...(22)
<223> r = g or a at position 22

<400> 5
catcatcatn ggraanarrt grtg 24

<210> 6
<211> 30
<212> DNA
<213> Artificial Sequence

<220>
<223> Primer R0754

<221> misc_feature
<222> (15)...(15)
<223> y = t/u or c at position 15

<221> misc_feature
<222> (18)...(18)
<223> y = t/u or c at position 19

<221> misc_feature
<222> (21)...(21)
<223> n = a or g or c or t/u, unknown, or other at position 21

<221> misc_feature
<222> (24)...(24)
<223> y = t/u or c at position 24

<221> misc_feature
<222> (27)...(27)
<223> n = a or g or c or t/u, unknown, or other at position 27

<221> misc_feature
<222> (30)...(30)
<223> y = t/u or c at position 30

<400> 6
ctactactac tacaycayac ntayacnaay 30

<210> 7
<211> 29
<212> DNA
<213> Artificial Sequence

<220>
<223> Primer R0923

<400> 7
cggtgcagtg gtggaagaac aagcacaac 29

<210> 8
<211> 30
<212> DNA
<213> Artificial Sequence

<220>
<223> Primer RO899

<400> 8
agcggataac aatttcacac aggaaacagc 30

<210> 9
<211> 30
<212> DNA
<213> Artificial Sequence

<220>

<223> Primer R0939

<400> 9

cgtagtactg ctcgaggagc ttgagcgccg 30

<210> 10
<211> 31
<212> DNA
<213> Artificial Sequence

<220>
<223> Primer R0898

<400> 10

cccagtcacg acgttgtaaa acgacggcca g 31

<210> 11
<211> 45
<212> DNA
<213> Artificial Sequence

<220>
<223> Primer RO951

<400> 11
tcaacagaat tcatggtcca ggggcaaaag gccgagaaga tctcg 45

<210> 12
<211> 47
<212> DNA
<213> Artificial Sequence

<220>
<223> Primer R0960

<400> 12
atacgtaagc ttttacatgg cgggaaactc cttgaagaac tcgatcg 47

<210> 13
<211> 1362
<212> DNA
<213> Saprolegnia diclina

<400> 13

```
atggtccagg ggcaaaaggc cgagaagatc tcgtgggcga ccatccgtga gcacaaccgc 60
caagacaacg cgtggatcgt gatccaccac aaggtgtacg acatctcggc ctttgaggac 120
cacccgggcg gcgtcgtcat gttcacgcag gccggcgaag acgcgaccga tgcgttcgct 180
gtcttccacc cgagctcggc gctcaagctc ctcgagcagt actacgtcgg cgacgtcgac 240
cagtcgacgg cggccgtcga cacgtcgatc tcggacgagg tcaagaagag ccagtcggac 300
ttcattgcgt cgtaccgcaa gctgcgcctt gaagtcaagc gcctcggctt gtacgactcg 360
agcaagctct actacctcta caagtgcgcc tcgacgctga gcattgcgct tgtgtcggcg 420
gccatttgcc tccactttga ctcgacggcc atgtacatgg tcgcggctgt catccttggc 480
ctcttttacc agcagtgcgg ctggctcgcc catgactttc tgcaccacca agtgtttgag 540
aaccacttgt ttggcgacct cgtcggcgtc atggtcggca acctctggca gggcttctcg 600
gtgcagtggt ggaagaacaa gcacaacacg caccatgcga tccccaacct ccacgcgacg 660
cccgagatcg ccttccacgg cgacccggac attgacacga tgccgattct cgcgtggtcg 720
ctcaagatgg cgcagcacgc ggtcgactcg cccgtcgggc tcttcttcat gcgctaccaa 780
gcgtacctgt actttcccat cttgctcttt gcgcgtatct cgtgggtgat ccagtcggcc 840
atgtacgcct tctacaacgt tgggcccggc ggcacctttg acaaggtcca gtacccgctg 900
ctcgagcgcg ccggcctcct cctctactac ggctggaacc tcggccttgt gtacgcagcc 960
aacatgtcgc tgctccaagc ggctgcgttc ctctttgtga gccaggcgtc gtgcggcctc 1020
ttcctcgcga tggtctttag cgtcggccac aacggcatgg aggtctttga caaggacagc 1080
aagcccgatt tttggaagct gcaagtgctc tcgacgcgca acgtgacgtc gtcgctctgg 1140
atcgactggt tcatgggcgg cctcaactac cagatcgacc accacttgtt cccgatggtg 1200
ccccggcaca acctcccggc gctcaacgtg ctcgtcaagt cgctctgcaa gcagtacgac 1260
atcccatacc acgagacggg cttcatcgcg ggcatggccg aggtcgtcgt gcacctcgag 1320
cgcatctcga tcgagttctt caaggagttt cccgccatgt aa                   1362
```

<210> 14

<211> 453
<212> PRT
<213> Saprolegnia diclina

<400> 14

```
Met Val Gln Gly Gln Lys Ala Glu Lys Ile Ser Trp Ala Thr Ile Arg
 1               5                  10                  15
Glu His Asn Arg Gln Asp Asn Ala Trp Ile Val Ile His His Lys Val
            20                  25                  30
Tyr Asp Ile Ser Ala Phe Glu Asp His Pro Gly Gly Val Val Met Phe
        35                  40                  45
Thr Gln Ala Gly Glu Asp Ala Thr Asp Ala Phe Ala Val Phe His Pro
    50                  55                  60
Ser Ser Ala Leu Lys Leu Leu Glu Gln Tyr Tyr Val Gly Asp Val Asp
65                  70                  75                  80
Gln Ser Thr Ala Ala Val Asp Thr Ser Ile Ser Asp Glu Val Lys Lys
                85                  90                  95
Ser Gln Ser Asp Phe Ile Ala Ser Tyr Arg Lys Leu Arg Leu Glu Val
            100                 105                 110
Lys Arg Leu Gly Leu Tyr Asp Ser Ser Lys Leu Tyr Tyr Leu Tyr Lys
```

```
        115                 120                 125
Cys Ala Ser Thr Leu Ser Ile Ala Leu Val Ser Ala Ala Ile Cys Leu
    130                 135                 140
His Phe Asp Ser Thr Ala Met Tyr Met Val Ala Ala Val Ile Leu Gly
145                 150                 155                 160
Leu Phe Tyr Gln Gln Cys Gly Trp Leu Ala His Asp Phe Leu His His
                165                 170                 175
Gln Val Phe Glu Asn His Leu Phe Gly Asp Leu Val Gly Val Met Val
            180                 185                 190
Gly Asn Leu Trp Gln Gly Phe Ser Val Gln Trp Trp Lys Asn Lys His
        195                 200                 205
Asn Thr His His Ala Ile Pro Asn Leu His Ala Thr Pro Glu Ile Ala
    210                 215                 220
Phe His Gly Asp Pro Asp Ile Asp Thr Met Pro Ile Leu Ala Trp Ser
225                 230                 235                 240
Leu Lys Met Ala Gln His Ala Val Asp Ser Pro Val Gly Leu Phe Phe
                245                 250                 255
Met Arg Tyr Gln Ala Tyr Leu Tyr Phe Pro Ile Leu Leu Phe Ala Arg
            260                 265                 270
Ile Ser Trp Val Ile Gln Ser Ala Met Tyr Ala Phe Tyr Asn Val Gly
        275                 280                 285
Pro Gly Gly Thr Phe Asp Lys Val Gln Tyr Pro Leu Leu Glu Arg Ala
    290                 295                 300
Gly Leu Leu Leu Tyr Tyr Gly Trp Asn Leu Gly Leu Val Tyr Ala Ala
305                 310                 315                 320
Asn Met Ser Leu Leu Gln Ala Ala Ala Phe Leu Phe Val Ser Gln Ala
                325                 330                 335
Ser Cys Gly Leu Phe Leu Ala Met Val Phe Ser Val Gly His Asn Gly
            340                 345                 350
Met Glu Val Phe Asp Lys Asp Ser Lys Pro Asp Phe Trp Lys Leu Gln
        355                 360                 365
Val Leu Ser Thr Arg Asn Val Thr Ser Ser Leu Trp Ile Asp Trp Phe
    370                 375                 380
Met Gly Gly Leu Asn Tyr Gln Ile Asp His His Leu Phe Pro Met Val
385                 390                 395                 400
Pro Arg His Asn Leu Pro Ala Leu Asn Val Leu Val Lys Ser Leu Cys
                405                 410                 415
Lys Gln Tyr Asp Ile Pro Tyr His Glu Thr Gly Phe Ile Ala Gly Met
            420                 425                 430
Ala Glu Val Val Val His Leu Glu Arg Ile Ser Ile Glu Phe Phe Lys
        435                 440                 445
Glu Phe Pro Ala Met
    450
```

<210> 15
<211> 23
<212> DNA
<213> Artificial Sequence

<220>
<223> Primer R0851

<400> 15
ccatcaagac gtaccttgcg atc 23

<210> 16
<211> 28
<212> DNA
<213> Artificial Sequence

<220>
<223> Primer RO941

<400> 16
gctgaacggg tggtacgagt cgaacgtg 28

<210> 17
<211> 42
<2'12> DNA
<213> Artificial Sequence

<220>
<223> Primer R0953

<400> 17
acgagagaat tcatggcccc gcagacggag ctccgccagc gc 42

<210> 18
<211> 46
<212> DNA
<213> Artificial Sequence

<220>
<223> Primer R0956

<400> 18
aaaagactcg agttagccca tgtggatcgt ggcggcgatg ccctgc 46

<210> 19
<211> 1413
<212> DNA
<213> Saprolegnia diclina

<400> 19

```
atggccccgc agacggagct ccgccagcgc cacgccgccg tcgccgagac gccggtggcc 60
ggcaagaagg cctttacatg gcaggaggtc gcgcagcaca acacggcggc ctcggcctgg 120
atcattatcc gcggcaaggt ctacgacgtg accgagtggg ccaacaagca ccccggcggc 180
cgcgagatgg tgctgctgca cgccggtcgc gaggccaccg acacgttcga ctcgtaccac 240
ccgttcagcg acaaggccga gtcgatcttg aacaagtatg agattggcac gttcacgggc 300
ccgtccgagt ttccgacctt caagccggac acgggcttct acaaggagtg ccgcaagcgc 360
gttggcgagt acttcaagaa gaacaacctc catccgcagg acggcttccc gggcctctgg 420
cgcatgatgg tcgtgtttgc ggtcgccggc ctcgccttgt acggcatgca cttttcgact 480
atctttgcgc tgcagctcgc ggccgcggcg ctctttggcg tctgccaggc gctgccgctg 540
ctccacgtca tgcacgactc gtcgcacgcg tcgtacacca acatgccgtt cttccattac 600
gtcgtcggcc gctttgccat ggactggttt gccggcggct cgatggtgtc atggctcaac 660
cagcacgtcg tgggccacca catctacacg aacgtcgcgg gctcggaccc ggatcttccg 720
gtcaacatgg acggcgacat ccgccgcatc gtgaaccgcc aggtgttcca gcccatgtac 780
gcattccagc acatctacct tccgccgctc tatggcgtgc ttggcctcaa gttccgcatc 840
caggacttca ccgacacgtt cggctcgcac acgaacggcc cgatccgcgt caacccgcac 900
gcgctctcga cgtggatggc catgatcagc tccaagtcgt tctgggcctt ctaccgcgtg 960
taccttccgc ttgccgtgct ccagatgccc atcaagacgt accttgcgat cttcttcctc 1020
gccgagtttg tcacgggctg gtacctcgcg ttcaacttcc aagtaagcca tgtctcgacc 1080
gagtgcggct acccatgcgg cgacgaggcc aagatggcgc tccaggacga gtgggcagtc 1140
```

```
tcgcaggtca agacgtcggt cgactacgcc catggctcgt ggatgacgac gttccttgcc 1200
ggcgcgctca actaccaggt cgtgcaccac ttgttcccca gcgtgtcgca gtaccactac 1260
ccggcgatcg cgcccatcat cgtcgacgtc tgcaaggagt acaacatcaa gtacgccatc 1320
ttgccggact ttacggcggc gttcgttgcc cacttgaagc acctccgcaa catgggccag 1380
cagggcatcg ccgccacgat ccacatgggc taa                               1413
```

<210> 20
<211> 470
<212> PRT
<213> Saprolegnia diclina

<400> 20

```
Met Ala Pro Gln Thr Glu Leu Arg Gln Arg His Ala Ala Val Ala Glu
 1               5                  10                  15
Thr Pro Val Ala Gly Lys Lys Ala Phe Thr Trp Gln Glu Val Ala Gln
            20                  25                  30
His Asn Thr Ala Ala Ser Ala Trp Ile Ile Ile Arg Gly Lys Val Tyr
        35                  40                  45
Asp Val Thr Glu Trp Ala Asn Lys His Pro Gly Gly Arg Glu Met Val
    50                  55                  60
Leu Leu His Ala Gly Arg Glu Ala Thr Asp Thr Phe Asp Ser Tyr His
65                  70                  75                  80
Pro Phe Ser Asp Lys Ala Glu Ser Ile Leu Asn Lys Tyr Glu Ile Gly
                85                  90                  95
Thr Phe Thr Gly Pro Ser Glu Phe Pro Thr Phe Lys Pro Asp Thr Gly
            100                 105                 110
Phe Tyr Lys Glu Cys Arg Lys Arg Val Gly Glu Tyr Phe Lys Lys Asn
        115                 120                 125
Asn Leu His Pro Gln Asp Gly Phe Pro Gly Leu Trp Arg Met Met Val
    130                 135                 140
Val Phe Ala Val Ala Gly Leu Ala Leu Tyr Gly Met His Phe Ser Thr
145                 150                 155                 160
Ile Phe Ala Leu Gln Leu Ala Ala Ala Ala Leu Phe Gly Val Cys Gln
                165                 170                 175
Ala Leu Pro Leu Leu His Val Met His Asp Ser Ser His Ala Ser Tyr
            180                 185                 190
Thr Asn Met Pro Phe Phe His Tyr Val Val Gly Arg Phe Ala Met Asp
        195                 200                 205
Trp Phe Ala Gly Gly Ser Met Val Ser Trp Leu Asn Gln His Val Val
    210                 215                 220
Gly His His Ile Tyr Thr Asn Val Ala Gly Ser Asp Pro Asp Leu Pro
225                 230                 235                 240
Val Asn Met Asp Gly Asp Ile Arg Arg Ile Val Asn Arg Gln Val Phe
                245                 250                 255
Gln Pro Met Tyr Ala Phe Gln His Ile Tyr Leu Pro Pro Leu Tyr Gly
            260                 265                 270
Val Leu Gly Leu Lys Phe Arg Ile Gln Asp Phe Thr Asp Thr Phe Gly
        275                 280                 285
Ser His Thr Asn Gly Pro Ile Arg Val Asn Pro His Ala Leu Ser Thr
    290                 295                 300
Trp Met Ala Met Ile Ser Ser Lys Ser Phe Trp Ala Phe Tyr Arg Val
305                 310                 315                 320
Tyr Leu Pro Leu Ala Val Leu Gln Met Pro Ile Lys Thr Tyr Leu Ala
                325                 330                 335
Ile Phe Phe Leu Ala Glu Phe Val Thr Gly Trp Tyr Leu Ala Phe Asn
            340                 345                 350
Phe Gln Val Ser His Val Ser Thr Glu Cys Gly Tyr Pro Cys Gly Asp
```

```
                355                           360                           365
Glu Ala Lys Met Ala Leu Gln Asp Glu Trp Ala Val Ser Gln Val Lys
    370                     375                     380
Thr Ser Val Asp Tyr Ala His Gly Ser Trp Met Thr Thr Phe Leu Ala
385                     390                     395                     400
Gly Ala Leu Asn Tyr Gln Val Val His His Leu Phe Pro Ser Val Ser
                405                     410                     415
Gln Tyr His Tyr Pro Ala Ile Ala Pro Ile Ile Val Asp Val Cys Lys
            420                     425                     430
Glu Tyr Asn Ile Lys Tyr Ala Ile Leu Pro Asp Phe Thr Ala Ala Phe
        435                     440                     445
Val Ala His Leu Lys His Leu Arg Asn Met Gly Gln Gln Gly Ile Ala
    450                     455                     460
Ala Thr Ile His Met Gly
465                 470
```

<210> 21
<211> 914
<212> DNA
<213> Homo sapiens

<400> 21

```
atggaacatt ttgatgcatc acttagtacc tatttcaagg cattgctagg ccctcgagat 60
actagagtaa aaggatggtt tcttctggac aattatatac ccacatttat ctgctctgtc 120
atatatttac taattgtatg gctgggacca aaatacatga ggaataaaca gccattctct 180
tgccgggga ttttagtggt gtataacctt ggactcacac tgctgtctct gtatatgttc 240
tgtgagttag taacaggagt atgggaaggc aaatacaact tcttctgtca gggcacacgc 300
accgcaggag aatcagatat gaagattatc cgtgtcctct ggtggtacta cttctccaaa 360
ctcatagaat ttatggacac tttcttcttc atcctgcgca agaacaacca ccagatcacg 420
gtcctgcacg tctaccacca tgcctcgatg ctgaacatct ggtggtttgt gatgaactgg 480
gtcccctgcg gccactctta ttttggtgcc acacttaata gcttcatcca cgtcctcatg 540
tactcttact atggtttgtc gtcagtccct tccatgcgtc catacctctg gtggaagaag 600
tacatcactc aggggcagct gcttcagttt gtgctgacaa tcatccagac cagctgcggg 660
gtcatctggc cgtgcacatt ccctcttggt tggttgtatt tccagattgg atacattatt 720
tccctgattg ctctcttcac aaacttctac attcagacct acaacaagaa aggggcctcc 780
cgaaggaaag accacctgaa ggaccaccag aatgggtccg tggctgctgt gaatggacac 840
accaacagct tttcacccct ggaaaacaat gtgaagccaa ggaagctgcg gaaggattga 900
agtcaaagaa ttga                                                   914
```

<210> 22
<211> 957
<212> DNA
<213> Mortierella alpina

<400> 22

```
atggagtcga ttgcgccatt cctcccatca aagatgccgc aagatctgtt tatggacctt 60
gccaccgcta tcggtgtccg ggccgcgccc tatgtcgatc ctctcgaggc cgcgctggtg 120
gcccaggccg agaagtacat ccccacgatt gtccatcaca cgcgtgggtt cctggtcgcg 180
gtggagtcgc ctttggcccg tgagctgccg ttgatgaacc cgttccacgt gctgttgatc 240
gtgctcgctt atttggtcac ggtctttgtg ggcatgcaga tcatgaagaa ctttgagcgg 300
ttcgaggtca agacgttttc gctcctgcac aacttttgtc tggtctcgat cagcgcctac 360
atgtgcggtg ggatcctgta cgaggcttat caggccaact atggactgtt tgagaacgct 420
gctgatcata ccttcaaggg tcttcctatg gccaagatga tctggctctt ctacttctcc 480
aagatcatgg agtttgtcga caccatgatc atggtcctca agaagaacaa ccgccagatc 540
tccttcttgc acgtttacca ccacagctcc atcttcacca tctggtggtt ggtcaccttt 600
gttgcaccca acggtgaagc ctacttctct gctgcgttga actcgttcat ccatgtgatc 660

atgtacggct actacttctt gtcggccttg ggcttcaagc aggtgtcgtt catcaagttc 720
tacatcacgc gctcgcagat gacacagttc tgcatgatgt cggtccagtc ttcctgggac 780
atgtacgcca tgaaggtcct tggccgcccc ggatacccct tcttcatcac ggctctgctt 840
tggttctaca tgtggaccat gctcggtctc ttctacaact tttacagaaa gaacgccaag 900
ttggccaagc aggccaaggc cgacgctgcc aaggagaagg caaggaagtt gcagtaa    957
```

<210> 23
<211> 30
<212> DNA
<213> Artificial Sequence

<220>
<223> Primer R0936

<400> 23
gtcgggcaag gcggaaaagt acctcaagag 30

<210> 24
<211> 30
<212> DNA
<213> Artificial Sequence

<220>
<223> Primer RO937

<400> 24
aaacctgtag acaatgtgga ggggcgtggg 30

<210> 25
<211> 42
<212> DNA
<213> Artificial Sequence

<220>
<223> Primer R0972

<400> 25
atacttgaat tcatgggacg cggcggcgaa ggtcaggtga ac 42

<210> 26
<211> 39
<212> DNA
<213> Artificial Sequence

<220>
<223> Primer R0949

<400> 26
cttatactcg agctaagcgg ccttggccgc cgcctggcc 39

<210> 27
<211> 41
<212> DNA
<213> Artificial Sequence

<220>
<223> Primer R0950

<400> 27
cttatactcg agtaaatggc tcgcgaggcg aagcgagtgg c 41

<210> 28
<211> 1320
<212> DNA
<213> Thraustochytrium aureum

<400> 28

```
atgggacgcg gcggcgaagg tcaggtgaac agcgcgcagg tggcacaagg cggtgcggga 60
acgcgaaaga cgatcctgat cgagggcgag gtctacgatg tcaccaactt taggcacccc 120
ggcgggtcga tcatcaagtt tctcacgacc gacggcaccg aggctgtgga cgcgacgaac 180
gcgtttcgcg agtttcactg ccggtcgggc aaggcggaaa agtacctcaa gagcctgccc 240
aagctcggcg cgccgagcaa gatgaagttt gacgccaagg agcaggcccg gcgcgacgcg 300
atcacgcgag actacgtcaa gctgcgcgag gagatggtgg ccgagggcct cttcaagccc 360
gcgcccctcc acattgtcta caggtttgcg gagatcgcag ccctgttcgc ggcctcgttc 420
tacctgtttt cgatgcgcgg aaacgtgttc gccacgctcg cggccatcgc agtcgggggc 480
atcgcgcagg gccgctgcgg ctggctcatg cacgagtgcg gacacttctc gatgaccggg 540
tacatcccgc ttgacgtgcg cctgcaggag ctggtgtacg gcgtggggtg ctcgatgtcg 600
gcgagctggt ggcgcgttca gcacaacaag caccacgcga ccccgcagaa actcaagcac 660
gacgtcgacc tcgacaccct gccgctcgtt gcgttcaacg agaagatcgc cgccaaggtg 720
cgccccggct cgttccaggc caagtggctc tcggcgcagg cgtacatttt tgcgccggtg 780
tcctgcttcc tggttggtct cttctggacc ctgtttctgc acccgcgcca catgccgcgc 840
acgagccact ttgctgagat ggccgccgtc gcggtgcgcg tcgtgggctg ggcggcgctc 900
atgcactcgt tcgggtacag cgggagcgac tcgttcggtc tctacatggc cacctttggc 960
tttggctgca cctacatctt caccaacttt gcggtcagcc acacgcacct cgacgtcacc 1020
gagccggacg agttcctgca ctgggtcgag tacgccgcgc tgcacacgac caacgtgtcc 1080
aacgactcgt ggttcatcac ctggtggatg tcgtacctca actttcagat cgagcaccac 1140
ctctttccgt cgctgcccca gctcaacgcc ccgcgcgtcg ccccgcgcgt ccgcgccctc 1200
ttcgagaagc acggcatggc ttacgacgag cgcccgtacc ttaccgcgct tggcgacacg 1260
tttgccaacc tgcacgccgt gggccaaaac gcgggccagg cggcggccaa ggccgcttag 1320
```

<210> 29
<211> 439
<212> PRT
<213> Thraustochytrium aureum

<400> 29

```
Met Gly Arg Gly Gly Glu Gly Gln Val Asn Ser Ala Gln Val Ala Gln
 1               5                  10                  15
Gly Gly Ala Gly Thr Arg Lys Thr Ile Leu Ile Glu Gly Glu Val Tyr
            20                  25                  30
Asp Val Thr Asn Phe Arg His Pro Gly Gly Ser Ile Ile Lys Phe Leu
        35                  40                  45
Thr Thr Asp Gly Thr Glu Ala Val Asp Ala Thr Asn Ala Phe Arg Glu
    50                  55                  60
Phe His Cys Arg Ser Gly Lys Ala Glu Lys Tyr Leu Lys Ser Leu Pro
65                  70                  75                  80
Lys Leu Gly Ala Pro Ser Lys Met Lys Phe Asp Ala Lys Glu Gln Ala
                85                  90                  95
Arg Arg Asp Ala Ile Thr Arg Asp Tyr Val Lys Leu Arg Glu Glu Met
            100                 105                 110
Val Ala Glu Gly Leu Phe Lys Pro Ala Pro Leu His Ile Val Tyr Arg
        115                 120                 125
Phe Ala Glu Ile Ala Ala Leu Phe Ala Ala Ser Phe Tyr Leu Phe Ser


    130                 135                 140
Met Arg Gly Asn Val Phe Ala Thr Leu Ala Ala Ile Ala Val Gly Gly
145                 150                 155                 160
Ile Ala Gln Gly Arg Cys Gly Trp Leu Met His Glu Cys Gly His Phe
                165                 170                 175
Ser Met Thr Gly Tyr Ile Pro Leu Asp Val Arg Leu Gln Glu Leu Val
            180                 185                 190
Tyr Gly Val Gly Cys Ser Met Ser Ala Ser Trp Trp Arg Val Gln His
        195                 200                 205
Asn Lys His His Ala Thr Pro Gln Lys Leu Lys His Asp Val Asp Leu
    210                 215                 220
Asp Thr Leu Pro Leu Val Ala Phe Asn Glu Lys Ile Ala Ala Lys Val
225                 230                 235                 240
Arg Pro Gly Ser Phe Gln Ala Lys Trp Leu Ser Ala Gln Ala Tyr Ile
                245                 250                 255
Phe Ala Pro Val Ser Cys Phe Leu Val Gly Leu Phe Trp Thr Leu Phe
            260                 265                 270
Leu His Pro Arg His Met Pro Arg Thr Ser His Phe Ala Glu Met Ala
        275                 280                 285
Ala Val Ala Val Arg Val Val Gly Trp Ala Ala Leu Met His Ser Phe
    290                 295                 300
Gly Tyr Ser Gly Ser Asp Ser Phe Gly Leu Tyr Met Ala Thr Phe Gly
305                 310                 315                 320
Phe Gly Cys Thr Tyr Ile Phe Thr Asn Phe Ala Val Ser His Thr His
                325                 330                 335
Leu Asp Val Thr Glu Pro Asp Glu Phe Leu His Trp Val Glu Tyr Ala
            340                 345                 350
Ala Leu His Thr Thr Asn Val Ser Asn Asp Ser Trp Phe Ile Thr Trp
        355                 360                 365
Trp Met Ser Tyr Leu Asn Phe Gln Ile Glu His His Leu Phe Pro Ser
    370                 375                 380
Leu Pro Gln Leu Asn Ala Pro Arg Val Ala Pro Arg Val Arg Ala Leu
385                 390                 395                 400
Phe Glu Lys His Gly Met Ala Tyr Asp Glu Arg Pro Tyr Leu Thr Ala
                405                 410                 415
Leu Gly Asp Thr Phe Ala Asn Leu His Ala Val Gly Gln Asn Ala Gly
            420                 425                 430
Gln Ala Ala Ala Lys Ala Ala
        435
```

<210> 30

<211> 1338
<212> DNA
<213> Thraustochytrium aureum

<400> 30

```
gaattcacca tgggtcgcgg agcacaggga gagccaaggc aggccacaga gctgaagagc      60
agcccaagtg agcagcgtaa ggtgttgctc attgacgggc agctgtacga tgcaaccaac     120
ttcaggcatc ctggtggctc catcatcaaa tatttgtgca ccgatggcaa ggaggtagtt     180
gatgcaaccg aagcgtacaa ggagttccac tgcagatcct cgaaggcggt caagtacctc     240
aactccctgc caaagatcga cggcccaatc aagtacaaat acgacgcaaa ggagcaggct     300
cgccatgaca aactcacgag ggagtatgta gctctccgcg aacagctcgt caaggaggga     360
tactttgacc ccagcccgct ccacattatc tacagatgcg ccgagttggc agccatgttc     420
gctctctcgt tctacctttt ctccttcaag ggtaacgtca tggccactat tgctgccatc     480
gtgattgggg ggtgcgtgca gggtcgttgt gggtggctca tgcatgaagc tggccactac     540
agcatgaccg gaaacatccc tgttgacttg cgccttcaag agtttttgta cggaattggg     600

tgtggcatga gcggggcttg gtggagaagc cagcacaaca agcaccacgc caccccccaa     660
aagctcaagc atgacgttga tttggacact cttcctcttg tcgcctggaa cgagaaaatt     720
gcccgtcgcg tcaagccagg tagcttccag gcaaagtggc ttcatctcca gggatacatc     780
tttgccccag tctcctgcct tctcgttggt ctcttctgga ctttgtactt gcatcctcgc     840
cacatgatcc gcaccaagcg caacttcgag atattttctg tcgctctgcg ctacgtatgc     900
tggttctcgc ttcttttgag catgggctac actgtcggag agtctctggg tctctatgtg     960
cttacttttg gacttggctg tacctacatc tttacgcatt ttgctgtaag ccacacccac    1020
ttgccagtgt ccgaggagga cgagtacctg cactgggtcg agtacgctgc gctgcacacc    1080
acgaacgttg ccatcgactc gtacgttgtc acctggctga tgagctacct caactttcag    1140
atcgagcacc acttgttccc ttgctgcccg cagttccgcc accctgcaat ctcttctcgc    1200
gtcaagaaac ttttcgagga caatggtctg gtatacgacg cccgctcata cgtccaggcg    1260
ctcaaggata ccttcggcaa cctacacgaa gtgggcgtca acgctggcca agctgccaag    1320
agcgagtaag atctcgag                                                   1338
```

<210> 31
<211> 439
<212> PRT
<213> Thraustochytrium aureum

<400> 31

```
Met Gly Arg Gly Ala Gln Gly Glu Pro Arg Gln Ala Thr Glu Leu Lys
 1               5                  10                   .  15
Ser Ser Pro Ser Glu Gln Arg Lys Val Leu Leu Ile Asp Gly Gln Leu
            20                  25                  30
Tyr Asp Ala Thr Asn Phe Arg His Pro Gly Gly Ser Ile Ile Lys Tyr
        35                  40                  45
Leu Cys Thr Asp Gly Lys Glu Val Val Asp Ala Thr Glu Ala Tyr Lys
    50                  55                  60
Glu Phe His Cys Arg Ser Ser Lys Ala Val Lys Tyr Leu Asn Ser Leu
65                  70                  75                  80
Pro Lys Ile Asp Gly Pro Ile Lys Tyr Lys Tyr Asp Ala Lys Glu Gln
                85                  90                  95
Ala Arg His Asp Lys Leu Thr Arg Glu Tyr Val Ala Leu Arg Glu Gln
            100                 105                 110
Leu Val Lys Glu Gly Tyr Phe Asp Pro Ser Pro Leu His Ile Ile Tyr
        115                 120                 125
Arg Cys Ala Glu Leu Ala Ala Met Phe Ala Leu Ser Phe Tyr Leu Phe
    130                 135                 140
Ser Phe Lys Gly Asn Val Met Ala Thr Ile Ala Ala Ile Val Ile Gly
145                 150                 155                 160
Gly Cys Val Gln Gly Arg Cys Gly Trp Leu Met His Glu Ala Gly His
                165                 170                 175
Tyr Ser Met Thr Gly Asn Ile Pro Val Asp Leu Arg Leu Gln Glu Phe
            180                 185                 190
Leu Tyr Gly Ile Gly Cys Gly Met Ser Gly Ala Trp Trp Arg Ser Gln
        195                 200                 205
His Asn Lys His His Ala Thr Pro Gln Lys Leu Lys His Asp Val Asp
    210                 215                 220
Leu Asp Thr Leu Pro Leu Val Ala Trp Asn Glu Lys Ile Ala Arg Arg
225                 230                 235                 240
Val Lys Pro Gly Ser Phe Gln Ala Lys Trp Leu His Leu Gln Gly Tyr
                245                 250                 255
Ile Phe Ala Pro Val Ser Cys Leu Leu Val Gly Leu Phe Trp Thr Leu
            260                 265                 270
Tyr Leu His Pro Arg His Met Ile Arg Thr Lys Arg Asn Phe Glu Ile
        275                 280                 285
Phe Ser Val Ala Leu Arg Tyr Val Cys Trp Phe Ser Leu Leu Leu Ser


    290                 295                 300
Met Gly Tyr Thr Val Gly Glu Ser Leu Gly Leu Tyr Val Leu Thr Phe
305                 310                 315                 320
Gly Leu Gly Cys Thr Tyr Ile Phe Thr His Phe Ala Val Ser His Thr
                325                 330                 335
His Leu Pro Val Ser Glu Glu Asp Glu Tyr Leu His Trp Val Glu Tyr
            340                 345                 350
Ala Ala Leu His Thr Thr Asn Val Ala Ile Asp Ser Tyr Val Val Thr
        355                 360                 365
Trp Leu Met Ser Tyr Leu Asn Phe Gln Ile Glu His His Leu Phe Pro
    370                 375                 380
Cys Cys Pro Gln Phe Arg His Pro Ala Ile Ser Ser Arg Val Lys Lys
385                 390                 395                 400
Leu Phe Glu Asp Asn Gly Leu Val Tyr Asp Ala Arg Ser Tyr Val Gln
                405                 410                 415
Ala Leu Lys Asp Thr Phe Gly Asn Leu His Glu Val Gly Val Asn Ala
            420                 425                 430
Gly Gln Ala Ala Lys Ser Glu
        435
```

<210> 32

<211> 1381
<212> DNA
<213> Thraustochytrium aureum

<400> 32

```
ccatgggccg cggcggcgag aaaagcgagg tggaccaggt gcagccacaa aagaccgagc      60
agctccagaa ggccaagtgg gaggatgttg ttcgcatcaa tggagtcgaa tacgacgtca     120
cggactatct cagaaaacac cctggtggca gcgtgatcaa gtacgggctt gccaacaccg     180
gcgctgatgc cacgtccctc tttgaagcgt tccacatgcg ctcaaagaag gctcagatgg     240
tgctcaagtc tctcccaaag cgtgctccgg tcctcgagat ccagccaaac cagcttccag     300
aggagcagac caaggaggcg gagatgctgc gtgattttaa aaaatttgag gatgagattc     360
gccgggatgg attgatggaa ccttccttct ggcatcgcgc ttacagatta tcagagcttg     420
taggtatgtt cacgctcggc ctctacctct tctcgttaaa cactcctctg tctattgctg     480
ctggtgtcct cgtccacggt ctctttggtg cattctgtgg atggtgccag catgaggcag     540
gccacggctc ctttttttac agcctttggt ggggcaagcg tgtacaggcc atgttgatcg     600
ggtttggtct aggaacatcc ggcgacatgt ggaacatgat gcacaacaag catcatgctg     660
ccacccaaaa ggttcatcac gaccttgaca ttgacacaac tccttttgta gctttcttca     720
acactgcatt tgagaaaaac agatggaagg gcttttccaa ggcttgggtc cgctttcagg     780
ctttcacgtt cattcctgtc accagcggca tgatcgtcat gctgttctgg ctgtttttc      840
tccaccctcg ccgcgtcgtt caaaagaaga actttgagga gggtttttgg atgctgtcga     900
gccacattgt gcgcacctat ctcttccacc ttgtgaccgg ctgggagagc ctcgctgcat     960
gctaccttgt tgggtattgg gcgtgcatgt gggtgtccgg tatgtatttg tttggccact    1020
tttcgctctc ccacactcat atggacattg tggaggcgga cgtgcataag aactgggtca    1080
ggtacgctgt tgaccacact gttgacatca gcccatccaa cccgctcgtg tgctgggtca    1140
tgggttacct caacatgcag accatccacc acttgtggcc tgccatgccc cagtaccacc    1200
aggtcgaggt ctcacgccgc tttgccatct tcgccaaaaa acacggcctc aactaccgcg    1260
tcgtctctta ctttgaggct tggcgcctga tgctccaaaa tcttgctgac gtcggttccc    1320
actaccatga gaacggtgtc aagcgcgccc caaagaaagc caaggcgcag tagaaagcta    1380
t                                                                    1381
```

<210> 33
<211> 456
<212> PRT
<213> Thraustochytrium aureum

<400> 33

```
Met Gly Arg Gly Gly Glu Lys Ser Glu Val Asp Gln Val Gln Pro Gln
 1               5                  10                  15
Lys Thr Glu Gln Leu Gln Lys Ala Lys Trp Glu Asp Val Val Arg Ile
            20                  25                  30
Asn Gly Val Glu Tyr Asp Val Thr Asp Tyr Leu Arg Lys His Pro Gly
        35                  40                  45
Gly Ser Val Ile Lys Tyr Gly Leu Ala Asn Thr Gly Ala Asp Ala Thr
    50                  55                  60
Ser Leu Phe Glu Ala Phe His Met Arg Ser Lys Lys Ala Gln Met Val
65                  70                  75                  80
Leu Lys Ser Leu Pro Lys Arg Ala Pro Val Leu Glu Ile Gln Pro Asn
                85                  90                  95
Gln Leu Pro Glu Glu Gln Thr Lys Glu Ala Glu Met Leu Arg Asp Phe
            100                 105                 110
Lys Lys Phe Glu Asp Glu Ile Arg Arg Asp Gly Leu Met Glu Pro Ser
        115                 120                 125
Phe Trp His Arg Ala Tyr Arg Leu Ser Glu Leu Val Gly Met Phe Thr
    130                 135                 140
Leu Gly Leu Tyr Leu Phe Ser Leu Asn Thr Pro Leu Ser Ile Ala Ala
145                 150                 155                 160
Gly Val Leu Val His Gly Leu Phe Gly Ala Phe Cys Gly Trp Cys Gln
                165                 170                 175
His Glu Ala Gly His Gly Ser Phe Phe Tyr Ser Leu Trp Trp Gly Lys
            180                 185                 190
Arg Val Gln Ala Met Leu Ile Gly Phe Gly Leu Gly Thr Ser Gly Asp
        195                 200                 205
Met Trp Asn Met Met His Asn Lys His His Ala Ala Thr Gln Lys Val
    210                 215                 220
His His Asp Leu Asp Ile Asp Thr Thr Pro Phe Val Ala Phe Phe Asn
225                 230                 235                 240
Thr Ala Phe Glu Lys Asn Arg Trp Lys Gly Phe Ser Lys Ala Trp Val
                245                 250                 255
Arg Phe Gln Ala Phe Thr Phe Ile Pro Val Thr Ser Gly Met Ile Val
            260                 265                 270
Met Leu Phe Trp Leu Phe Phe Leu His Pro Arg Arg Val Val Gln Lys
        275                 280                 285
Lys Asn Phe Glu Glu Gly Phe Trp Met Leu Ser Ser His Ile Val Arg
    290                 295                 300
Thr Tyr Leu Phe His Leu Val Thr Gly Trp Glu Ser Leu Ala Ala Cys
305                 310                 315                 320
Tyr Leu Val Gly Tyr Trp Ala Cys Met Trp Val Ser Gly Met Tyr Leu
                325                 330                 335
Phe Gly His Phe Ser Leu Ser His Thr His Met Asp Ile Val Glu Ala
            340                 345                 350
Asp Val His Lys Asn Trp Val Arg Tyr Ala Val Asp His Thr Val Asp
        355                 360                 365
Ile Ser Pro Ser Asn Pro Leu Val Cys Trp Val Met Gly Tyr Leu Asn
    370                 375                 380
Met Gln Thr Ile His His Leu Trp Pro Ala Met Pro Gln Tyr His Gln
385                 390                 395                 400
Val Glu Val Ser Arg Arg Phe Ala Ile Phe Ala Lys Lys His Gly Leu
                405                 410                 415
Asn Tyr Arg Val Val Ser Tyr Phe Glu Ala Trp Arg Leu Met Leu Gln
            420                 425                 430
Asn Leu Ala Asp Val Gly Ser His Tyr His Glu Asn Gly Val Lys Arg
        435                 440                 445
Ala Pro Lys Lys Ala Lys Ala Gln
```

450 455

<210> 34
<211> 1329
<212> DNA
<213> Isochrysis galbana

<400> 34

```
atggtggcag gcaaatcagg cgctgcggcg cacgtgactc acagctcgac attgccccgt      60
gagtaccatg gcgcgaccaa cgactcgcgc tctgaggcgg ccgacgtcac cgtctctagc     120
atcgatgctg aaaaggagat gatcatcaac ggccgcgtgt atgacgtgtc gtcatttgtg     180
aagcggcacc caggtggctc ggtgatcaag ttccagctgg gcgccgacgc gagcgacgcg     240
tacaacaact ttcacgtccg ctccaagaag gcggacaaga tgctgtattc gctcccgtcc     300
cggccggccg aggccggcta cgcccaggac gacatctccc gcgactttga gaagctgcgc     360
ctcgagctga aggaggaggg ctacttcgag cccaacctgg tgcacgtgag ctacaggtgt     420
gtggaggttc ttgccatgta ctgggctggc gtccagctca tctggtccgg gtactggttc     480
ctcggcgcga tcgtggccgg cattgcgcag ggccgctgcg gctggctcca gcatgagggt     540
gggcactact cgctcaccgg caacatcaag atcgaccggc atctgcagat ggccatctat     600
gggcttggct gcggcatgtc gggctgctac tggcgcaacc agcacaacaa gcaccacgcc     660
acgccgcaga agctcgggac cgaccccgac ctgcagacga tgccgctggt ggccttccac     720
aagatcgtcg gcgccaaggc gcgaggcaag ggcaaggcgt ggctggcgtg gcaggcgccg     780
ctcttctttg gcgggatcat ctgctcgctc gtctctttcg gctggcagtt cgtgctccac     840
cccaaccacg cgctgcgcgt gcacaatcac ctggagctcg cgtacatggg cctgcggtac     900
gtgctgtggc acctggcctt tggccacctc gggctgctga gctcgctccg cctgtacgcc     960
ttttacgtgg ccgtgggcgg cacctacatc ttcaccaact tcgccgtctc gcacacccac    1020
aaggacgtcg tcccgcccac caagcacatc tcgtgggcac tctactcggc caaccacacg    1080
accaactgct ccgactcgcc ctttgtcaac tggtggatgg cctacctcaa cttccagatc    1140
gagcaccacc tcttcccgtc gatgccgcag tacaaccacc ccaagatcgc ccccgcgggtg   1200
cgcgcgctct tcgagaagca cggggtcgag tatgacgtcc ggccatacct ggagtgtttt    1260
cgggtcacgt acgtcaacct gctcgccgta ggcaacccgg agcactccta ccacgagcac    1320
acgcactag                                                           1329
```

<210> 35
<211> 442
<212> PRT
<213> Isochrysis galbana

<400> 35

```
Met Val Ala Gly Lys Ser Gly Ala Ala Ala His Val Thr His Ser Ser
 1               5                  10                  15
Thr Leu Pro Arg Glu Tyr His Gly Ala Thr Asn Asp Ser Arg Ser Glu
            20                  25                  30
Ala Ala Asp Val Thr Val Ser Ser Ile Asp Ala Glu Lys Glu Met Ile
        35                  40                  45
Ile Asn Gly Arg Val Tyr Asp Val Ser Ser Phe Val Lys Arg His Pro
    50                  55                  60
Gly Gly Ser Val Ile Lys Phe Gln Leu Gly Ala Asp Ala Ser Asp Ala
65                  70                  75                  80
Tyr Asn Asn Phe His Val Arg Ser Lys Lys Ala Asp Lys Met Leu Tyr
                85                  90                  95
Ser Leu Pro Ser Arg Pro Ala Glu Ala Gly Tyr Ala Gln Asp Asp Ile
            100                 105                 110
Ser Arg Asp Phe Glu Lys Leu Arg Leu Glu Leu Lys Glu Glu Gly Tyr
        115                 120                 125
Phe Glu Pro Asn Leu Val His Val Ser Tyr Arg Cys Val Glu Val Leu
    130                 135                 140
Ala Met Tyr Trp Ala Gly Val Gln Leu Ile Trp Ser Gly Tyr Trp Phe
145                 150                 155                 160
Leu Gly Ala Ile Val Ala Gly Ile Ala Gln Gly Arg Cys Gly Trp Leu
                165                 170                 175
Gln His Glu Gly Gly His Tyr Ser Leu Thr Gly Asn Ile Lys Ile Asp
            180                 185                 190
Arg His Leu Gln Met Ala Ile Tyr Gly Leu Gly Cys Gly Met Ser Gly
        195                 200                 205
Cys Tyr Trp Arg Asn Gln His Asn Lys His His Ala Thr Pro Gln Lys
    210                 215                 220
Leu Gly Thr Asp Pro Asp Leu Gln Thr Met Pro Leu Val Ala Phe His
225                 230                 235                 240
Lys Ile Val Gly Ala Lys Ala Arg Gly Lys Gly Lys Ala Trp Leu Ala
                245                 250                 255
Trp Gln Ala Pro Leu Phe Phe Gly Gly Ile Ile Cys Ser Leu Val Ser
            260                 265                 270
Phe Gly Trp Gln Phe Val Leu His Pro Asn His Ala Leu Arg Val His
        275                 280                 285
Asn His Leu Glu Leu Ala Tyr Met Gly Leu Arg Tyr Val Leu Trp His
    290                 295                 300
Leu Ala Phe Gly His Leu Gly Leu Leu Ser Ser Leu Arg Leu Tyr Ala
305                 310                 315                 320
Phe Tyr Val Ala Val Gly Gly Thr Tyr Ile Phe Thr Asn Phe Ala Val
                325                 330                 335
Ser His Thr His Lys Asp Val Val Pro Pro Thr Lys His Ile Ser Trp
            340                 345                 350
Ala Leu Tyr Ser Ala Asn His Thr Thr Asn Cys Ser Asp Ser Pro Phe
        355                 360                 365
Val Asn Trp Trp Met Ala Tyr Leu Asn Phe Gln Ile Glu His His Leu
    370                 375                 380
Phe Pro Ser Met Pro Gln Tyr Asn His Pro Lys Ile Ala Pro Arg Val
385                 390                 395                 400
Arg Ala Leu Phe Glu Lys His Gly Val Glu Tyr Asp Val Arg Pro Tyr
                405                 410                 415
Leu Glu Cys Phe Arg Val Thr Tyr Val Asn Leu Leu Ala Val Gly Asn
            420                 425                 430
Pro Glu His Ser Tyr His Glu His Thr His
        435                 440
```

<210> 36
<211> 36
<212> DNA
<213> Artificial Sequence

<220>
<223> Primer R0838

<221> misc_feature
<222> (7)...(7)
<223> v = a or g or c at position 7

<221> misc_feature
<222> (10)...(10)
<223> r = g or a at position 10

<221> misc_feature
<222> (13)...(13)
<223> s = g or c at position 13

<221> misc_feature
<222> (16)...(16)
<223> r = g or a at position 16

<221> misc_feature
<222> (19)...(19)
<223> r = g or a at position 19

<221> misc_feature
<222> (22)...(22)
<223> y = t/u or c at position 22

<221> misc_feature
<222> (25)...(25)
<223> r = g or a at position 25

<221> misc_feature
<222> (31)...(31)
<223> r = g or a at position 31

<400> 36
catggtvggr aasagrtgrt gytcratctg rtagtt 36

<210> 37
<211> 25
<212> DNA
<213> Artificial Sequence

<220>

<223> Primer RO1065

<400> 37

cgacaagagg aagagtgtcc aaatc 25

<210> 38
<211> 30

<212> DNA
<213> Artificial Sequence

<220>
<223> Primer RO1064

<400> 38

cgccttcaag agtttttgta cggaattggg 30

<210> 39
<211> 30
<212> DNA
<213> Artificial Sequence

<220>

<223> Primer R01097

<400> 39
cttgtaccat gggtcgcgga gcacagggag 30

<210> 40
<211> 30
<212> DNA
<213> Artificial Sequence

<220>
<223> Primer RO1098

<400> 40
tgaagcttac tcgctcttgg cagcttggcc 30

<210> 41
<211> 30
<212> DNA
<213> Artificial Sequence

<220>
<223> Primer RO1107

<400> 41
tttaaccatg ggccgcggcg gcgagaaaag 30

<210> 42
<211> 36
<212> DNA
<213> Artificial Sequence

<220>
<223> Primer RO1108

<400> 42
gggaagaagc tttctactgc gccttggctt tctttg 36

<210> 43
<211> 26
<212> DNA

<213> Artificial Sequence

<220>
<223> Primer RO1235

<400> 43
cgaagttggt gaagatgtag gtgccg 26

<210> 44
<211> 27
<212> DNA
<213> Artificial Sequence

<220>
<223> Primer RO1232

<400> 44
gagcgacgcg tacaacaact ttcacgt 27

<210> 45
<211> 23
<212> DNA
<213> Artificial Sequence

<220>
<223> Primer

<400> 45
cgactggagc acgaggacac tga 23

<210> 46
<211> 25
<212> DNA
<213> Artificial Sequence

<220>
<223> GeneRacer 3 Prime Primer

<400> 46
gctgtcaacg atacgctacg taacg 25

<210> 47
<211> 26
<212> DNA
<213> Artificial Sequence

<220>
<223> Nested Primer RO1234

<400> 47
agctccaggt gattgtgcac gcgcag 26

<210> 48
<211> 27
<212> DNA
<213> Artificial Sequence

<220>

<223> Primer RO1233

<400> 48
gactttgaga agctgcgcct cgagctg 27

<210> 49
<211> 26
<212> DNA
<213> Artificial Sequence

<220>
<223> Nested 5 Prime Primer <400> 49

ggacactgac atggactgaa ggagta 26

<210> 50
<211> 23
<212> DNA
<213> Artificial Sequence

<220>
<223> Nested 3 Prime Primer

<400> 50
cgctacgtaa cggcatgaca gtg 23

<210> 51
<211> 35
<212> DNA
<213> Artificial Sequence

<220>
<223> Primer RO1309

<400> 51
atgatggaat tcatggtggc aggcaaatca ggcgc 35

<210> 52
<211> 35
<212> DNA
<213> Artificial Sequence

<220>
<223> Primer RO1310

<400> 52
aataatgtcg acctagtgcg tgtgctcgtg gtagg 35

<210> 53
<211> 14
<212> PRT
<213> Artificial Sequence

<220>
<223> Consensus Peptide Sequence

<400> 53

Val Tyr Asp Val Thr Glu Trp Val Lys Arg His Pro Gly Gly
1 5 10

<210> 54
<211> 15
<212> PRT
<213> Artificial Sequence

<220>
<223> Consensus Peptide Sequence

<400> 54

Gly Ala Ser Ala Asn Trp Trp Lys His Gln His Asn Val His His
1 5 10 15

<210> 55
<211> 12
<212> PRT
<213> Artificial Sequence

<220>
<223> Consensus Peptide Sequence

<400> 55

Asn Tyr Gln Ile Glu His His Leu Phe Pro Thr Met
1 5 10

## Description

### Technical Field

The subject invention relates to the identification and isolation of genes that encodes enzymes (i.e. Saprolegnia diclina Δ5-desaturase, and Saprolegnia diclina Δ6-desaturase) involved in the synthesis of polyunsaturated fatty acids and to uses thereof. In particular, Δ5-desaturase catalyzes the conversion of, for example, dihomo-γ-linolenic acid (DGLA) to arachidonic acid (AA) and (n-3)-eicosatetraenoic acid (20:4n-3) to eicosapentaenoic acid (20:5n-3). Delta-6 desaturase catalyzes the conversion of, for example, α-linolenic acid (ALA) to stearidonic acid (STA). The converted products may then be utilized as substrates in the production of other polyunsaturated fatty acids (PUFAs). The product or other polyunsaturated fatty acids may be added to pharmaceutical compositions, nutritional composition, animal feeds as well as other products such as cosmetics.

### Background Information

Desaturases are critical in the production of long-chain polyunsaturated fatty acids that have many important functions. For example, polyunsaturated fatty acids (PUFAs) are important components of the plasma membrane of a cell, where they are found in the form of phospholipids. They also serve as precursors to mammalian prostacyclins, eicosanoids, leukotrienes and prostaglandins. Additionally, PUFAs are necessary for the proper development of the developing infant brain as well as for tissue formation and repair. In view of the biological significance of PUFAs, attempts are being made to produce them, as well as intermediates leading to their production, in an efficient manner.

A number of enzymes are involved in PUFA biosynthesis in addition to Δ5-desaturase and Δ6-desaturase. For example, elongase (elo) catalyzes the conversion of γ-linolenic acid (GLA) to dihomo-γ-linolenic acid (DGLA) and of stearidonic acid (18:4n-3) to (n-3)-eicosatetraenoic acid (20:4n-3). Linoleic acid (LA, 18:2-Δ9,12 or 18:2n-6) is produced from oleic acid (18:1-Δ9) by a Δ12-desaturase. GLA (18:3-Δ6,9,12) is produced from linoleic acid by a Δ6-desaturase.

It must be noted that animals cannot desaturate beyond the Δ9 position and therefore cannot convert oleic acid into linoleic acid. Likewise, α-linolenic acid (ALA, 18:3-Δ9,12,15) cannot be synthesized by mammals. However, α-linolenic acid can be converted to stearidonic acid (STA, 18:4-ΔA6,9,12,15) by a Δ6-desaturase (see PCT publication WO 96/13591 and The Faseb Journal, Abstracts, Part I, Abstract 3093, page A532 (Experimental Biology 98, San Francisco, CA, April 18-22, 1998); see also U.S. Patent No. 5,552,306), followed by elongation to (n-3)-eicosatetraenoic acid (20:4-Δ8,11,14,17) in mammals and algae. This polyunsaturated fatty acid (i.e., 20:4-Δ8,11,14,17) can then be converted to eicosapentaenoic acid (EPA, 20:5-Δ5,8,11,14,17) by a Δ5-desaturase, such as that of the present invention. Other eukaryotes, including fungi and plants, have enzymes which desaturate at carbon 12 (see PCT publication WO 94/11516 and U.S. Patent No. 5,443,974) and carbon 15 (see PCT publication WO 93/11245). The major polyunsaturated fatty acids of animals therefore are either derived from diet and/or from desaturation and elongation of linoleic acid or α-linolenic acid. In view of these difficulties, it is of significant interest to isolate genes involved in PUFA synthesis from species that naturally produce these fatty acids and to express these genes in a microbial, plant, or animal system which can be altered to provide production of commercial quantities of one or more PUFAs.

One of the most important long chain PUFAs, noted above, is arachidonic acid (AA). AA is found in filamentous fungi and can also be purified from mammalian tissues including the liver and adrenal glands. As noted above, AA production from dihomo-γ-linolenic acid is catalyzed by a Δ5-desaturase. EPA is another important long-chain PUFA. EPA is found in fungi and also in marine oils. As noted above, EPA is produced from (n-3)-eicosatetraenoic acid and is catalyzed by a Δ5-desaturase. In view of the above discussion, there is a definite need for the Δ5-desaturase and Δ6-desaturase enzymes, the respective genes encoding these enzymes, as well as recombinant methods of producing these enzymes. Additionally, a need exists for oils containing levels of PUFAs beyond those naturally present as well as those enriched in novel PUFAs. Such oils can only be made by isolation and expression of the Δ5-desaturase and Δ6-desaturase genes.

### SUMMARY OF THE INVENTION

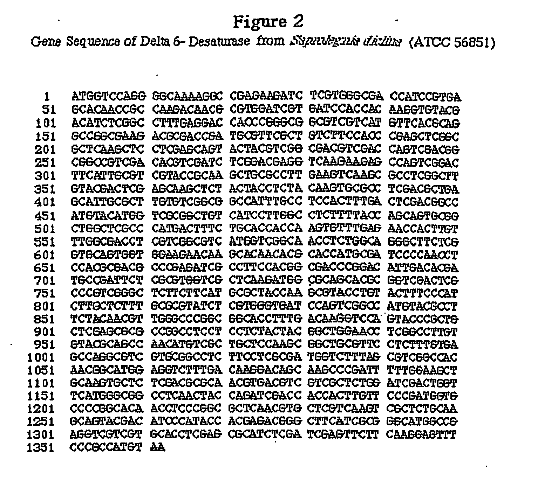

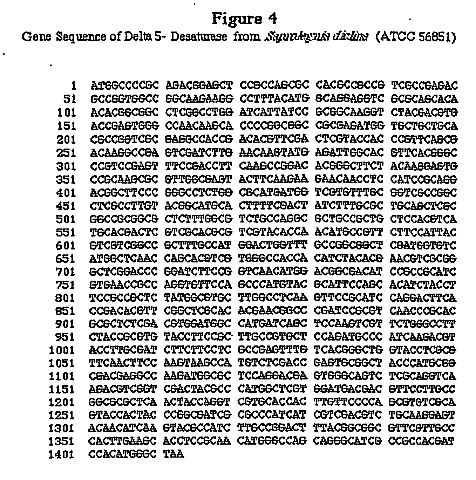

The present invention includes an isolated nucleotide sequence or fragment comprising or complementary to at least 90% of a nucleotide sequence comprising SEQ ID NO:13 (Figure 2) or SEQ ID NO:19 (Figure 4). In particular, the isolated nucleotide sequence may be represented by SEQ ID NO:13 or SEQ ID NO:19. These sequences may encode a functionally active desaturase which utilizes a polyunsaturated fatty acid as a substrate.

Furthermore, the present invention encompasses an isolated nucleotide sequence comprising or complementary to a nucleotide sequence encoding a polypeptide having desaturase activity and having at least 90% sequence identity to an amino acid sequence consisting of SEQ ID NO:20.

The nucleotide sequences may be derived from, for example, a fungus such as Saprolegnia diclina (SEQ ID NO:13 and SEQ ID NO:19).

The present invention also includes purified proteins or polypeptides encoded by the nucleotide sequences referred to above.

Additionally, the present invention includes a purified polypeptide which desaturates polyunsaturated fatty acids at carbon 5 or carbon 6 and has at least 90% amino acid identity to the amino acid sequence of the purified proteins of SEQ ID NO:14 or SEQ ID NO:20.

Furthermore, the present invention also encompasses a method of producing a desaturase (i.e., Δ5 or Δ6). This method comprises the steps of: a) isolating the nucleotide sequence selected from the group consisting of SEQ ID NO:19 and SEQ ID NO:13 ; b) constructing a vector comprising: i) the isolated nucleotide sequence operably linked to ii) a regulatory sequence; and c) introducing the vector into a host cell for a time and under conditions sufficient for expression of the Δ5-desaturase or Δ6-desaturase, as appropriate. The host cell may be, for example, a eukaryotic cell or a prokaryotic cell. In particular, the prokaryotic cell may be, for example, E. coli, Cyanobacteria or B. subtilis. The eukaryotic cell may be, for example, a non-human mammalian cell, an insect cell, a plant cell or a fungal cell (e.g., a yeast cell such as Saccharomyces cerevisiae, Saccharomyces carlsbergensis, Candida spp., Lipomyces starkey, Yarrowia lipolytica, Kluyveromyces spp., Hansenula spp., Trichoderma spp. or Pichia Spp.).

Additionally, the present invention also encompasses a vector comprising: a) a nucleotide sequence selected from the group consisting of SEQ ID NO:13 and SEQ ID NO:19 operably linked to b) a regulatory sequence. The invention also includes a non-human host cell comprising this vector. The non-human host cell may be, for example, a eukaryotic cell or a prokaryotic cell. Suitable eukaryotic cells and prokaryotic cells are as defined above.

Moreover, the present invention also includes a plant cell, plant or plant tissue comprising the above vector, wherein expression of the nucleotide sequence of the vector results in production of at least one polyunsaturated fatty acids by the plant cell, plant or plant tissue. The polyunsaturated fatty acid may be, for example, selected from the group consisting of AA, EPA, GLA and STA, depending upon whether the nucleotide sequence encodes a Δ5- or Δ6-desaturase.

Additionally, the present invention also encompasses a transgenic plant comprising the above vector, wherein expression of the nucleotide sequence of the vector results in production of a polyunsaturated fatty acid in seeds of the transgenic plant.

Also, the invention includes a non-human mammalian cell comprising the above vector wherein expression of the nucleotide sequence of the vector results in production of altered levels of AA, EPA, GLA or STA when the cell is grown in a culture media comprising a fatty acid selected from the group consisting of LA, ALA, DGLA and SP.

Additionally, the present invention includes a method (i.e., "first" method) for producing a polyunsaturated fatty acid comprising the steps of: a) isolating the nucleotide sequence of SEQ ID NO:19 ; b) constructing a vector comprising the isolated nucleotide sequence; c) introducing the vector into a host cell under time and conditions sufficient for expression of Δ5-desaturase enzyme; and d) exposing the expressed Δ5-desaturase enzyme to a substrate polyunsaturated fatty acid in order to convert the substrate to a product polyunsaturated fatty acid. The substrate polyunsaturated fatty acid may be DGLA or 20:4n-3 and the product polyunsaturated fatty acid may be AA or EPA, respectively. This method may further comprise the step of exposing the product polyunsaturated fatty acid to an elongase in in order to convert the product polyunsaturated fatty acid to another polyunsaturated fatty acid (i.e., "second" method). In this method containing the additional step (i.e., "second" method), the product polyunsaturated fatty acid may be AA or EPA, and the "another" polyunsaturated fatty acid may be adrenic acid or (n-3)-docosapentaenoic acid, respectively. The method containing the additional step may further comprise a step of exposing the another polyunsaturated fatty acid to an additional desaturase in order to convert the another polyunsaturated fatty acid to a final polyunsaturated fatty acid (i.e., "third" method). The final polyunsaturated fatty acid may be (n-6)-docosapentaenoic acid or docosahexaenoic (DHA) acid.

Additionally, the present invention includes a method for producing a polyunsaturated fatty acid comprising the steps of: a) isolating the nucleotide sequence represented by SEQ ID NO:13 ; b) constructing a vector comprising the isolated nucleotide sequence; c) introducing the vector into a host cell for a time and under conditions sufficient for expression of Δ6-desaturase enzyme; and d) exposing the expressed Δ6-desaturase enzyme to a substrate polyunsaturated fatty acid in order to convert the substrate to a product polyunsaturated fatty acid. The substrate polyunsaturated fatty acid may be LA or ALA, and the product polyunsaturated fatty acid may be GLA or STA, respectively. This method may further comprise the step of exposing the product polyunsaturated fatty acid to an elongase in order to convert the product polyunsaturated fatty acid to another polyunsaturated fatty acid. In this method containing the additional step, the product polyunsaturated fatty acid may be GLA or STA, and the "another" polyunsaturated fatty acid may be DGLA or eicosatetraenoic acid (ETA), respectively. The method containing the additional step may further comprise a step of exposing the another polyunsaturated fatty acid to an additional desaturase in order to convert the another polyunsaturated fatty acid to a final polyunsaturated fatty acid. The final polyunsaturated fatty acid may be AA or EPA.

It should also be noted that each nucleotide and amino acid sequence referred to herein has been assigned a particular sequence identification number. The Sequence Listing (which is found herein) lists each such sequence and its corresponding number.

BRIEF DESCRIPTION OF THE DRAWINGS

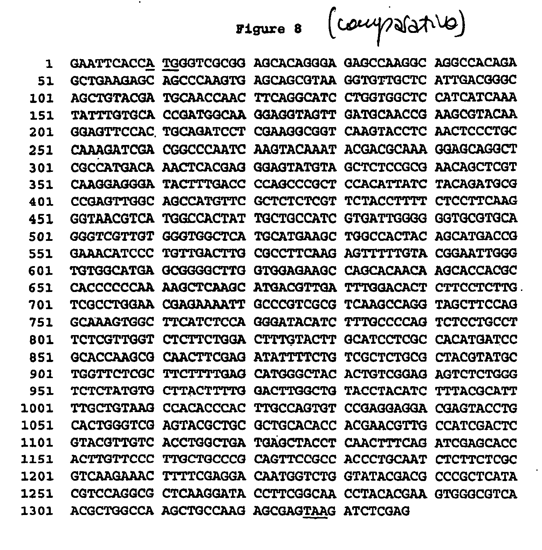

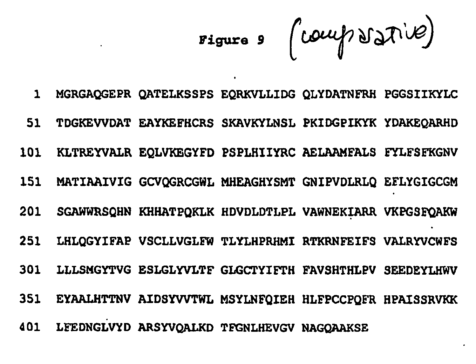

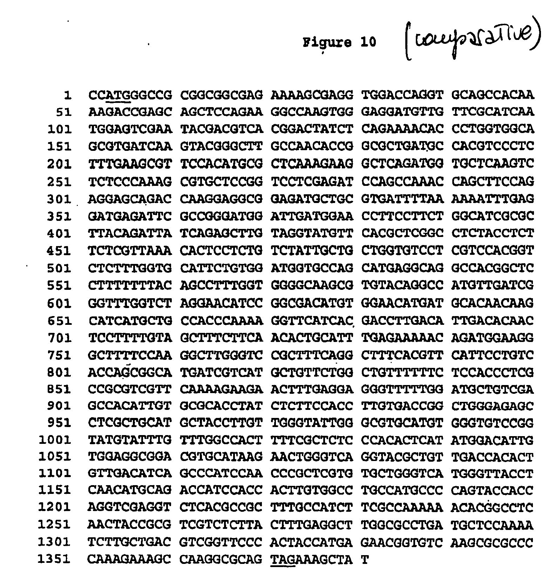

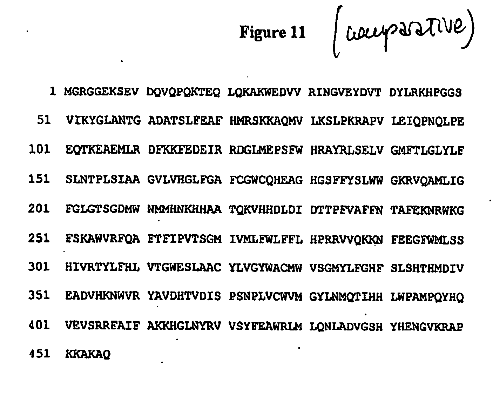

Figures 6-15 are not part of the present invention. Figure 1 illustrates the fatty acid biosynthetic pathway and the roles of Δ5-desaturase and Δ6-desaturase in this pathway.

Figure 2 illustrates the nucleotide sequence encoding Δ6-desaturase of Saprolegnia diclina (ATCC 56851)(SEQ ID NO:13).

Figure 3 illustrates the amino acid sequence of Δ6-desaturase of Saprolegnia diclina (ATCC 56851)(SEQ ID NO:14).

Figure 4 illustrates the nucleotide sequence encoding Δ5-desaturase of Saprolegnia diclina (ATCC 56851) (SEQ ID NO:19).

Figure 5 illustrates the amino acid sequence of Δ5-desaturase of Saprolegnia diclina (ATCC 56851)(SEQ ID NO:20).

Figure 6 illustrates the nucleotide sequence encoding Δ5-desaturase of Thraustochytrium aureum (ATCC 34304) (SEQ ID NO:28).

Figure 7 illustrates the amino acid sequence of Δ5-desaturase of Thraustochytrium aureum (ATCC 34304)(SEQ ID NO:29).

Figure 8 illustrates the nucleotide sequence encoding Δ5-desaturase from Thraustochytrium aureum (BICC7091) (SEQ ID NO:30).

Figure 9 illustrates the translated amino acid sequence of Δ5-desaturase from Thraustochytrium aureum (HICC7091) (SEQ ID NO:31).

Figure 10 illustrates the nucleotide sequence encoding Δ6-desaturase from Thraustochytrium aureum (BICC7091) (SEQ ID NO:32).

Figure 11 illustrates the translated amino acid sequence of Δ6-desaturase from Thraustochytrium aureum (BICC7091)(SEQ ID NO:33).

Figure 12 illustrates the Δ5-desaturase amino acid sequence identity between pRAT-2a and pRAT-2c clones.

Figure 13 illustrates the Δ6-desaturase amino acid sequence identity between pRAT-1a and pRAT-1b clones.

Figure 14 illustrates the nucleotide sequence encoding Δ5-desaturase gene from Isochrysis galbana CCMP1323 (SEQ ID NO:34).

Figure 15 illustrates the translated amino acid sequence from Δ5-desaturase from Isochrysis galbana CCMP1323 (SEQ ID NO:35).

DETAILED DESCRIPTION OF THE INVENTION

The subject invention relates to the nucleotide and translated amino acid sequences of a Δ5-desaturase gene derived from Saprolegnia diclina and a Δ6-desaturase gene derived from Saprolegnia diclina. Furthermore, the subject invention also includes uses of these genes and of the enzymes encoded by these genes. For example, the genes and corresponding enzymes may be used in the production of polyunsaturated fatty acids such as, for instance, arachidonic acid, eicosapentaenoic acid, and/or adrenic acid which may be added to pharmaceutical compositions, nutritional compositions and to other valuable products.

The Δ5-Desaturase Genes, The Δ6-Desaturase Genes, and Enzymes Encoded Thereby

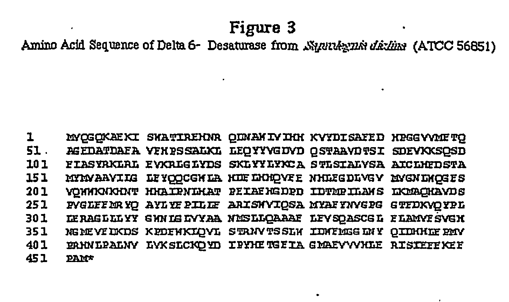

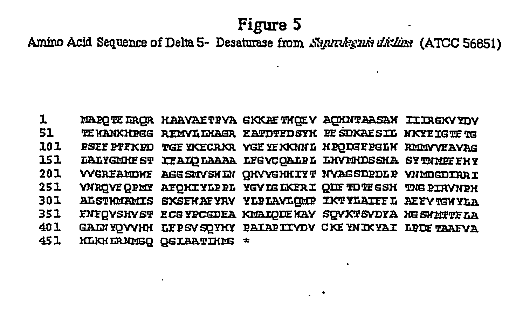

As noted above, the enzymes encoded by the Δ5-desaturase genes and the Δ6-desaturase genes of the present invention are essential in the production of highly unsaturated polyunsaturated fatty acids having a length greater than 20 and 18 carbons, respectively. The nucleotide sequence of the isolated Saprolegnia diclina Δ5-desaturase gene is shown in Figure 4, and the amino acid sequence of the corresponding purified protein is shown in Figure 5. The nucleotide sequence of the isolated Saprolegnia diclina Δ6-desaturase gene is shown in Figure 2, and the amino acid sequence of the corresponding purified protein is shown in Figure 3.

As an example of the importance of the genes of the present invention, the isolated Δ5-desaturase genes convert DGLA to AA or convert eicosatetraenoic acid to EPA. AA, for example, cannot be synthesized without the Δ5-desaturase genes and enzymes encoded thereby. The isolated Δ6-desaturase gene of the present invention converts, for example, linoleic acid (18:2n-6) to γ-linoleic acid (GLA) and γ-linolenic acid (GLA) to stearidonic acid (STA).

It should be noted that the present invention also encompasses nucleotide sequences (and the corresponding encoded proteins) having sequences comprising or complementary to at least 90% of the nucleotides in sequence (i.e., having sequence identity) to SEQ ID NO:19 (i.e., the nucleotide sequence of the Δ5-desaturase gene of Saprolegnia diclina), SEQ ID NO:13 (i.e., the nucleotide sequence of the Δ6-desaturase gene of saprolegnia diclina. (All integers between 90% and 100% are also considered to be within the scope of the present invention with respect to percent identity.) Such sequences may be derived from human sources as well as other non-human sources (e.g., C. elegans or mouse).

The invention also includes a purified polypeptide which desaturates polyunsaturated fatty acids at the carbon 5 position or carbon 6 position and has at least 90% amino acid identity to the amino acid sequences (i.e., SEQ ID NO: 14 (shown in Figure 3) and SEQ ID NO:20 (shown in Figure 5)) of the above-noted proteins which are, in turn, encoded by the above-described nucleotide sequences. All integers between 90-100% similarity or identity are also included within the scope of the invention.

The term "identity" refers to the relatedness of two sequences on a nucleotide-by-nucleotide basis over a particular comparison window or segment. Thus, identity is defined as the degree of sameness, correspondence or equivalence between the same strands (either sense or antisense) of two DNA segments. "Percentage of sequence identity" is calculated by comparing two optimally aligned sequences over a particular region, determining the number of positions at which the identical base occurs in both sequence in order to yield the number of matched positions, dividing the number of such positions by the total number of positions in the segment being compared and multiplying the result by 100. Optimal alignment of sequences may be conducted by the algorithm of Smith & Waterman, Appl. Math. 2:482 (1981), by the algorithm of Needleman & Wunsch, J. Mol. Biol. 48:443 (1970), by the method of Pearson & Lipman, Proc. Natl. Acad. Sci. (USA) 85:2444 (1988) and by computer programs which implement the relevant algorithms (e.g., Clustal Macaw Pileup (http://cmgm.stanford.edu/ biochem218/11Multiple.pdf; Higgins et al., CABIOS. 5L151-153 (1989)), FASTDB (Intelligenetics), BLAST (National Center for Biomedical Information; Altschul et al., Nucleic Acids Research 25:3389-3402 (1997)), PILEUP (Genetics Computer Group, Madison, WI) or GAP, BESTFIT, FASTA and TFASTA (Wisconsin Genetics Software Package Release 7.0, Genetics Computer Group, Madison, WI). (See U.S. Patent No. 5,912,120.)

For purposes of the present invention, "complementarity is defined as the degree of relatedness between two DNA segments. It is determined by measuring the ability of the sense strand of one DNA segment to hybridize with the antisense strand of the other DNA segment, under appropriate conditions, to form a double helix. In the double helix, adenine appears in one strand, thymine appears in the other strand. Similarly, wherever guanine is found in one strand, cytosine is found in the other. The greater the relatedness between the nucleotide sequences of two DNA segments, the greater the ability to form hybrid duplexes between the strands of the two DNA segments.

"Similarity" between two amino acid sequences is defined as the presence of a series of identical as well as conserved amino acid residues in both sequences. The higher the degree of similarity between two amino acid sequences, the higher the correspondence, sameness or equivalence of the two sequences. ("Identity between two amino acid sequences is defined as the presence of a series of exactly alike or invariant amino acid residues in both sequences.) The definitions of "complementarity", "identity" and "similarity" are well known to those of ordinary skill in the art.

"Encoded by" refers to a nucleic acid sequence which codes for a polypeptide sequence, wherein the polypeptide sequence or a portion thereof contains an amino acid sequence of at least 3 amino acids, more preferably at least 8 amino acids, and even more preferably at least 15 amino acids from a polypeptide encoded by the nucleic acid sequence.

The present invention also encompasses an isolated nucleotide sequence which encodes PUFA desaturase activity and that is hybridizable, under moderately stringent conditions, to a nucleic acid having a nucleotide sequence comprising or complementary to the nucleotide sequence comprising SEQ ID NO:13 (shown in Figure 2), SEQ ID NO: 19 (shown in Figure 4). A nucleic acid molecule is "hybridizable" to another nucleic acid molecule when a single-stranded form of the nucleic acid molecule can anneal to the other nucleic acid molecule under the appropriate conditions of temperature and ionic strength (see Sambrook et al., "Molecular Cloning: A Laboratory Manual, Second Edition (1989), Cold Spring Harbor Laboratory Press, Cold Spring Harbor, New York)). The conditions of temperature and ionic strength determine the "stringency" of the hybridization. "Hybridization" requires that two nucleic acids contain complementary sequences. However, depending on the stringency of the hybridization, mismatches between bases may occur. The appropriate stringency for hybridizing nucleic acids depends on the length of the nucleic acids and the degree of complementation. Such variables are well known in the art. More specifically, the greater the degree of similarity or homology between two nucleotide sequences, the greater the value of Tm for hybrids of nucleic acids having those sequences. For hybrids of greater than 100 nucleotides in length, equations for calculating Tm have been derived (see Sambrook et al., supra). For hybridization with shorter nucleic acids, the position of mismatches becomes more important, and the length of the oligonucleotide determines its specificity (see Sambrook et al., *supra*).

As used herein, an "isolated nucleic acid fragment or sequence" is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA. (A "fragment" of a specified polynucleotide refers to a polynucleotide sequence which comprises a contiguous sequence of approximately at least about 6 nucleotides, preferably at least about 8 nucleotides, more preferably at least about 10 nucleotides, and even more preferably at least about 15 nucleotides, and most preferable at least about 25 nucleotides identical or complementary to a region of the specified nucleotide sequence.) Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The terms "fragment or subfragment that is functionally equivalent" and "functionally equivalent fragment or subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric constructs to produce the desired phenotype in a transformed plant. Chimeric constructs can be designed for use in co-suppression or antisense by linking a nucleic acid fragment or subfragment thereof, whether or not it encodes an active enzyme, in the appropriate orientation relative to a plant promoter sequence.

The terms "homology", "homologous", "substantially similar" and " corresponding substantially " are used interchangeably herein. They refer to nucleic acid fragments wherein changes in one or more nucleotide bases does not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

"Gene" refers to a nucleic acid fragment that expresses a specific protein, including regulatory sequences preceding (5' non-coding sequences) and following (3' non-coding sequences) the coding sequence.

"Native gene" refers to a gene as found in nature with its own regulatory sequences. In contrast, "chimeric construct" refers to a combination of nucleic acid fragments that are not normally found together in nature. Accordingly, a chimeric construct may comprise regulatory sequences and coding sequences that are derived from different sources, or regulatory sequences and coding sequences derived from the same source, but arranged in a manner different than that normally found in nature. (The term "isolated" means that the sequence is removed from its natural environment.)

A "foreign" gene refers to a gene not normally found in the host organism, but that is introduced into the host organism by gene transfer. Foreign genes can comprise native genes inserted into a non-native organism, or chimeric constructs. A "transgene" is a gene that has been introduced into the genome by a transformation procedure.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue-specificity of a promoter. Promoter sequences can also be located within the transcribed portions of genes, and/or downstream of the transcribed sequences. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters". New promoters of various types useful in plant cells are constantly being discovered; numerous examples may be found in the compilation by Okamuro and Goldberg, (1989) Biochemistry of Plants 15:1-82. It is further recognized that since in most cases the exact boundaries of regulatory sequences have not been completely defined, DNA fragments of some variation may have identical promoter activity.

An "intron" is an intervening sequence in a gene that does not encode a portion of the protein sequence. Thus, such sequences are transcribed into RNA but are then excised and are not translated. The term is also used for the excised RNA sequences. An "exon" is a portion of the sequence of a gene that is transcribed and is found in the mature messenger RNA derived from the gene, but is not necessarily a part of the sequence that encodes the final gene product.

The "translation leader sequence" refers to a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. (1995) Molecular Biotechnology 3:225).

The "3' non-coding sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA (mRNA) " refers to the RNA that is without introns and that can be translated into protein by the cell. "cDNA" refers to a DNA that is complementary to and synthesized from a mRNA template using the enzyme reverse transcriptase. The cDNA can be single-stranded or converted into the double-stranded form using the Klenow fragment of DNA polymerase I. "Sense" RNA refers to RNA transcript that includes the mRNA and can be translated into protein within a cell or in vitro. "Antisense RNA" refers to an RNA transcript that is complementary to all or part of a target primary transcript or mRNA and that blocks the expression of a target gene (U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "endogenous RNA" refers to any RNA which is encoded by any nucleic acid sequence present in the genome of the host prior to transformation with the recombinant construct of the present invention, whether naturally-occurring or non-naturally occurring, i.e., introduced by recombinant means, mutagenesis, etc.

The term "non-naturally occurring" means artificial, not consistent with what is normally found in nature.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA, or 3' to the target mRNA, or within the target mRNA, or a first complementary region is 5' and its complement is 3' to the target mRNA.

The term "expression", as used herein, refers to the production of a functional end-product. Expression of a gene involves transcription of the gene and translation of the mRNA into a precursor or mature protein. "Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020).

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms. The preferred method of cell transformation of rice, corn and other monocots is the use of particle-accelerated or "gene gun" transformation technology (Klein et al., (1987) Nature (London) 327:70-73; U.S. Patent No. 4,945,050), or an Agrobacterium-mediated method using an appropriate Ti plasmid containing the transgene (Ishida Y. et al., 1996, Nature Biotech. 14:745-750). The term "transformation" as used herein refers to both stable transformation and transient transformation.

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor, 1989 (hereinafter "Sambrook").

The term "recombinant" refers to an artificial combination of two otherwise separated segments of sequence, e.g., by chemical synthesis or by the manipulation of isolated segments of nucleic acids by genetic engineering techniques.

"PCR" or "Polymerase Chain Reaction" is a technique for the synthesis of large quantities of specific DNA segments, consists of a series of repetitive cycles (Perkin Elmer Cetus Instruments, Norwalk, CT). Typically, the double stranded DNA is heat denatured, the two primers complementary to the 3' boundaries of the target segment are annealed at low temperature and then extended at an intermediate temperature. One set of these three consecutive steps is referred to as a cycle.

Polymerase chain reaction ("PCR") is a powerful technique used to amplify DNA millions of fold, by repeated replication of a template, in a short period of time. (Mullis et al, Cold Spring Harbor Symp. Quant. Biol. 51:263-273 (1986); Erlich et al, European Patent Application 50,424; European Patent Application 84,796; European Patent Application 258,017, European Patent Application 237,362; Mullis, European Patent Application 201,184, Mullis et al U.S. Patent No. 4,683,202; Erlich, U.S. Patent No. 4,582,788; and Saiki et al, U.S. Patent No. 4,683,194). The process utilizes sets of specific in vitro synthesized oligonucleotides to prime DNA synthesis. The design of the primers is dependent upon the sequences of DNA that are desired to be analyzed. The technique is carried out through many cycles (usually 20-50) of melting the template at high temperature, allowing the primers to anneal to complementary sequences within the template and then replicating the template with DNA polymerase.

The products of PCR reactions are analyzed by separation in agarose gels followed by ethidium bromide staining and visualization with UV transillumination. Alternatively, radioactive dNTPs can be added to the PCR in order to incorporate label into the products. In this case the products of PCR are visualized by exposure of the gel to x-ray film. The added advantage of radiolabeling PCR products is that the levels of individual amplification products can be quantitated.

The terms "recombinant construct", "expression construct" and "recombinant expression construct" are used interchangeably herein. These terms refer to a functional unit of genetic material that can be inserted into the genome of a cell using standard methodology well known to one skilled in the art. Such construct may be itself or may be used in conjunction with a vector. If a vector is used then the choice of vector is dependent upon the method that will be used to transform host plants as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

Production of the Δ5-Desaturase Enzymes and the Δ6-Desaturase Enzymes

Once the gene encoding any one of the desaturase enzymes has been isolated, it may then be introduced into either a prokaryotic or eukaryotic host cell through the use of a vector or construct. The vector, for example, a bacteriophage, cosmid or plasmid, may comprise the nucleotide sequence encoding either of the Δ5-desaturase enzymes, or the Δ6-desaturase enzyme, as well as any regulatory sequence (e.g., promoter) which is functional in the host cell and is able to elicit expression of the desaturase encoded by the nucleotide sequence. The regulatory sequence is in operable association with or operably linked to the nucleotide sequence. (As noted above, regulatory is said to be "operably linked" with a coding sequence if the regulatory sequence affects transcription or expression of the coding sequence.) Suitable promoters include, for example, those from genes encoding alcohol dehydrogenase, glyceraldehyde-3-phosphate dehydrogenase, phosphoglucoisomerase, phosphoglycerate kinase, acid phosphatase, T7, TPI, lactase, metallothionein, cytomegalovirus immediate early, whey acidic protein, glucoamylase, and promoters activated in the presence of galactose, for example, GAL1 and GAL10. Additionally, nucleotide sequences which encode other proteins, oligosaccharides, lipids, etc. may also be included within the vector as well as other regulatory sequences such as a polyadenylation signal (e.g., the poly-A signal of SV-40T-antigen, ovalalbumin or bovine growth hormone). The choice of sequences present in the construct is dependent upon the desired expression products as well as the nature of the host cell.

As noted above, once the vector has been constructed, it may then be introduced into the host cell of choice by methods known to those of ordinary skill in the art including, for example, transfection, transformation and electroporation (see Molecular Cloning: A Laboratory Manual, 2nd ed., Vol. 1-3, ed. Sambrook et al., Cold Spring Harbor Laboratory Press (1989)). The host cell is then cultured under suitable conditions permitting expression of the genes leading to the production of the desired PUFA, which is then recovered and purified.

Examples of suitable prokaryotic host cells include, for example, bacteria such as Escherichia coli, Bacillus subtilis as well as cyanobacteria such as Spirulina spp. (i.e., blue-green algae). Examples of suitable eukaryotic host cells include, for example, mammalian cells, plant cells, yeast cells such as Saccharomyces cerevisiae, Saccharomyces carlsbergensis, Lipomyces starkey, Candida spp. such as Yarrowia (Candida) lipolytica, Kluyveromyces spp., Pichia spp., Trichoderma spp. or Hansenula spp., or fungal cells such as filamentous fungal cells, for example, Aspergillus, Neurospora and Penicillium. Preferably, Saccharomyces cerevisiae (baker's yeast) cells are utilized.

Expression in a host cell can be accomplished in a transient or stable fashion. Transient expression can occur from introduced constructs which contain expression signals functional in the host cell, but which constructs do not replicate and rarely integrate in the host cell, or where the host cell is not proliferating. Transient expression also can be accomplished by inducing the activity of a regulatable promoter operably linked to the gene of interest, although such inducible systems frequently exhibit a low basal level of expression. Stable expression can be achieved by introduction of a construct that can integrate into the host genome or that autonomously replicates in the host cell. Stable expression of the gene of interest can be selected for through the use of a selectable marker located on or transfected with the expression construct, followed by selection for cells expressing the marker. When stable expression results from integration, the site of the construct's integration can occur randomly within the host genome or can be targeted through the use of constructs containing regions of homology with the host genome sufficient to target recombination with the host locus. Where constructs are targeted to an endogenous locus, all or some of the transcriptional and translational regulatory regions can be provided by the endogenous locus.

A transgenic non-human mammal may also be used in order to express the enzyme(s) of interest (i.e., one or more of the Δ5-desaturases, one or more of the Δ6-desaturases, or a combination thereof), and ultimately the PUFA(s) of interest. More specifically, once the above-described construct is created, it may be inserted into the pronucleus of an embryo. The embryo may then be implanted into a recipient female. Alternatively, a nuclear transfer method could also be utilized (Schnieke et al., Science 278:2130-2133 (1997)). Gestation and birth are then permitted (see, e.g., U.S. Patent No. 5,750,176 and U.S. Patent No. 5,700,671). Milk, tissue or other fluid samples from the offspring should then contain altered levels of PUFAs, as compared to the levels normally found in the non-transgenic animal. Subsequent generations may be monitored for production of the altered or enhanced levels of PUFAs and thus incorporation of the gene encoding the desired desaturase enzyme into their genomes. The non-human mammal utilized as the host may be selected from the group consisting of, for example, a mouse, a rat, a rabbit, a pig, a goat, a sheep, a horse and a cow. However, any non-human mammal may be used provided it has the ability to incorporate DNA encoding the enzyme of interest into its genome.

For expression of a desaturase polypeptide, functional transcriptional and translational initiation and termination regions are operably linked to the DNA encoding the desaturase polypeptide. Transcriptional and translational initiation and termination regions are derived from a variety of nonexclusive sources, including the DNA to be expressed, genes known or suspected to be capable of expression in the desired system, expression vectors, chemical synthesis, or from an endogenous locus in a host cell. Expression in a plant tissue and/or plant part presents certain efficiencies, particularly where the tissue or part is one which is harvested early, such as seed, leaves, fruits, flowers, roots, etc. Expression can be targeted to that location with the plant by utilizing specific regulatory sequence such as those of U.S. Patent Nos. 5,463,174, 4,943,674, 5,106,739, 5,175,095, 5,420,034, 5,188,958, and 5,589,379. Alternatively, the expressed protein can be an enzyme which produces a product which may be incorporated, either directly or upon further modifications, into a fluid fraction from the host plant. Expression of a desaturase gene, or antisense desaturase transcripts, can alter the levels of specific PUFAs, or derivatives thereof, found in plant parts and/or plant tissues. The desaturase polypeptide coding region may be expressed either by itself or with other genes, in order to produce tissues and/or plant parts containing higher proportions of desired PUFAs or in which the PUFA composition more closely resembles that of human breast milk (Prieto et al., PCT publication WO 95/24494). The termination region may be derived from the 3' region of the gene from which the initiation region was obtained or from a different gene. A large number of termination regions are known to and have been found to be satisfactory in a variety of hosts from the same and different genera and species. The termination region usually is selected as a matter of convenience rather than because of any particular property.

As noted above, a plant (e.g., Glycine max (soybean) or Brassica napus (canola)) or plant tissue may also be utilized as a host or host cell, respectively, for expression of the desaturase enzyme which may, in turn, be utilized in the production of polyunsaturated fatty acids. More specifically, desired PUFAS can be expressed in seed. Methods of isolating seed oils are known in the art. Thus, in addition to providing a source for PUFAs, seed oil components may be manipulated through the expression of the desaturase gene, as well as perhaps other desaturase genes and elongase genes, in order to provide seed oils that can be added to nutritional compositions, pharmaceutical compositions, animal feeds and cosmetics. Once again, a vector which comprises a DNA sequence encoding the desaturase operably linked to a promoter, will be introduced into the plant tissue or plant for a time and under conditions sufficient for expression of the desaturase gene. The vector may also comprise one or more genes that encode other enzymes, for example, Δ4-desaturase, elongase, Δ12-desaturase, Δ15-desaturase, Δ17-desaturase, and/or Δ19-desaturase. The plant tissue or plant may produce the relevant substrate (e.g., DGLA (in the case of Δ5-desaturase), ALA (in the case of Δ6-desaturase), etc.) upon which the enzymes act or a vector encoding enzymes which produce such substrates may be introduced into the plant tissue, plant cell or plant. In addition, substrate may be sprayed on plant tissues expressing the appropriate enzymes. Using these various techniques, one may produce PUFAs (e.g., n-6 unsaturated fatty acids such as AA, or n-3 fatty acids such as EPA or STA) by use of a plant cell, plant tissue or plant. It should also be noted that the invention also encompasses a transgenic plant comprising the above-described vector, wherein expression of the nucleotide sequence of the vector results in production of a polyunsaturated fatty acid in, for example, the seeds of the transgenic plant.

The regeneration, development, and cultivation of plants from single plant protoplast transformants or from various transformed explants is well known in the art (Weissbach and Weissbach, In: Methods for Plant Molecular Biology, (Eds.), Academic Press, Inc. San Diego, CA, (1988)). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil.

The development or regeneration of plants containing the foreign, exogenous gene that encodes a protein of interest is well known in the art. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant of the present invention containing a desired polypeptide is cultivated using methods well known to one skilled in the art.

There are a variety of methods for the regeneration of plants from plant tissue. The particular method of regeneration will depend on the starting plant tissue and the particular plant species to be regenerated.

Methods for transforming dicots, primarily by use of *Agrobacterium tumefaciens,* and obtaining transgenic plants have been published for cotton (U.S. Patent No. 5,004,863, U.S. Patent No. 5,159,135, U.S. Patent No. 5,518, 908); soybean (U.S. Patent No. 5,569,834, U.S. Patent No. 5,416,011, McCabe et. al., BiolTechnology 6:923 (1988), Christou et al., Plant Physiol. 87:671-674 (1988)); *Brassica* (U.S. Patent No. 5,463,174); peanut (Cheng et al., Plant Cell Rep. 15:653-657 (1996), McKently et al., Plant Cell Rep. 14:699-703 (1995)); papaya; and pea (Grant et al., Plant Cell Rep. 15:254-258, (1995)).

Transformation of monocotyledons using electroporation, particle bombardment, and Agrobacterium have also been reported. Transformation and plant regeneration have been achieved in asparagus (Bytebier et al., Proc. Natl. Acad. Sci. (USA) 84:5354, (1987)); barley (Wan and Lemaux, Plant Physiol 104:37 (1994)); *Zea mays* (Rhodes et al., Science 240:204 (1988), Gordon-Kamm et al., Plant Cell 2:603-618 (1990), Fromm et al., BiolTechnology 8:833 (1990), Koziel et al., BiolTechnology 11: 194, (1993), Armstrong et al., Crop Science 35:550-557 (1995)); oat (Somers et al., BiolTechnology 10: 15 89 (1992)); orchard grass (Horn et al., Plant Cell Rep. 7:469 (1988)); rice (Toriyama et al., TheorAppl. Genet. 205:34, (1986); Part et al., Plant Mol. Biol. 32:1135-1148, (1996); Abedinia et al., Aust. J. Plant Physiol. 24:133-141 (1997); Zhang and Wu, Theor. Appl. Genet. 76:835 (1988); Zhang et al. Plant Cell Rep. 7:379, (1988); Battraw and Hall, Plant Sci. 86:191-202 (1992); Christou et al., Bio/Technology 9:957 (1991)) ; rye (De la Pena et al., Nature 325:274 (1987)) ; sugarcane (Bower and Birch, Plant J. 2:409 (1992)); tall fescue (Wang et al., BiolTechnology 10:691 (1992)), and wheat (Vasil et al., Bio/Technology 10:667 (1992); U.S. Patent No. 5, 631, 152).

Assays for gene expression based on the transient expression of cloned nucleic acid constructs have been developed by introducing the nucleic acid molecules into plant cells by polyethylene glycol treatment, electroporation, or particle bombardment (Marcotte et al., Nature 335:454-457 (1988); Marcotte et al., Plant Cell 1:523-532 (1989); McCarty et al., Cell 66:895-905 (1991); Hattori et al., Genes Dev. 6:609-618 (1992); Goff et al., EMBO J. 9:2517-2522 (1990)).

Transient expression systems may be used to functionally dissect gene constructs (see generally, Maliga et al., Methods in Plant Molecular Biology, Cold Spring Harbor Press (1995)). It is understood that any of the nucleic acid molecules of the present invention can be introduced into a plant cell in a permanent or transient manner in combination with other genetic elements such as vectors, promoters, enhancers etc.

In addition to the above discussed procedures, practitioners are familiar with the standard resource materials which describe specific conditions and procedures for the construction, manipulation and isolation of macromolecules (e.g., DNA molecules, plasmids, etc.), generation of recombinant organisms and the screening and isolating of clones, (see for example, Sambrook et al., Molecular Cloning: A Laboratory Manual, Cold Spring Harbor Press (1989); Maliga et al., Methods in Plant Molecular Biology, Cold Spring Harbor Press (1995); Birren et al., Genome Analysis: Detecting Genes, 1, Cold Spring Harbor, New York (1998); Birren et al., Genome Analysis: Analyzing DNA, 2, Cold Spring Harbor, New York (1998); Plant Molecular Biology: A Laboratory Manual, eds. Clark, Springer, New York (1997)).

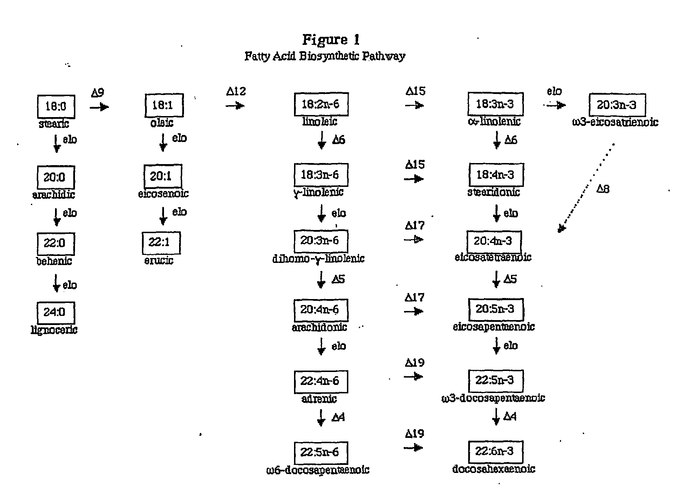

The substrates which may be produced by the host cell either naturally or transgenically, as well as the enzymes which may be encoded by DNA sequences present in the vector which is subsequently introduced into the host cell, are shown in Figure 1.

In view of the above, the present invention encompasses a method of producing the desaturase enzymes (i.e., Δ5 or Δ6) comprising the steps of: 1) isolating the nucleotide sequence of the gene encoding the desaturase enzyme; 2) constructing a vector comprising said nucleotide sequence operably linked to a regulatory sequence and 3) introducing said vector into a host cell under time and conditions sufficient for the production of the desaturase enzyme.

With regard to desaturases polyunsaturated fatty acids may be produced by exposing an acid to the enzyme such that the desaturase converts the acid to a polyunsaturated fatty acid. For example, when 20:3n-6 is exposed to a Δ5-desaturase enzyme, it is converted to AA. AA may then be exposed to elongase which elongates the AA to adrenic acid (i.e., 22:4n-6). Alternatively, Δ5-desaturase may be utilized to convert 20:4n-3 to 20:5n-3 which may be exposed to elongase and converted to (n-3)-docosapentaenoic acid. The (n-3)-docosapentaenoic acid may then be converted to DHA by use of Δ4-desaturase. Thus, Δ5-desaturase may be used in the production of polyunsaturated fatty acids which may be used, in turn, for particular beneficial purposes.

With respect to the role of Δ6-desaturase, linoleic acid may be exposed to the enzyme such that the enzyme converts the acid to GLA. An elongase may then be used to convert the GLA to DGLA. The DGLA then may be converted to AA by exposing the DGLA to a Δ5-desaturase. As another example, ALA may be exposed to a Δ6-desaturase in order to convert the ALA to STA. The STA may then be converted to 20:4n-3 by using an elongase. Subsequently, the 20:

4n-3 may be converted to EPA by exposing the 20:4n-3 to a Δ5-desaturase. Thus, the Δ6-desaturase may be used in the production of PUFAs which have may advantageous properties or may be used in the production of other PUFAs.

Uses of the Δ5-Desaturases Genes, the Δ6-Desaturase Genes, and Enzymes Encoded Thereby

As noted above, the isolated desaturase genes and the desaturase enzymes encoded thereby have many uses. For example, the gene and corresponding enzyme may be used indirectly or directly in the production of polyunsaturated fatty acids, for example, Δ5-desaturase may be used in the production of AA, adrenic acid or EPA. Delta-6 desaturase may be used either indirectly or directly in the production of GLA, DGLA, STA or 20:4n-3. ("Directly" is meant to encompass the situation where the enzyme directly converts the acid to another acid, the latter of which is utilized in a composition (e.g., the conversion of DGLA to AA). "Indirectly" is meant to encompass the situation where an acid is converted to another acid (i.e., a pathway intermediate) by the desaturase (e.g., DGLA to AA) and then the latter acid is converted to another acid by use of a non-desaturase enzyme (e.g., AA to adrenic acid by elongase or by use of another desaturase enzyme (e.g., AA to EPA by Δ17-desaturase.)). These polyunsaturated fatty acids (i.e., those produced either directly or indirectly by activity of the desaturase enzyme) may be added to, for example, nutritional compositions, pharmaceutical compositions, cosmetics, and animal feeds, all of which are encompassed by the present invention. The nutritional compositions, pharmaceutical compositions and veterinary applications described in the following paragraphs do not form part of the claimed invention.

Nutritional Compositions

The present invention includes nutritional compositions. Such compositions, for purposes of the present invention, include any food or preparation for human consumption including for enteral or parenteral consumption, which when taken into the body (a) serve to nourish or build up tissues or supply energy and/or (b) maintain, restore or support adequate nutritional status or metabolic function.

The nutritional composition of the present invention comprises at least one oil or acid produced directly or indirectly by use of the desaturase gene, in accordance with the present invention, and may either be in a solid or liquid form. Additionally, the composition may include edible macronutrients, vitamins and minerals in amounts desired for a particular use. The amount of such ingredients will vary depending on whether the composition is intended for use with normal, healthy infants, children or adults having specialized needs such as those which accompany certain metabolic conditions (e.g., metabolic disorders).

Examples of macronutrients which may be added to the composition include but are not limited to edible fats, carbohydrates and proteins. Examples of such edible fats include but are not limited to coconut oil, soy oil, and mono- and diglycerides. Examples of such carbohydrates include but are not limited to glucose, edible lactose and hydrolyzed search. Additionally, examples of proteins which may be utilized in the nutritional composition of the invention include but are not limited to soy proteins, electrodialysed whey, electrodialysed skim milk, milk whey, or the hydrolysates of these proteins.

With respect to vitamins and minerals, the following may be added to the nutritional compositions of the present invention: calcium, phosphorus, potassium, sodium, chloride, magnesium, manganese, iron, copper, zinc, selenium, iodine, and Vitamins A, E, D, C, and the B complex. Other such vitamins and minerals may also be added.

The components utilized in the nutritional compositions of the present invention will be of semi-purified or purified origin. By semi-purified or purified is meant a material which has been prepared by purification of a natural material or by synthesis.

Examples of nutritional compositions of the present invention include but are not limited to infant formulas, dietary supplements, dietary substitutes, and rehydration compositions. Nutritional compositions of particular interest include but are not limited to those utilized for enteral and parenteral supplementation for infants, specialist infant formulas, supplements for the elderly, and supplements for those with gastrointestinal difficulties and/or malabsorption.

The nutritional composition of the present invention may also be added to food even when supplementation of the diet is not required. For example, the composition may be added to food of any type including but not limited to margarines, modified butters, cheeses, milk, yogurt, chocolate, candy, snacks, salad oils, cooking oils, cooking fats, meats, fish and beverages.

In a preferred embodiment of the present invention, the nutritional composition is an enteral nutritional product, more preferably, an adult or pediatric enteral nutritional product. This composition may be administered to adults or children experiencing stress or having specialized needs due to chronic or acute disease states. The composition may comprise, in addition to polyunsaturated fatty acids produced in accordance with the present invention, macronutrients, vitamins and minerals as described above. The macronutrients may be present in amounts equivalent to those present in human milk or on an energy basis, i.e., on a per calorie basis.

Methods for formulating liquid or solid enteral and parenteral nutritional formulas are well known in the art.

(See also the Examples below.)

The enteral formula, for example, may be sterilized and subsequently utilized on a ready-to-feed (RTF) basis or stored in a concentrated liquid or powder. The powder can be prepared by spray drying the formula prepared as indicated above, and reconstituting it by rehydrating the concentrate. Adult and pediatric nutritional formulas are well known in the art and are commercially available (e.g., Similac®, Ensure®, Jevity® and Alimentum® from Ross Products Division, Abbott Laboratories, Columbus, Ohio). An oil or acid produced in accordance with the present invention may be added to any of these formulas.

The energy density of the nutritional compositions of the present invention, when in liquid form, may range from about 0.6 Kcal to about 3 Kcal per ml. When in solid or powdered form, the nutritional supplements may contain from about 1.2 to more than 9 Kcals per gram, preferably about 3 to 7 Kcals per gm. In general, the osmolality of a liquid product should be less than 700 mOsm and, more preferably, less than 660 mOsm.

The nutritional formula may include macronutrients, vitamins, and minerals, as noted above, in addition to the PUFAs produced in accordance with the present invention. The presence of these additional components helps the individual ingest the minimum daily requirements of these elements. In addition to the provision of PUFAs, it may also be desirable to add zinc, copper, folic acid and antioxidants to the composition. It is believed that these substance boost a stressed immune system and will therefore provide further benefits to the individual receiving the composition. A pharmaceutical composition may also be supplemented with these elements.

In a more preferred embodiment, the nutritional composition comprises, in addition to antioxidants and at least one PUFA, a source of carbohydrate wherein at least 5 weight percent of the carbohydrate is indigestible oligosaccharide.

In a more preferred embodiment, the nutritional composition additionally comprises protein, taurine, and carnitine.

As noted above, the PUFAs produced in accordance with the present invention, or derivatives thereof, may be added to a dietary substitute or supplement, particularly an infant formula, for patients undergoing intravenous feeding or for preventing or treating malnutrition or other conditions or disease states. As background, it should be noted that human breast milk has a fatty acid profile comprising from about 0.15% to about 0.36% as DHA, from about 0.03% to about 0.13% as EPA, from about 0.30% to about 0.88% as AA, from about 0.22% to about 0.67% as DGLA, and from about 0.27% to about 1.04% as GLA. Thus, fatty acids such as AA, EPA and/or docosahexaenoic acid (DHA), produced in accordance with the present invention, can be used to alter, for example, the composition of infant formulas in order to better replicate the PUFA content of human breast milk or to alter the presence of PUFAs normally found in a non-human mammal's milk. In particular, a composition for use in a pharmacologic or food supplement, particularly a breast milk substitute or supplement, will preferably comprise one or more of AA, DGLA and GLA. More preferably, the oil will comprise from about 0.3 to 30% AA, from about 0.2 to 30% DGLA, and/or from about 0.2 to about 30% GLA.

Parenteral nutritional compositions comprising from about 2 to about 30 weight percent fatty acids calculated as triglycerides are encompassed by the present invention. The preferred composition has about 1 to about 25 weight percent of the total PUFA composition as GLA (U.S. Patent No. 5,196,198). Other vitamins, particularly fat-soluble vitamins such as vitamin A, D, E and L-carnitine can optionally be included. When desired, a preservative such as alpha-tocopherol may be added in an amount of about 0.1% by weight.

In addition, the ratios of AA, DGLA and GLA can be adapted for a particular given end use. When formulated as a breast milk supplement or substitute, a composition which comprises one or more of AA, DGLA and GLA will be provided in a ratio of about 1:19:30 to about 6:1:0.2, respectively. For example, the breast milk of animals can vary in ratios of AA:DGLA:GLA ranging from 1:19:30 to 6:1:0.2, which includes intermediate ratios which are preferably about 1:1:1, 1:2:1, 1:1:4. When produced together in a host cell, adjusting the rate and percent of conversion of a precursor substrate such as GLA and DGLA to AA can be used to precisely control the PUFA ratios. For example, a 5% to 10% conversion rate of DGLA to AA can be used to produce an AA to DGLA ratio of about 1:19, whereas a conversion rate of about 75% TO 80% can be used to produce an AA to DGLA ratio of about 6:1. Therefore, whether in a cell culture system or in a host animal, regulating the timing, extent and specificity of desaturase expression, as well as the expression of other desaturases and elongases, can be used to modulate PUFA levels and ratios. The PUFAs/acids produced in accordance with the present invention (e.g., AA and EPA) may then be combined with other PUFAs/acids (e.g., GLA) in the desired concentrations and ratios.

Additionally, PUFA produced in accordance with the present invention or host cells containing them may also be used as animal food supplements to alter an animal's tissue or milk fatty acid composition to one more desirable for human or animal consumption.

Pharmaceutical Compositions

The present invention also encompasses a pharmaceutical composition comprising one or more of the acids and/or resulting oils produced using the desaturase genes described herein, in accordance with the methods described herein. More specifically, such a pharmaceutical composition may comprise one or more of the acids and/or oils as well as a standard, well-known, non-toxic pharmaceutically acceptable carrier, adjuvant or vehicle such as, for example, phosphate buffered saline, water, ethanol, polyols, vegetable oils, a wetting agent or an emulsion such as a water/oil emulsion. The composition may be in either a liquid or solid form. For example, the composition may be in the form of a tablet, capsule, ingestible liquid or powder, injectible, or topical ointment or cream. Proper fluidity can be maintained, for example, by the maintenance of the required particle size in the case of dispersions and by the use of surfactants. It may also be desirable to include isotonic agents, for example, sugars, sodium chloride and the like. Besides such inert diluents, the composition can also include adjuvants, such as wetting agents, emulsifying and suspending agents, sweetening agents, flavoring agents and perfuming agents.

Suspensions, in addition to the active compounds, may comprise suspending agents such as, for example, ethoxylated isostearyl alcohols, polyoxyethylene sorbitol and sorbitan esters, microcrystalline cellulose, aluminum metahydroxide, bentonite, agar-agar and tragacanth or mixtures of these substances.

Solid dosage forms such as tablets and capsules can be prepared using techniques well known in the art. For example, PUFAs produced in accordance with the present invention can be tableted with conventional tablet bases such as lactose, sucrose, and cornstarch in combination with binders such as acacia, cornstarch or gelatin, disintegrating agents such as potato starch or alginic acid, and a lubricant such as stearic acid or magnesium stearate. Capsules can be prepared by incorporating these excipients into a gelatin capsule along with antioxidants and the relevant PUFA(s). The antioxidant and PUFA components should fit within the guidelines presented above.

For intravenous administration, the PUFAs produced in accordance with the present invention or derivatives thereof may be incorporated into commercial formulations such as Intralipids™. The typical normal adult plasma fatty acid profile comprises 6.64 to 9.46% of AA, 1.45 to 3.11% of DGLA, and 0.02 to 0.08% of GLA. These PUFAs or their metabolic precursors can be administered alone or in combination with other PUFAs in order to achieve a normal fatty acid profile in a patient. Where desired, the individual components of the formulations may be provided individually, in kit form, for single or multiple use. A typical dosage of a particular fatty acid is from 0.1 mg to 20 g (up to 100 g) daily and is preferably from 10 mg to 1, 2, 5 or 10 g daily.

Possible routes of administration of the pharmaceutical compositions of the present invention include, for example, enteral (e.g., oral and rectal) and parenteral. For example, a liquid preparation may be administered, for example, orally or rectally. Additionally, a homogenous mixture can be completely dispersed in water, admixed under sterile conditions with physiologically acceptable diluents, preservatives, buffers or propellants in order to form a spray or inhalant. The route of administration will, of course, depend upon the desired effect. For example, if the composition is being utilized to treat rough, dry, or aging skin, to treat injured or burned skin, or to treat skin or hair affected by a disease or condition, it may perhaps be applied topically.

The dosage of the composition to be administered to the patient may be determined by one of ordinary skill in the art and depends upon various factors such as weight of the patient, age of the patient, immune status of the patient, etc.

With respect to form, the composition may be, for example, a solution, a dispersion, a suspension, an emulsion or a sterile powder which is then reconstituted.

The present invention also includes the treatment of various disorders by use of the pharmaceutical and/or nutritional compositions described herein. In particular, the compositions of the present invention may be used to treat restenosis after angioplasty. Furthermore, symptoms of inflammation, rheumatoid arthritis, asthma and psoriasis may also be treated with the compositions of the invention. Evidence also indicates that PUFAs may be involved in calcium metabolism; thus, the compositions of the present invention may, perhaps, be utilized in the treatment or prevention of osteoporosis and of kidney or urinary tract stones.

Additionally, the compositions of the present invention may also be used in the treatment of cancer. Malignant cells have been shown to have altered fatty acid compositions. Addition of fatty acids has been shown to slow their growth, cause cell death and increase their susceptibility to chemotherapeutic agents. Moreover, the compositions of the present invention may also be useful for treating cachexia associated with cancer.

The compositions of the present invention may also be used to treat diabetes (see U.S. Patent No. 4,826,877 and Horrobin et al., Am. J. Clin. Nutr. Vol. 57 (Suppl.) 732S-737S). Altered fatty acid metabolism and composition have been demonstrated in diabetic animals.

Furthermore, the compositions of the present invention, comprising PUFAs produced either directly or indirectly through the use of the desaturase enzymes, may also be used in the treatment of eczema, in the reduction of blood pressure, and in the improvement of mathematics examination scores. Additionally, the compositions of the present invention may be used in inhibition of platelet aggregation, induction of vasodilation, reduction in cholesterol levels, inhibition of proliferation of vessel wall smooth muscle and fibrous tissue (Brenner et al., Adv. Exp. Med. Biol. Vol. 83, p.85-101, 1976), reduction or prevention of gastrointestinal bleeding and other side effects of non-steroidal anti-inflammatory drugs (see U.S. Patent No. 4,666,701), prevention or treatment of endometriosis and premenstrual syndrome (see U.S. Patent No. 4,758,592), and treatment of myalgic encephalomyelitis and chronic fatigue after viral infections (see U.S. Patent No. 5,116,871).

Further uses of the compositions of the present invention include use in the treatment of AIDS, multiple sclerosis, and inflammatory skin disorders, as well as for maintenance of general health.
Additionally, the composition of the present invention may be utilized for cosmetic purposes. It may be added to pre-existing cosmetic compositions such that a mixture is formed or may be used as a sole composition.

Veterinary Applications

It should be noted that the above-described pharmaceutical and nutritional compositions may be utilized in connection with animals (i.e., domestic or non-domestic), as animals experience many of the same needs and conditions as humans. For example, the oil or acids of the present invention may be utilized in animal or aquaculture feed supplements, animal feed substitutes, animal vitamins or in animal topical ointments.
The present invention may be illustrated by the use of the following non-limiting examples:

Example 1

Design of Degenerate Oligonucleotides for the Isolation of Desaturases from Fungi and cDNA Library Construction

Analysis of the fatty acid composition of *Saprolegnia diclina* (*S.diclina*)(ATCC 56851) revealed the presence of a considerable amount of arachidonic acid (ARA, 20:4 n-6) and eicosapentanoic acid (EPA, 20:5 n-3). Thus, it was thought that this organism contained an active Δ6-desaturase capable of converting linoleic acid (LA, 18:2 n-6) to gamma-linolenic acid (GLA, 18:3 n-6), and an active Δ5-desaturase that would convert dihomo-gamma-linolenic acid (DGLA, 20:3 n-6) to arachidonic acid (ARA, 20:4 n-6) (Figure 1). In addition, it was thought that *S. diclina* also contained a Δ17-desaturase capable of desaturating ARA to EPA.
The goal thus was to attempt to isolate these predicted desaturase genes from *S.diclina* and eventually to verify the functionality by expression in an alternate host.
To isolate genes encoding functional desaturase enzymes, a cDNA library was constructed for S. diclina. Saprolegnia diclina (ATCC 56851) cultures were grown in potato dextrose media Difco # 336 (Difco Laboratories, Detroit, Michigan) at room temperature for 4 days with constant agitation. The mycelia were harvested by filtration through several layers of cheese cloth, and the cultures crushed in liquid nitrogen using a mortar and pestle. Total RNA was purified from it using the Qiagen RNeasy Maxi kit (Qiagen, Valencia, CA) as per manufacturer's protocol.
mRNA was isolated from total RNA from each organism using oligo dT cellulose resin. The pBluescript II XR library construction kit (Stratagene, La Jolla, CA) was then used to synthesize double stranded cDNA which was then directionally cloned (5' *EcoRI*/3' *XhoI*) into pBluescript II SK(+) vector. The *S.diclina* library contained approximately 2.5 x $10^6$ clones with an average insert size of approximately 700 bp. Genomic DNA from PUFA producing cultures of *S.diclina* was isolated by crushing the culture in liquid nitrogen and purified using Qiagen Genomic DNA Extraction Kit (Qiagen, Valencia, CA).
The approach taken was to design degenerate oligonucleotides (i.e., primers) that represent amino acid motifs that are conserved in known desaturases. These primers could be used in a PCR reaction to identify a fragment containing the conserved regions in the predicted desaturase genes from fungi. Since the only fungal desaturases identified are Δ5- and Δ6-desaturase genes from *Mortierella alpina* (Genbank accession numbers AF067650, AB020032, respectively), desaturase sequences from plants as well as animals were taken into consideration during the design of these degenerate primers. Known Δ5- and Δ6-desaturase sequences from the following organisms were used for the design of these degenerate primers: *Mortierella alpina, Borago officinalis, Helianthus annuus, Brassica napus, Dictyostelium discoideum, Rattus norvegicus, Mus musculus, Homo sapien, Caenorhabditis elegans, Arabidopsis thaliana, and Ricinus communis.* The degenerate primers used were as follows using the CODEHOP Blockmaker program (http://blocks.fhcrc.org/codehop.html):

A. Protein motif 1 (SEQ ID NO:53):
$NH_3$- VYDVTEWVKRHPGG -COOH
Primer RO 834 (SEQ ID NO:1):
5'-GTBTAYGAYGTBACCGARTGGGTBAAGCGYCAYCCBGGHGGH-3'

B. Protein Motif 2 (SEQ ID NO:54):
$NH_3$- GASANWWKHQHNVHH -COOH
Primer RO835 (Forward) (SEQ ID NO:2):
5'-GGHGCYTCCGCYAACTGGTGGAAGCAYCAGCAYAACGTBCAYCAY-3'
Primer RO836 (Reverse) (SEQ ID NO:3)
5'-RTGRTGVACGTTRTGCTGRTGCTTCCACCAGTTRGCGGARGCDCC-3'

C. Protein Motif 3 (SEQ ID NO:55):
$NH_3$- NYQIEHHLFPTM -COOH
Primer R0838 (Reverse) (SEQ ID NO:4)
5'-TTGATRGTCTARCTYGTRGTRGASAARGGVTGGTAC-3'

In addition, two more primers were designed based on the 2nd and 3rd conserved 'Histidine-box' found in known Δ6-desaturases. These were:

Primer RO753 (SEQ ID NO:5)
5'-CATCATCATNGGRAANARRTGRTG-3'
Primer RO754 (SEQ ID NO:6)
5'-CTACTACTACTACAYCAYACNTAYACNAAY-3'

The degeneracy code for the oligonucleotide sequences was: B=C,G,T; H=A,C,T; S=C,G; R=A,G; V=A,C,G; Y=C,T; D= A+T+C; N= A,C,G,or T/U, unknown or other

Example 2

Isolation of Δ6-Desaturase Nucleotide Sequences from *Saprolegnia diclina* (ATCC 56851)

Total RNA from *Saprolegnia diclina* (ATCC 56851) was isolated using the lithium chloride method (Hoge, et al., Exp. Mycology (1982) 6:225-232). Five μg of the total RNA was reverse transcribed; using the Superscript Preamplification system (LifeTechnologies, Rockville, MD) and the oligo (dT) $_{12-18}$ primer supplied with the kit, to generate the first strand cDNA.

To isolate the Δ6-desaturase gene, various permutations and combinations of the above mentioned degenerate oligonucleotides were used in PCR reactions. Of the various primer sets tried, the only primers to give distinct bands were RO834 /RO838. PCR amplification was carried out in a 100 μl volume containing: 2 μl of the first strand cDNA template, 20mM Tris-HCl, pH 8.4, 50mM KCl, 1.5mM $MgCl_2$, 200 μM each deoxyribonucleotide triphosphate and 2 pmole of each primer. Thermocycling was carried out at two different annealing temperatures, 42°C and 45°C, and these two PCR reactions were combined, resolved on a 1.0% agarose gel, and the band of ~1000bp was gel purified using the QiaQuick Gel Extraction Kit (Qiagen, Valencia, CA). The staggered ends on these fragments were 'filled-in' using T4 DNA polymerase (LifeTechnologies, Rockville, MD) as per manufacturer's specifications, and these DNA fragments were cloned into the PCR-Blunt vector (Invitrogen, Carlsbad, CA). The recombinant plasmids were transformed into TOP10 supercompetent cells (Invitrogen, Carlsbad, CA), and clones were sequenced.

Two clones were thus isolated that showed sequence homology to previously identified Δ6-desaturases. These clones are described as follows:

a. Clone#20-2 was partially sequenced and the deduced amino acid sequence from 702 bp showed 30.2% identity with Δ6-desaturase from *Mortierella alpina* as the highest scoring match in a TfastA search.

b. Clone #30-1 was partially sequenced, and the deduced amino acid sequence of 687 bp showed 48.5% amino acid identity with *Mortierella alpina's* Δ6-desaturase as the highest scoring match in a TfastA search. These two sequences also overlapped each other indicating they belonged to a single putative Δ6-desaturase from *S. diclina*. This novel Δ6-desaturase sequence was then used to design primers to retrieve the 3'- and the 5'-end of the full-length Δ6-desaturase gene from the cDNA library generated from the mRNA of *S. diclina.*

To isolate the 3'-end, PCR amplification was carried out using plasmid DNA purified from the cDNA library as the template and oligonucleotides R0923 (SEQ ID NO:7) (5'-CGGTGCAGTGGTGGAAGAACAAGCACAAC-3') and R0899 (SEQ ID NO:8) (5'-AGCGGATAACAATTTCACACAGGAAACAGC-3'). Oligonucleotide R0923 was designed based on the #20-2 fragment of this putative Δ6-desaturase, and oligonucleotide RO899 corresponded to sequence from the pBluescript II SK(+) vector used for preparation of the cDNA library. Amplification was carried out using 10 pmols of each primer and the Taq PCR Master Mix (Qiagen, Valencia, CA). Samples were denatured initially at 94°C for 3 minutes, followed by 30 cycles of the following: 94°C for 1 minute, 60°C for 1 minute, 72°C for 2 minutes. A final extension cycle at 72°C for 10 minutes was carried out before the reaction was terminated. The PCR fragments were resolved on a 0.8% agarose gel and gel purified using the Qiagen Gel Extraction Kit. The staggered end on these fragments were 'filled-in' using T4 DNA polymerase (LifeTechnologies, Rockville, MD) as per manufacturer's specifications, and these DNA fragments were cloned into the PCR-Blunt vector (Invitrogen, Carlsbad, CA). The recombinant plasmids were transformed into TOP10 supercompetent cells (Invitrogen, Carlsbad, CA), and clones were sequenced.

Clone sd2-2 contained a 958 bp insert which was identified to contain the 3'-end of the putative Δ6-gene based on sequence homology with known Δ6-desaturases and the presence of the 'TAA' stop codon and Poly A tail.

To isolate the 5'-end of this Δ6-desaturase from *Saprolegnia diclina*, the oligonucleotide RO939 (SEQ ID NO: 9) (5'-CGTAGTACTGCTCGAGGAGCTTGAGCGCCG-3') was designed based on the sequence of the #30-1 fragment identified earlier. This oligonucleotide was used in combination with RO898 (SEQ ID NO:10) (5'-CCCAGTCACGACGTTGTAAAACGACGGCCAG-3') (designed based on the sequence of from the pBluescript SK(+) vector) to PCR amplify the 5'-end of the Δ6-desaturase from the cDNA library. In this case, the Advantage-GC cDNA PCR kit (Clonetech, Palo Alto, CA) was used to overcome PCR amplification problems that occur with GC rich regions, predicted to be present at the 5'-end of this Δ6-desaturase. PCR thermocycling conditions were as follows: The template was initially denatured at 94˚C for 1 minute, followed by 30 cycles of [94˚C for 30 seconds, 68˚C for 3 minutes], and finally an extension cycle at 68˚C for 5 minutes. The PCR products thus obtained were cloned into the PCR-Blunt vector (Invitrogen, Carlsbad, CA) following the same protocol as described above. Clone sd21-2 was thus obtained that contained a 360 bp insert that contained the putative 'ATG' start site of the novel Δ6-desaturase. The deduced amino acid sequence of this fragment, when aligned with known Δ6-desaturases showed 37-45% identity.

This novel Δ6-desaturase gene was isolated in its entirety by PCR amplification using, the *S. diclina* cDNA library, or *S. diclina* genomic DNA as a template, and the following oligonucleotides:

a. RO 951 (SEQ ID NO:11)
(5'- TCAACAGAATTCATGGTCCAGGGGCAAAAGGCCGAGAAGATCTCG-3') that contained sequence from the 5' end of clone sd21-2 as well as an *EcoRI* site (underlined) to facilitate cloning into a yeast expression vector

b. RO960 (SEQ ID NO:12)
(5'- ATACGTAAGCTTTTACATGGCGGGAAACTCCTTGAAGAACTCGATCG-3') that contained sequence from the 3' end of clone sd2-2 including the stop codon as well as a HindIII site (underlined) for cloning in an expression vector.

PCR amplification was carried out using 200 ng of the cDNA library plasmid template, 10 pmoles of each primer and the Taq PCR Master Mix (Qiagen, Valencia, CA), or 200 ng of genomic DNA, 10 pmoles of each primer, and the Advantage-GC cDNA PCR kit (Clonetech, Palo Alto, CA). Thermocycling conditions were as follows: the template was initially denatured at 94˚C for 1 minute, followed by 30 cycles of [94˚C for 30 seconds, 68˚C for 3 minutes], and finally an extension cycle at 68˚C for 5 minutes. The PCR product thus obtained was digested with *EcoRI/HindIII* and cloned into the yeast expression vector pYX242 (Invitrogen, Carlsbad, CA) to generate clones pRSP1 (genomic DNA-derived) and pRSP2 (library-derived) which were then sequenced and used for expression studies.

The Δ6-desatura full-length gene insert was 1362 bp (SEQ ID NO:13, Figure 2) in length and, beginning with the first ATG, contained an open reading frame encoding 453 amino acids. (The nucleotide sequence encoding the Δ6-desaturase was deposited as plasmid pRSP1 with the American Type Culture Collection (ATCC), 10801 University Boulevard, Manassas, VA 20110 under the terms of the Budapest Treaty on January 23, 2001 and was accorded accession number PTA-2829.) The amino acid sequence of the full-length gene (SEQ ID NO:14, Figure 3) contained regions of homology to Δ6-desaturases from *Mortierella alpina*, *Caenorhabditis elegans and Borago officinalis.* It also contained the three conserved 'histidine boxes' found in all known membrane-bound desaturases (Okuley, et al. (1994) The Plant Cell 6: 147-158). These were present at amino acid positions 171-176, 208-212, and 391-395. As with other membrane-bound Δ6-desaturases, the third Histidine-box motif (HXXHH) in the *S.diclina* Δ6-desaturase was found to be QXXHH. This sequence also contained a cytochrome b5 domain at the 5'-end. This cytochrome b5 domain is found in a number of membrane-bound desaturase enzymes, and cytochrome b5 is thought to function as an electron donor in these enzymes. The presence of this domain may be advantageous when expressing the desaturase in heterologous systems for PUFA production. Since the proposed use of this gene is for the reconstruction of the PUFA biosynthetic pathway in plants, the base composition of this gene may be important. (It is known that some recombinant genes show poor expression because of variations in their base composition as compared to that of the host. The overall G+C content of this gene was 59%, which is close to that of the *M. alpina* desaturases that have been successfully expressed in plants.)

Example 3

Isolation of Δ5-Desaturase Nucleotide Sequences from *Saprolegnia diclina* (ATCC 56851)

*Saprolegnia diclina* (ATCC 56851) produces both arachidonic acid (ARA, 20:4 n-6) and eicosapentanoic acid (EPA, 20:5 n-3); thus, it was thought to have, perhaps, a Δ5-desaturase which can convert dihomo-gamma-linolenic acid (DGLA, 20:3n-6) to arachidonic acid (ARA, 20:4 n-6).

As with the Δ6-desaturase isolation, for the Δ5-desaturase isolation from *S. diclina,* various combinations of the degenerate primers were used in PCR reactions, using first strand cDNA as the template. The primer combination, R0753 and RO754, generated a distinct band of 588 bp using the following PCR conditions: 2 $\mu$l of the first strand cDNA template, 20mM Tris-HCl, pH 8.4, 50mM KCl, 1.5mM $MgCl_2$, 200 $\mu$M each deoxyribonucleotide triphosphate, 2 pmole of each primer and 1U cDNA polymerase (Clonetech, Palo Alto, CA), in a final reaction volume of 50 $\mu$l. Thermocycling was carried out as follows: an initial denaturation at 94˚C for 3 minutes, followed by 35 cycles of: denaturation at 94˚C for 30 seconds, annealing at 60˚C for 30 seconds and extension at 72˚C for 1 minute. This was followed by a final extension at 72˚C for 7 minutes, and the reaction was terminated at 4˚C. This fragment thus generated was cloned (clone # 18-1), sequenced and, when translated, showed 43% amino acid identity with *Mortierella alpina* $\Delta$5-desaturase (Genbank accession # AF067654) and 38.7% identity with *Dictyostelium discoideum* $\Delta$5-desaturase (Genbank accession # AB029311). The second PCR fragment was identified using Primers R0834 and R0838 in the reaction described in Example 2. This fragment, of approximately 1000 bp in length, was cloned (Clone # 20-8) and the deduced amino acid sequence derived from 775 bp showed 42% identity with $\Delta$5-desaturase from *Dictyostelium discoideum* $\Delta$5-desaturase (Genbank accession # AB029311). These two sequences, #18-1 and #20-8, overlapped each other indicating they belonged to a single putative $\Delta$5-desaturase from *S. diclina.* These sequences were then used to design primers to retrieve the 3'- and the 5'-end of the novel $\Delta$5-desaturase gene from the cDNA library generated from the mRNA of *S. diclina*.

To isolate the 3'-end of this putative $\Delta$5-desaturase, PCR amplification was carried out using plasmid DNA purified from the cDNA library, as the template and oligonucleotides RO851 (SEQ ID NO:15) (5'-CCATCAAGACGTACCTTGCGATC-3') and R0899 (SEQ ID NO:8) (5'- AGCGGATAACAATTTCACACAGGAAACAGC-3'). Oligonucleotide RO851 was designed based on the #18-1 fragment of this putative $\Delta$5-desaturase, and oligonucleotide RO899 corresponded to sequence from the pBluescript II SK(+) vector. Amplification was carried out using 200 ng of template plasmid DNA, 10 pmoles of each primer and the Taq PCR Master Mix (Qiagen, Valencia, CA). Samples were denatured initially at 94˚C for 3 minutes, followed by 35 cycles of the following: 94˚C for 30 seconds, 60˚C for 30 seconds, 72˚C for 1 minutes. A final extension cycle at 72˚C for 7 minutes was carried out before the reaction was terminated. The PCR fragments were cloned into the PCR-Blunt vector (Invitrogen, Carlsbad, CA) as per the protocol described in Example 2. The recombinant plasmids were transformed into TOP10 supercompetent cells (Invitrogen, Carlsbad, CA), and clones were sequenced. Clone sd12-11 contained a 648 bp insert which contained the 3'-end of the putative $\Delta$5-gene based on sequence homology with known $\Delta$5-desaturases and the presence of the 'TAA' stop codon and polyA tail.

The 5'-end of this $\Delta$5-desaturase from *Saprolegnia diclina* was isolated using primers RO941 and R0898. The oligonucleotide R0941 (SEQ ID NO:16) (5'-GCTGAACGGGTGGTACGAGTCGAACGTG-3') was designed based on the sequence of the #20-8 fragment identified earlier. This oligonucleotide was used in combination with RO898 (SEQ ID NO:10) (5'-CCCAGTCACGACGTTGTAAAACGACGGCCAG-3') (designed based on the sequence of from the pBluescript II SK(+) vector) in a PCR amplification reaction using the cDNA library plasmid DNA as the template. Here the Advantage-GC cDNA PCR kit (Clonetech, Palo Alto, CA) was used as per the manufacturer's protocol, and the thermocycling conditions were as follows: an initial denaturation was carried out at 94˚C for 1 minute, followed by 30 cycles of [denaturation at 94˚C for 30 seconds, annealing and extension 68˚C for 3 minutes], and a final extension cycle at 68˚C for 5 minutes. These PCR products were purified, cloned into the PCR-Blunt vector (Invitrogen, Carlsbad, CA), and sequenced as described above. Clone sd24-1 was identified to contain a 295 bp insert that contained the putative 'ATG' start site of the novel $\Delta$5-desaturase. Analysis of the deduced amino acid sequence of this fragment showed regions of high homology with known $\Delta$5-desaturases and also the presence of a cytochrome b5 domain.

The full-length $\Delta$5-desaturase gene was isolated by PCR amplification using *S.diclina* genomic DNA as a template and the following oligonucleotides:

a. RO 953 (SEQ ID NO:17)
(5'-ACGAGAGAATTCATGGCCCCGCAGACGGAGCTCCGCCAG CGC-3') that contained sequence from the 5' end of clone sd24-1 as well as an *EcoRI* site (underlined) to facilitate cloning into a yeast expression vector; and

b. RO956 (SEQ ID NO:18)
(5'- AAAAGACTCGAGTTAGCCCATGTGGATCGTGGCGGCGATGCCC
TGC-3') that contained sequence from the 3' end of clone sd12-11 including the stop codon as well as a *XhoI* site (underlined) for cloning in an expression vector.

Conditions for the PCR amplification of the 'full length' gene were similar to those described for the amplification of the $\Delta$6-desaturase from genomic DNA (Example 2). The PCR product thus obtained was digested with *EcoRI/XhoI* and cloned into the yeast expression vector pYX242 (Invitrogen, Carlsbad, CA). Clone pRSP3 (genomic DNA-derived) was shown to contain a 1413 bp insert and was used for expression studies.

The 1413 bp full-length gene (SEQ ID NO:19, Figure 4) of the putative $\Delta$5-desaturase from *S.diclina* contained an open reading frame encoding 471 amino acids (SEQ ID NO:20, Figure 5). (The nucleotide sequence encoding the $\Delta$5-desaturase was deposited (as plasmid pRSP3) with the American Type Culture Collection, 10810 University Boulevard, Manassas, VA 20110 under the terms of the Budapest Treaty on January 23, 2001 and was accorded accession number PTA-2928.) This translated protein showed 40.5% overall identity with the *Mortierella alpina* Δ5-desaturasae (Genbank accession # AF067654) and 39.5% identity with the *Dictyostelium discoideum* Δ5-desaturase (Genbank accession # AB022097). It also contained the three conserved 'histidine boxes' at amino acid positions 186-190, 223-228, 406-410. Like the Δ6-desaturase, this sequence also contained a cytochrome b5 domain at the 5'-end. The overall G+C content of this gene was 61.5%.

Example 4

Expression of *S. diclina* Desaturase Genes in Baker's Yeast

Clone pRSP2, which consisted of the full length Δ6-desaturase cloned into PYX242 (Invitrogen, Carlsbad, CA), and clone pRSP3, which consisted of the full-length Delta 5-desaturase gene in pYX242, were transformed into competent *Saccharomyces cerevisiae* strain 334. Yeast transformation was carried out using the Alkali-Cation Yeast Transformation Kit (BIO 101, Vista, CA) according to conditions specified by the manufacturer. Transformants were selected for leucine auxotrophy on media lacking leucine (DOB [-Leu]). To detect the specific desaturase activity of these clones, transformants were grown in the presence of 50 μM specific fatty acid substrates as listed below:

a. Stearic acid (18:0) (conversion to oleic acid would indicate Δ9-desaturase activity)
b. Oleic acid (18:1) (conversion to linoleic acid would indicated Δ12-desaturase activity)
c. Linoleic acid (18:2 n-6) (conversion to alpha-linolenic acid would indicate Δ15-desaturase activity and conversion to gamma-linolenic acid would indicate Δ6-desaturase activity)
d. Alpha-linolenic acid (18:3 n-3) (conversion to stearidonic acid would indicate Δ6-desaturase activity)
e. Dihomo-gamma-linolenic acid (20:3 n-6) (conversion to arachidonic acid would indicate Δ5-desaturase activity).

The negative control strain was *S. cerevisiae* 334 containing the unaltered pYX242 vector, and these were grown simultaneously. The cultures were vigorously agitated (250 rpm) and grown for 48 hours at 24˚C in the presence of 50 μM (final concentration) of the various substrates. The cells were pelleted and vortexed in methanol; chloroform was added along with tritridecanoin (as an internal standard). These mixtures were incubated for at least an hour at room temperature or at 4˚C overnight. The chloroform layer was extracted and filtered through a Whatman filter with 1 gm anhydrous sodium sulfate to remove particulates and residual water. The organic solvents were evaporated at 40˚C under a stream of nitrogen. The extracted lipids were then derivitized to fatty acid methyl esters (FAME) for gas chromatography analysis (GC) by adding 2 ml of 0.5 N potassium hydroxide in methanol to a closed tube. The samples were heated to 95˚C-100˚C for 30 minutes and cooled to room temperature. Approximately 2 ml of 14% borontrifluoride in methanol were added and the heating repeated. After the extracted lipid mixture cooled, 2 ml of water and 1 ml of hexane were added to extract the FAME for analysis by GC. The percent conversion was calculated by dividing the product produced by the sum of (the product produced + the substrate added) and then multiplying by 100.

Table 1 represents the enzyme activity of the genes isolated based on the percent conversion of substrate added. The pRSP1 clone that contained the Δ6-desaturase gene from *S. diclina* converted 28% of the 18:2n-6 substrate to 18:3n-3, as well was 37% of the 18:3n-3 substrate to 18:4n-3. This confirms that the gene encodes a Δ6-desaturase. There was no background (non-specific conversion of substrate) in this case. (All tables referred to herein are presented after the Abstract of the Disclosure.)

The pRSP3 clone that contained the Δ5-desaturase gene from *S. diclina* was capable of converting 27% of the added 20:3n-6 substrate to 20:4n-6, indicating that the enzyme it encodes is a Δ5-desaturase. In this case too, there was no background substrate conversion detected. This data indicates that desaturases with different substrate specificity can be expressed in a heterologous system and can also be used to produce polyunsaturated fatty acids.

Table 2 represents fatty acids of interest as a percentage of the total lipid extracted from *S. cerevisiae* 334 with the indicated plasmid. No glucose was present in the growth media. Affinity gas chromatography was used to separate the respective lipids. GC/MS was employed to identify the products. From this table, it is apparent that exogenously added substrates, when added in the free form was taken up by the recombinant yeast and the incorporated into their membranes. In the yeast clone containing the Δ6-desaturase gene (pRSP1), GLA (γ-18:3) was identified as a novel PUFA when LA (18:2) was added as the substrate, and arachidonic acid was detected in yeast containing the Δ5-desaturase gene (pRSP3) when DGLA (20:3) was added as a substrate.

Example 5

Co-Expression of *S. diclina* Desaturases with Elongases

The plasmid pRSP1 (Δ6) and pRSP3 (Δ5) were individually co-transformed with pRAE73-A3, a clone that contains the Human Elongase gene (SEQ ID NO:21) in the yeast expression vector pYES2, into yeast as described in Example 4. This elongase gene catalyzes some of the elongation steps in the PUFA pathway. Co-transformants were selected on minimal media lacking leucine and uracil (DOB[-Leu-Ura]).

Table 3 shows that when 50 μM of the substrate LA (18:2 n-6) was added, that the Δ6-desaturase converted this substrate to GLA (18:3 n-6) and the elongase was able to add two carbons to GLA to produce DGLA (20:3 n-6). The percent conversion of the substrate to the final product by these co-transformed enzymes is 26.4%, with no background observed from the negative control. Similarly, the co-transformed enzymes can act on ALA (18:3n-3) to finally form (20:4n-3) with a percentage conversion of 34.39%. Thus, *S. diclina* Δ6-desaturase was able to produce a product in a heterologous expression system that could be further utilized by another heterologous enzyme from the PUFA biosynthetic pathway to produce the expected PUFA.

Table 4 shows results of the pRSP3(Δ5)/Human Elongase co-transformation experiment. In this case, substrate GLA (18:3n-6) was converted to DGLA (20:3n-6) by human elongase and this was further converted to ARA (20:4n-6) by the action of *S. diclina* Δ5-desaturase. The percent conversion of the substrate to the final product by these co-transformed enzymes is 38.6%, with no background observed from the negative control.

The other substrate tested in this case was STA (18:4 n-3) which was eventually converted to EPA (20:5n-3) by the concerted action of the two enzymes. Similar results were observed when the pRSP1 and pRSP3 were cotransformed with an elongase gene derived from *M. alpina* (pRPB2) (SEQ ID NO:22), and both genes were shown to be functional in the presence of each other (see Table 3 and Table 4).

Example 6 (comparative)

Isolation of Δ5-Desaturase Nucleotide Sequences from *Thraustochytrium aureum* (ATCC 34303)

To isolate putative desaturase genes, total RNA was Isolated as described in Example 2. Approximately 5 μg was reverse transcribed using the SuperScript Preamplification system (LifeTechnologies, Rockville, MD) as shown in Example 2 to produce first strand cDNA. Using the degenerate primers RO834 (SEQ ID NO:1) and 838 (SEQ ID NO: 4) designed with the block maker program in a 50 μl reaction, the following components were combined: 2 μl of the first strand cDNA template, 20mM Tris-HCl, pH 8.4, 50mM KCl, 1.5mM $MgCl_2$, 200 μM each deoxyribonucleotide triphosphate, 2 pmole final concentration of each primer and cDNA polymerase (Clonetech, Palo Alto, CA). Thermocycling was carried out as follows: an initial denaturation at 94°C for 3 minutes, followed by 35 cycles of denaturation at 94°C for 30 seconds, annealing at 60°C for 30 seconds and extension at 72°C for 1 minute. This was followed by a final extension at 72°C for 7 minutes. Two faint bands of approximately 1000 bp were separated on a 1% agarose gel, excised, and purified with the QiaQuick Gel Extraction Kit (Qiagen, Valencia, CA). The ends were filled in with T4 DNA polymerase and the blunt-end fragments cloned into PCR Blunt as described in Example 2. Sequencing of the obtained clones identified the partial sequence of 680 bp from clone 30-9 whose translation of 226 amino acids had 31.5% identity with Δ6-desaturase from adult zebrafish (Genbank accession number AW281238). A similar degree of amino acid (29.6%-28.7%) homology was found with human Δ6-desaturase (Genbank accession number AF126799), *Physcomitrella patens* (moss) Δ6-desaturase (Genbank accession number AJ222980), *Brassica napus* (canola) Δ8-sphingolipid desaturase (Genbank accession number AJ224160), and human Δ5-desaturase (ATCC accession number 203557, Genbank accession number AF199596). Since there was a reasonable degree of amino acid homology to known desaturases, a full-length gene encoding a potential desaturase was sought to determine its activity when expressed in yeast.

To isolate the 3' end of the gene, 10 pmol of primer RO936 (SEQ ID NO:23) (5'-GTCGGGCAAGGCGGAAAAGTACCTCAAGAG-3') and vector primer RO899 (SEQ ID NO:8) were combined in a reaction with 100 ng of purified plasmid from the *T. aureum* cDNA library in reaction volume of 100 μl in Taq PCR Master Mix (Qiagen, Valencia, CA). Thermocycling conditions were as follows: an initial melt at 94°C for 3 minutes followed by 30 cycles of 94°C for 1 minute, 60°C for 1 minute, and 72°C for 2 minutes. This was followed by an extension step of 10 minutes at 72°C. Several bands, including the predicted size of 1.2 kb, were separated on a 1 % agarose gel and purified as stated earlier. Also as described earlier, the ends of the fragments were blunt ended, cloned into PCR Blunt and sequenced. Fragment #70-2 of approximately 1.2 kb was sequenced and contained an open reading frame and a stop codon, which overlapped fragment 30-9.

To isolate the 5' end of the gene, RO937 (SEQ ID NO:24) (5'-AAACCTGTAGACAATGTGGAGGGGCGTGGG-3') and RO 899 (SEQ ID NO:8) were used in a 50 μl PCR reaction with Advantage-GC cDNA PCR kit (Clonetech, Palo Alto, CA), as per the manufacturer's protocol, with 100 ng of purified plasmid DNA from the library and 10 pmol of each primer. The thermocycling conditions were as follows: An initial denaturation was carried out at 94˚C for 1 minute, followed by 30 cycles of [denaturation at 94˚C for 30 seconds, annealing and extension 68˚C for 3 minutes], and a final extension cycle at 68˚C for 5 minutes. A band of approximately 500bp, in the range of the expected size, was gel purified, blunt ended and cloned into PCR Blunt as previously described. Clone 95-2 contained an open reading frame with a start codon. This fragment also overlapped with clone 30-9, indicating that they were indeed pieces of the same gene.

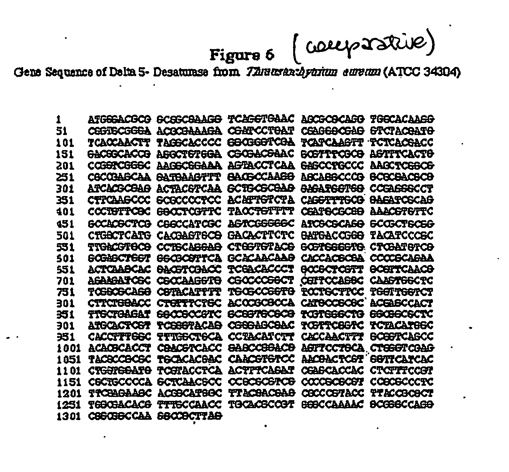

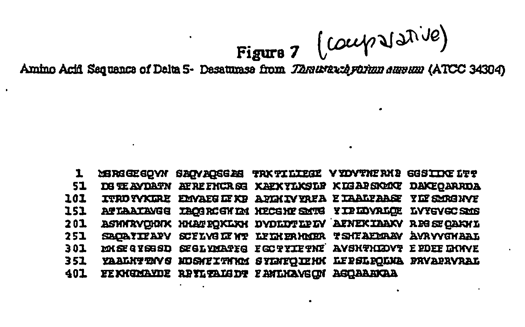

To isolate the full-length gene, primers were designed with restriction sites 5' and 3' (underlined) with *EcoRI* and *XhoI*, respectively, as follows: 5' primer RO972 (SEQ ID NO:25) (5'-ATACTTGAATTCATGGGACGCGGCGGCGAAGGTCAGGTGAAC-3'), 3' primer RO949 (SEQ ID NO: 26) (5'-CTTATACTCGAGCTAAGCGG CCTTGGCCGCCGCCTGGCC-3') and 3' primer RO950 (SEQ ID NO:27) (5'-CTTATACTCGAGTAAATGGCTCGCGAGGCGAAGCGAGTGGC-3'). Two primers were used for the 3' end of the gene in the initial isolation attempt since the primer RO949, containing the stop codon had 66% GC content, while the alternate primer RO950, which was outside the stop codon, had only a 56% GC content. A 50 μl PCR reaction with RO972/RO949 and RO972/950 was performed with Advantage-GC cDNA PCR kit (Clonetech, Palo Alto, CA) under identical conditions noted in the preceding paragraph. Only the primer set RO972/950 produced a band of approximately 1.6 kb. Use of genomic DNA as a template (under identical conditions with 100 ng of target) also produced a similar-sized band. Fragments were separated on an agarose gel, gel purified, blunt-ended and cloned into PCR Blunt as previously described. Fragments were evaluated by sequencing, and a number of clones were cut with *EcoRI*/*XboI* to excise the full length gene, ligated to pYX242 *EcoRI*/*XhoI* which had been treated with shrimp alkaline phosphatase (Roche, Indianapolis, IN) with the Rapid ligation kit (Roche, Indianapolis, IN). Clone 99-3, designated pRTA4, contained the full length gene of 1317 bp (SEQ ID NO:28, Figure 6) and an open reading frame of 439 aa (SEQ IN NO: 29, Figure 7). (The nucleotide sequence encoding the Δ5-desaturase was deposited with the ATCC, 10801 University Boulevard, Manassas, VA 20110 under the terms of the Budapest Treaty on January 23, 2001 and was accorded accession number PTA-2927.) This gene contained three histidine boxes at amino acid numbers 171-175, 208-212, and 376-380. The 5'-end of the gene, when translated, also shows homology to cytochrome b5.

## Example 7 (Comparative)

### Expression of *T. aureum* Desaturase Gene in Baker's Yeast

The clone pRTA4 containing the full-length gene was transformed into the yeast host *S. cerevisiae* 334 and plated on selective media as described in Example 4. The cultures were grown at 24˚C for 48 hours in minimal media lacking leucine with 50 μM of exogenous free fatty acid added as a substrate as shown in Table 5. The only conversion of a substrate was DGLA (20:3n-6) to ARA (20:4n-6). The conversion of 23.7% of the added DGLA indicates that this gene encodes for a Δ5-desaturase.

Table 6 shows some of the fatty acids as a percentage of the lipid extracted from the yeast host. For Δ5-desaturase activity, there was no background (detection of ARA observed in the negative control containing the yeast expression plasmid, PYX242.)

## Example 8 (Comparative)

### Co-Expression of *T. aureum* Desaturase Gene with Elongases

The plasmid pRTA4 was co-transformed with an additional enzyme in the PUFA pathway, pRAE73-A3 which contains the human elongase gene in the yeast expression vector pYES2 as described in Example 4, and co-transformants were selected on minimal media lacking leucine and uracil.

Table 7 shows that when 100 μM of the substrate DGLA was added, that the Δ5-desaturase actively produced ARA, to which the elongase was able to add two carbons to produce ADA. The percent conversion of *T. aureum* Δ5-desaturase, which consists of both ARA and ADA (products), was 16.7%, with no background observed from the negative control.

In view of the above results, *T. aureum* Δ5-desaturase is able to produce a product in a heterologous expression system that can be used by an additional heterologous enzyme in the PUFA biosynthetic pathway to produce the expected PUFA.

## Example 9 (Comparative)

### Isolation of Δ5-desaturase Nucleotide Sequences from *Thraustochytrium aureum* BICC7091 (T7091)

A partial desaturase candidate was isolated using the degenerate primer combination of RO834/RO838, listed in Example 1. The genomic DNA was prepared from Thraustochytrium aureum BICC7091 (Biocon India Ltd., Bangalore, India) using the DNeasy plant maxi kit (Qiagen, Valencia, CA). The T7091 gDNA was amplified with primers RO834 (5' -GTB TAY GAY GTB ACC GAR TGG GTB AAG CGY CAY CCB GGH GGH- 3') (SEQ ID NO:1) and R0838 (5' - CAT GGT VGG RAA SAG RTG RTG YTC RAT CTG RTA GTT- 3') (SEQ ID NO:36). PCR amplification was carried out in a 100 µl volume containing: 5 µl of isolated T7091 gDNA, 0.2 M dNTP mix, 50 pM each primer, 10 µl of 10X buffer and 1.0 U of cDNA Polymerase. Thermocycler conditions in Perkin Elmer 9600 were as follows: 94°C for 3 min, then 35 cycles of 94 °C for 30 sec., 60 °C for 30 sec., and 72°C for 1 min. PCR was followed by an additional extension at 72°C for 7 minutes. The PCR amplified mixture was run on a 1.0% agarose gel, and amplified fragments of approximately 1.2 Kb and 1.4 Kb were gel purified using the Qiaquick Gel Extraction Kit (Qiagen, Valencia, CA). The staggered ends on these fragments were filled-in using T4 DNA Polymerase (LifeTechnologies, Rockville, MD), the isolated fragments were cloned into the pCR-Blunt vector (Invitrogen, Co., Carlsbad, CA), and the recombinant plasmids were transformed into TOP10 supercompetent cells (Invitrogen, Carlsbad, CA).

Twenty-four clones were prepared and sequenced using ABI 373A DNA Sequencer (Applied Biosystems, Foster City, CA). The translated sequences were used as queries to search the GenEmbl database (Genetics Computer Group (GCG) (Madison, WI)), using the tFastA algorithm (a Pearson and Lipman search for similarity between a protein query sequence and any group of nucleotide sequences). A gene fragment, T7091B2, was further pursued because it had identity with known desaturases. T7091B2 had 26.8% identity in 362 amino acids with Human Δ5-desaturase (GenBank accession number AF226273).

To isolate the 3' and 5'-ends, new primers were designed based on the T7091B2 internal sequence. The T7091 cDNA library, which contains approximately 2 X $10^7$ clones with an average insert size of 1 Kb, was PCR amplified using the new primers and a vector primer. The primers that produced more 5' and 3'sequences with the vector primers RO898 (5' -CCC AGT CAC GAC GTT GTA AAA CGA CGG CCA G- 3') (SEQ ID NO:10) and R0899 (5' -AGC GGA TAA CAA TTT CAC ACA GGA AAC AGC- 3') (SEQ ID NO:8) were RO1065 (5' -CGA CAA GAG GAA GAG TGT CCA AAT C- 3') (SEQ ID NO:37) and RO1064 (5' -CGC CTT CAA GAG TTT TTG TAC GGA ATT GGG-3')(SEQ ID NO:38), respectively, for clone T7091B2. Two new primers were designed based on the T7091B2 5' and 3' sequences:

a. RO1097 (5' -CTT GTA CCA TGG GTC GCG GAG CAC AGG GAG-3) (SEQ ID NO:39), which has an added NcoI restriction site (underlined)
b. RO1098 (5' -TGA AGC TTA CTC GCT CTT GGC AGC TTG GCC-3') (SEQ ID NO:40), which has an added *Hind*III restriction site (underlined)

This novel Δ5-desaturase gene was isolated in its entirely by PCR amplification, using the T. aureum 7091 gDNA. PCR was carried out in a 50 µl volume containing:

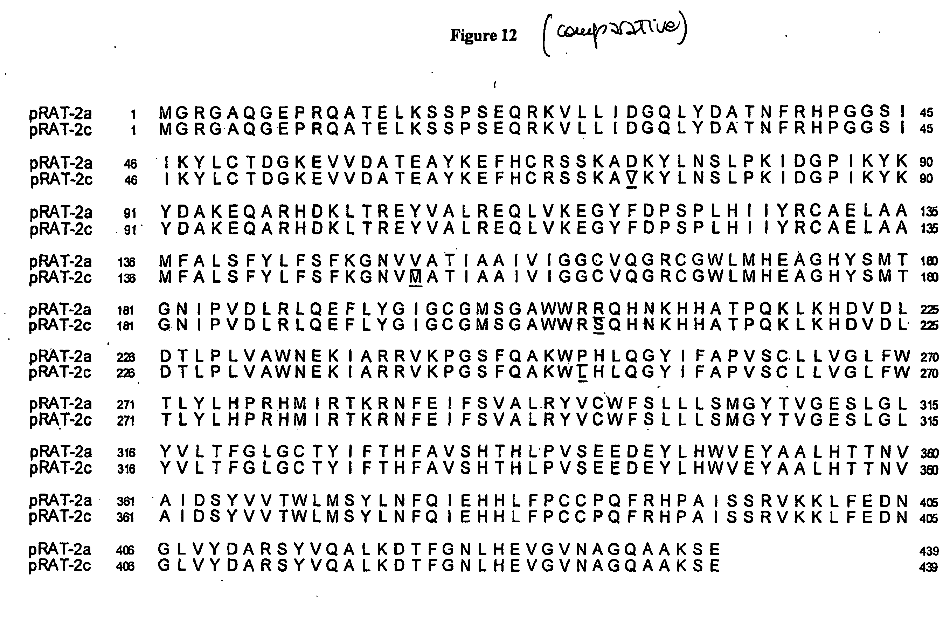

1 µl of isolated T7091 gDNA, 0.2 µM dNTP mix, 50 pM each primer, 5 µl of 10 X buffer, 1.5 µl of 50 mM $MgSO_4$, and 0.5 U of *Taq* DNA Polymerase. Thermocycler conditions in Perkin Elmer 9600 were as follows: 94°C for 3 min, then 30 cycles of 95 °C for 45 sec., 55 °C for 30 sec., and 68°C for 2 min. The PCR amplified mixture was run on a gel, an amplified fragment of approximately 1.3 Kb was gel purified, and the isolated fragment was cloned into the pYX242(*Nco*I/*Eco*RV) vector. Two clones, designated as pRAT-2a and 2c, were prepared and sequenced. (Plasmid pRAT-2c was deposited with the American Type Culture Collection, 10801 University Blvd., Manassas, VA 20110-2209 on January _ , 2002 under the terms of the Budapest Treaty and was accorded ATCC deposit number _.) The sequences were different by four amino acids (Figure 12), and the translated sequence had 67.4% identity in 436 amino acids with T. aureum (ATCC34303) Δ5-desaturase (see Example 6).

Example 10 (comparative)

Isolation of Δ6-desaturase Nucleotide Sequences from *Thraustochytrium aureum* BICC7091 (T7091)

Three hundred and seventy-three templates from the T7091 cDNA library were sequenced to determine the viability of the library. The translated sequences were used as queries to search the GenEmbl database using the tFastA algorithm. Clone 602187281R1 (or clone 281 for short) had identity with several known desaturases. The 5' and 3' ends of the EST clone were sequenced in order to design primers for amplification. Primers RO1107 (5' -TTT AAC CAT GGG CCG CGG CGG CGA GAA AAG-3') (SEQ ID NO:41), which has an added NcoI restriction site (underlined), and RO1108 (5' -GGG AAG AAG CTT TCT ACT GCG CCT TGG CTT TCT TTG- 3') (SEQ ID NO:42), which has an added *Hind*III restriction site (underlined), were used to PCR amplify the T7091 gDNA.

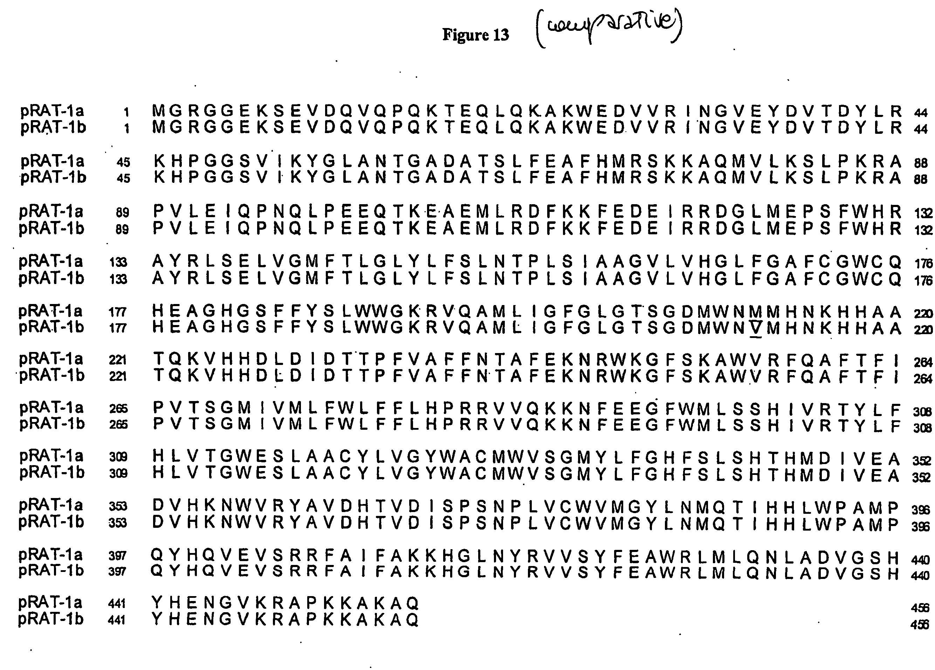

PCR was carried out in a 50 µl volume with *Taq* DNA Polymerase as above. The PCR amplified mixture was run on a gel, an amplified fragment of approximately 1.3 Kb was gel purified, and the isolated fragment was cloned into the pYX242(*Nco*I/*Eco*RV) vector. Two clones, designated as pRAT-1a and 1b, were prepared and sequenced. The sequences were different by one amino acid (Figure 13), and the translated sequence had 25% identity in 430 amino acids with the Human Δ5-desaturase. (Plasmid pRAT-1a was deposited with the American Type Culture Collection, 10801 University Blvd., Manassas, VA 20110-2209 on January __ , 2002 under the terms of the Budapest Treaty and was accorded ATCC deposit number__.)

Example 11 (comparative)

Expression of T. aureum 7091 Desaturase Genes in Baker's Yeast

Clones pRAT-2a and pRAT-2c, which consisted of the full-length Δ5-desaturase cloned into pYX242 (Invitrogen, Carlsbad, CA), and clones pRAT-1a and pRAT-1b, which consisted of the full-length Δ6-desaturase gene in pYX242, were transformed into competent Saccharomyces cerevisiae 334. Yeast transformation was carried out using the Alkali-Cation Yeast Transformation Kit (BIO 101, Vista, CA). Transformants were selected for leucine auxotrophy on media lacking leucine (DOB[-leu]). Because the translated sequence of pRAT-1 cDNA did not have a strong identity with any particular known desaturase, several fatty acid substrates were tested to determine the activity of the expressed enzyme. To detect the specific desaturase activity of pRAT-1a and pRAT-1b clones, transformants were grown in the presence of 100 μM specific fatty acid substrates as listed below:

a. Linoleic acid (LA, 18:2n-6) (conversion to α-linolenic acid would indicate Δ15-desaturase activity and conversion to γ-linolenic acid would indicate Δ6-desaturase activity)
b. α-linolenic acid (ALA, 18:3n-3) (conversion to stearidonic acid would indicate Δ6-desaturase activity)
c. ω6-eicosadienoic acid (EDA, 20:2n-6) (conversion to dihomo-γ-linolenic acid would indicate Δ8-desaturase activity)
d. dihomo-γ-linolenic acid (DGLA, 20:3n-6) (conversion to arachidonic acid would indicate Δ5-desaturase activity)

The substrate for pRAT-2 clones was 100 μM of DGLA. S. cerevisiae 334 containing the unaltered pYX242 vector was used as a negative control. Both pRAT-1a and pRAT-2c were also co-transformed into *S. cerevisiae* 334 with pRAE-73-A3 (i.e, a vector containing the human elongase enzyme which converts 18C fatty acids to 20C fatty acids, SEQ ID NO:21) (see Example 5). The substrates for pRAT-1a/pRAE-73-A3 clones were LA and ALA, and the substrates for pRAT-2c/pRAE-73-A3 clones were LA, ALA, and GLA. The cultures were grown for 48 hours at 24°C, in selective media, in the presence of a particular substrate. Fatty acid analyses were performed as outlined in Example 4.

Table 8 includes an example of the production of AA by strain 334 (pRAT-2c) vs. that by the control strain 334 (pYX242). The amount of AA produced was 22.98%, vs. no detectable amount in the control strain. Strain 334(pRAT-2a) also had no detectable amount of AA produced (data not shown). The four amino acid difference between the two clones (Figure 12, see underlined) rendered the enzyme expressed from strain 334(pRAT-2a) to be inactive. The Δ5-desaturase activity from clone pRAT-2c was further detected when co-expressed with pRAE-73-A3 in the presence of GLA.

Table 9 is the fatty acid analysis results of the strains 334(pRAT-1a) and 334(pYX242) expressed in the presence of substrates LA, ALA, EDA, and DGLA. The Δ6-desaturation of LA will produce GLA, the Δ8-desaturation of EDA will produce DGLA, and the Δ5-desaturation of DGLA will produce AA. All three activities were detected from both strains containing the T7091 gene vs. the control strain. Based on the conversion rates, this enzyme is an active Δ6-desaturase, which can behave like a Δ5- or Δ8-desaturase, given the proper substrates. However, the conversion rates of these substrates to their respective desaturated fatty acids are low, in comparison to the substrates for a Δ6-desaturation. The single amino acid difference between the two clones (Figure 13, see underlined) had no affect on the enzymatic activity (data not shown). The percent conversions are shown in the lower box of Table 9. The Δ6-desaturase activity was further detected when pRAT-1a was co-expressed with pRAE-73-A3 in the presence of LA or ALA. The percent conversions were not calculated for the co-expression experiment, since it is difficult to determine whether the production of the new fatty acids is due to the activity of the desaturase, or elongase, or the combination of the two enzymes.

Two different methods of isolating a novel gene were explored in order to identify desaturase genes from T. aureum 7091. These methods led to the isolation of the T7091 Δ5-desaturase and Δ6-desaturase genes. In the presence of these genes and the appropriate substrates, S. cerevisiae produced desaturated fatty acids at a much higher level than the control strain. Co-transformation of the constructs containing the T7091B2 gene (pRAT-2c) and the Human elongase gene (pRAE-73-A3) in yeast resulted in the conversion of the substrate GLA to AA. This experiment confirmed that the enzyme expressed from pRAT-2c must desaturate the DGLA (produced by the elongase) to AA. Co-transformation of the constructs containing the "281 " gene (pRAT-1a) and the Human elongase gene (pRAE-73-A3) in yeast resulted in the conversion of the substrate LA to DGLA. This experiment confirmed that the enzyme expressed from pRAT-1a must desaturate LA to GLA.

Example 13 (comparative)

Isolation of Δ5-desaturase Nucleotide Sequence from *Isochrysis galbana* 1323

A partial desaturase candidate was isolated using the degenerate primer combination of RO834/RO838, listed in Example 1. The genomic DNA was prepared from Isochrysis galbana CCMP1323 (Provasoli-Guillard National Center for the Culture of Marine Phytoplankton (CCMP), West Boothbay Harbor, MA) using the DNeasy plant maxi kit (Qiagen, Valencia, CA). The I. galbana gDNA was amplified with primers R0834 (5' -GTB TAY GAY GTB ACC GAR TGG GTB AAG CGY CAY CCB GGH GGH- 3') (SEQ ID NO:1) and RO838 (5' -CAT GGT VGG RAA SAG RTG RTG YTC RAT CTG RTA GTT-3') (SEQ ID NO:10). PCR was carried out in a 50 µl volume containing: 1 µl of isolated I. galbana gDNA, 0.2 µM dNTP mix, 50 pM each primer, 5 µl of 10 X buffer, 1.5 µl of 50 mM $MgSO_4$, and 0.5 U of Taq DNA Polymerase. Thermocycler conditions in Perkin Elmer 9600 were as follows: 94˚C for 3 min, then 30 cycles of 95 ˚C for 45 sec., 55 ˚C for 30 sec., and 68˚C for 2 min. The PCR amplified mixture was run on a 1.0% agarose gel, and an amplified fragment of approximately 1.1 Kb was gel purified using the Qiaquick Gel Extraction Kit (Qiagen, Valencia, CA). The staggered ends of the fragment were filled-in using T4 DNA Polymerase (LifeTechnologies, Rockville, MD), the isolated fragment was cloned into the pCR-Blunt vector (Invitrogen, Co., Carlsbad, CA), and the recombinant plasmids were transformed into TOP10 supercompetent cells (Invitrogen, Carlsbad, CA).

Six clones were prepared and sequenced using ABI 373A DNA Sequencer (Applied Biosystems, Foster City, CA). All of the sequences were the same. The translated sequence of the isolated fragment had 47.5% identity in 335 amino acids with with T. aureum (ATCC 34303) Δ5-desaturase in clone pRTA4 (Example 6), and 45.3% identity in 278 amino acids with T. aureum BICC7091 Δ5-desaturase in clone pRAT-2c (Example 9).

To isolate the 5' and 3'-ends, new primers were designed based on the internal sequence of the isolated I. galbana fragment. For the 5 prime end of the gene RO1235 (5'- CGA AGT TGG TGA AGA TGT AGG TGC CG-3') (SEQ ID NO:43) was used, while RO1232 (5'-GAG CGA CGC GTA CAA CAA CTT TCA CGT-3') (SEQ ID NO:44) was used for the 3 prime end of the gene. Approximately 1.4 µg of total RNA was used, according to the manufacturer's direction, with the Rapid amplification of cDNA ends or RACE with the GeneRacer™ kit (Invitrogen, Carlsbad, CA) and Superscript II™ enzyme (Invitrogen, Carlsbad, CA) for reverse transcription to produce cDNA target. For the initial amplification of the ends, the following thermocycling protocol was used in a Perkin Elmer 9600: initial melt at 94˚C for 2 minutes; followed by 5 cycles of 94˚C for 30 seconds and 72˚C for 3 minutes; 10 cycles of 94˚C 30 seconds, 70˚C for 30 seconds, and 72˚C for 3 minutes; and 20 cycles of 94˚C for 30 seconds, 68˚C for 30 seconds and 72˚C for 3 minutes; followed by an extension of 72˚C for 10 minutes. This first PCR reaction was performed with 10 pMol of RO1235 or RO1232 and GeneRacer™ 5 prime primer (5'- CGA CTG GAG CAC GAG GAC ACT GA-3') (SEQ ID NO:45) , or GeneRacer™ 3 prime primer (5'- GCT GTC AAC GAT ACG CTA CGT AAC G-3') (SEQ ID NO:46), respectively, with 1 1 of Thermozyme™ (Invitrogen, Carlsbad, CA) and 1 µl of cDNA in a final volume of 50 µl, according to the manufacturer's directions.

A nested reaction was performed with 2 µl of the initial reaction, 10 pmol of nested primer RO1234 (5'-AGC TCC AGG TGA TTG TGC ACG CGC AG-3') (SEQ ID NO:47) or RO1233 (5'- GAC TTT GAG AAG CTG CGC CTC GAG CTG-3') (SEQ ID NO:48) and 30 pmol the GeneRacer™ nested 5 prime primer (5'- GGA CAC TGA CAT GGA CTG AAG GAG TA-3') (SEQ ID NO:49) and GeneRacer™ nested 3 prime primer (5'- CGC TAC GTA ACG GCA TGA CAG TG -3') (SEQ ID NO:50) respectively, and Platinum Taq™ PCRx (Clonetech, Palo Alto, CA) using $MgSO_4$ according to the manufacturer's protocol. The thermocycling parameter was as follows in a Perkin Elmer 9600: initial melt at 94˚C for 2 minutes; followed by 5 cycles of 94˚C for 30 seconds and 72˚C for 2 minutes; 5 cycles of 94˚C 30 seconds, 70˚C for 2 minutes; 20 cycles of 94˚C for 30 seconds, 65˚C for 30 seconds and 68˚C for 2 minutes; followed by an extension of 68˚C for 10 minutes. Agarose gel analysis of the PCR products showed a band around 800 base pairs for the 5 prime reactions and approximately a 1.2 kilobase band for 3 prime reaction. Subsequent cloning into pCR Blunt (Invitrogen, Carlsbad, CA), transformation into Top10 competent cells (Invitrogen, Carlsbad, CA), and sequencing, revealed an open reading frame with both a start and stop codons.

Primers RO1309 (5'- ATG ATG GAA TTC ATG GTG GCA GGC AAA TCA GGC GC-3') (SEQ ID NO:51) and RO1310 (5'- AAT AAT GTC GAC CTA GTG CGT GTG CTC GTG GTA GG-3') (SEQ ID NO:52) with restrictions sites added for cloning (see underlined *Eco*RI, and *Sal*I, respectively) were used to isolate a full length gene.

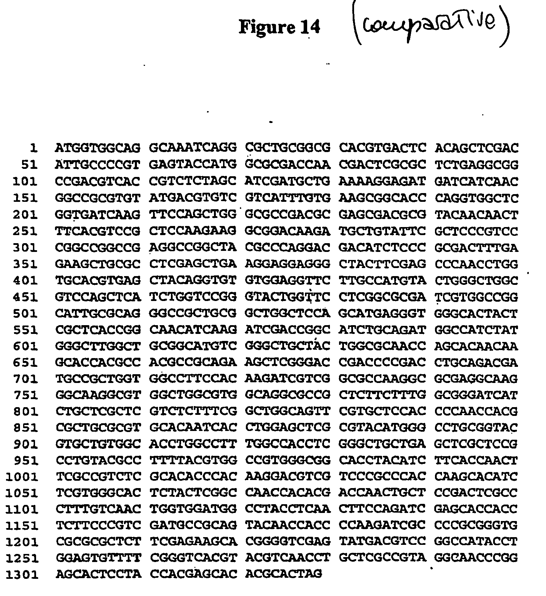

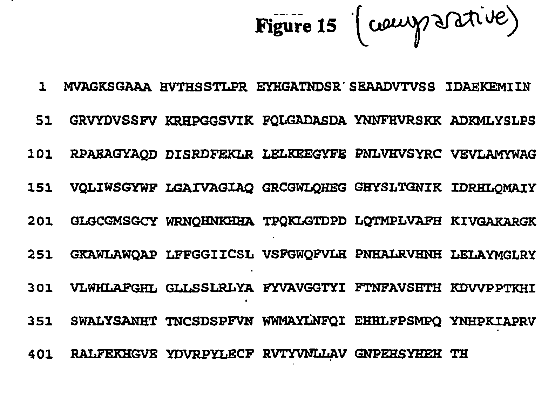

As shown above, 10 pmol of primers RO1309 and 1310 were used with Platinum Taq™ PCRx (Clonetech, Palo Alto, CA) using $MgSO_4$ according to the manufacturer's protocol, with 2 µl of the cDNA as target. The thermocycling parameters were as follows: initial melt at 94˚C for 2 minutes; followed by 5 cycles of 94˚C for 30 seconds and 72˚C for 2 minutes; 5 cycles of 94˚C 30 seconds, 70˚C for 2 minutes; 20 cycles of 94˚C for 30 seconds, 65˚C for 30 seconds and 68˚C for 2 minutes; followed by an extension of 68˚C for 10 minutes. The single product of the reaction was gel purified using the QiaQuick gel purification kit (Qiagen, Valencia, CA), cut with *EcoR*I and *Sal*I, ligated to pYX242 *EcoR*I/*Xho*I linearized DNA with the Rapid ligation kit (Roche, Indianapolis, IN), and designated pRIG-1. The clone pRIG-1 contained a full length gene of 1329 bp (SEQ ID NO:34; Figure 14) and an open reading frame of 442 amino acid (SEQ ID NO: 35; Figure 15). (Plasmid pRIG-1 was deposited with the American Type Culture Collection, 10801 University Blvd., Manassas, VA 20110-2209 on January __, 2002 under the terms of the Budapest Treaty and was accorded ATCC deposit number __.)

Example 13 (comparative)

Expression of *I. galbana* Desaturase Gene in Baker's Yeast

The clone pRIG-1 containing the full-length gene was transformed into the yeast host S. cerevisiae 334 and plated on selective media as described in Example 4. The cultures were grown at 24˚C for 48 hours in minimal media lacking leucine, with 50 μM of exogenous free fatty acid added as a substrate as shown in Table 10. The conversion of substrates was ETA (20:4n-3) to EPA (20:5n-3) and DGLA (20:3n-6) to AA (20:4n-6). The 45.4% conversion to ARA and 59.75% conversion to EPA indicate that this gene encodes for a Δ5-desaturase. Table 10 shows some of the fatty acids as a percentage of the lipid extracted from the yeast host. For Δ5-desaturase activity, there was little or no background (detection of ARA or EPA observed in the negative control containing the yeast expression plasmid, pYX242.)

Example 14 (comparative)

Co-Expression of *I. galbana* Desaturase Gene with Elongases

The plasmid pRIG-1 could be co-transformed with an additional enzyme in the PUFA pathway, such as pRAE-73-A3 which contains the human elongase gene in the yeast expression vector pYES2 as described in Example 4, and co-transformants selected on minimal media lacking leucine and uracil. Substrates such as DGLA or ETA could be added so that the Δ5-desaturase would actively produced ARA or EPA, to which the elongase is able to add two carbons to produce ADA or ω3-DPA. Therefore, I. galbana Δ5-desaturase could produce a product in a heterologous expression system that can be used by an additional heterologous enzyme in the PUFA biosynthetic pathway, to produce the expected PUFA.

The Following nutritional compositions do not form part of the claimed invention and are described for comparative reasons.

Nutritional Compositions

The PUFAs described in the Detailed Description may be utilized in various nutritional supplements, infant formulations, nutritional substitutes and other nutritional solutions.

I. INFANT FORMULATIONS

A. Isomil® Soy Formula with Iron:

Usage: As a beverage for infants, children and adults with an allergy or sensitivity to cows milk. A feeding for patients with disorders for which lactose should be avoided: lactase deficiency, lactose intolerance and galactosemia.

Features:

- Soy protein isolate to avoid symptoms of cow's-milk-protein allergy or sensitivity.
- Lactose-free formulation to avoid lactose-associated diarrhea.
- Low osmolality (240 mOs/kg water) to reduce risk of osmotic diarrhea.
- Dual carbohydrates (corn syrup and sucrose) designed to enhance carbohydrate absorption and reduce the risk of exceeding the absorptive capacity of the damaged gut.
- 1.8 mg of Iron (as ferrous sulfate) per 100 Calories to help prevent iron deficiency.
- Recommended levels of vitamins and minerals.

- Vegetable oils to provide recommended levels of essential fatty acids.
- Milk-white color, milk-like consistency and pleasant aroma.

Ingredients: (Pareve) 85% water, 4.9% corn syrup, 2.6% sugar (sucrose), 2.1 % soy oil, 1.9% soy protein isolate, 1.4% coconut oil, 0.15% calcium citrate, 0. 11 % calcium phosphate tribasic, potassium citrate, potassium phosphate monobasic, potassium chloride, mono- and disglycerides, soy lecithin, carrageenan, ascorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

B. Isomil® DF Soy Formula For Diarrhea:

Usage: As a short-term feeding for the dietary management of diarrhea in infants and toddlers.

Features:

- First infant formula to contain added dietary fiber from soy fiber specifically for diarrhea management.
- Clinically shown to reduce the duration of loose, watery stools during mild to severe diarrhea in infants.
- Nutritionally complete to meet the nutritional needs of the infant.
- Soy protein isolate with added L-methionine meets or exceeds an infant's requirement for all essential amino acids.
- Lactose-free formulation to avoid lactose-associated diarrhea.
- Low osmolality (240 mOsm/kg water) to reduce the risk of osmotic diarrhea.
- Dual carbohydrates (corn syrup and sucrose) designed to enhance carbohydrate absorption and reduce the risk of exceeding the absorptive capacity of the damaged gut.
- Meets or exceeds the vitamin and mineral levels recommended by the Committee on Nutrition of the American Academy of Pediatrics and required by the Infant Formula Act.
- 1.8 mg of iron (as ferrous sulfate) per 100 Calories to help prevent iron deficiency.
- Vegetable oils to provide recommended levels of essential fatty acids.

Ingredients: (Pareve) 86% water, 4.8% corn syrup, 2.5% sugar (sucrose), 2.1% soy oil, 2.0% soy protein isolate, 1.4% coconut oil, 0.77% soy fiber, 0.12% calcium citrate, 0.11% calcium phosphate tribasic, 0.10% potassium citrate, potassium chloride, potassium phosphate monobasic, mono and diglycerides, soy lecithin, carrageenan, magnesium chloride, ascorbic acid, L-methionine, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

C. Isomil® SF Sucrose-Free Soy Formula With Iron:

Usage: As a beverage for infants, children and adults with an allergy or sensitivity to cow's-milk protein or an intolerance to sucrose. A feeding for patients with disorders for which lactose and sucrose should be avoided.

Features:

- Soy protein isolate to avoid symptoms of cow's-milk-protein allergy or sensitivity.
- Lactose-free formulation to avoid lactose-associated diarrhea (carbohydrate source is Polycose® Glucose Polymers).
- Sucrose free for the patient who cannot tolerate sucrose.
- Low osmolality (180 mOsm/kg water) to reduce risk of osmotic diarrhea.
- 1.8 mg of iron (as ferrous sulfate) per 100 Calories to help prevent iron deficiency.
- Recommended levels of vitamins and minerals.
- Vegetable oils to provide recommended levels of essential fatty acids.
- Milk-white color, milk-like consistency and pleasant aroma.

Ingredients: (Pareve) 75% water, 11.8% hydrolized cornstarch, 4.1% soy oil, 4.1 % soy protein isolate, 2.8% coconut oil, 1.0% modified cornstarch, 0.38% calcium phosphate tribasic, 0. 17% potassium citrate, 0.13% potassium chloride, mono- and diglycerides, soy lecithin, magnesium chloride, abscorbic acid, L-methionine, calcium carbonate, sodium chloride, choline chloride, carrageenan, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

D. Isomil® 20 Soy Formula With Iron Ready To Feed, 20 Cal/fl oz.:

Usage: When a soy feeding is desired.

Ingredients: (Pareve) 85% water, 4.9% corn syrup, 2.6% sugar(sucrose), 2.1 % soy oil, 1.9% soy protein isolate, 1.4% coconut oil, 0.15% calcium citrate, 0. 11% calcium phosphate tribasic, potassium citrate, potassium phosphate monobasic, potassium chloride, mono- and diglycerides, soy lecithin, carrageenan, abscorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

E. Similac® Infant Formula:

Usage: When an infant formula is needed: if the decision is made to discontinue breastfeeding before age 1 year, if a supplement to breastfeeding is needed or as a routine feeding if breastfeeding is not adopted.

Features:

- Protein of appropriate quality and quantity for good growth; heat-denatured, which reduces the risk of milk-associated enteric blood loss.
- Fat from a blend of vegetable oils (doubly homogenized), providing essential linoleic acid that is easily absorbed.
- Carbohydrate as lactose in proportion similar to that of human milk.
- Low renal solute load to minimize stress on developing organs.
- Powder, Concentrated Liquid and Ready To Feed forms.

Ingredients: (-D) Water, nonfat milk, lactose, soy oil, coconut oil, mono- and diglycerides, soy lecithin, abscorbic acid, carrageenan, choline chloride, taurine, m-inositol, alpha-tocopheryl acetate, zinc sulfate, niacinamide, ferrous sulfate, calcium pantothenate, cupric sulfate, vitamin A palmitate, thiamine chloride hydrochloride, riboflavin, pyridoxine hydrochloride, folic acid, manganese sulfate, phylloquinone, biotin, sodium selenite, vitamin D3 and cyanocobalamin.

F.Similac® NeoCare Premature Infant Formula With Iron:

Usage: For premature infants' special nutritional needs after hospital discharge. Similac NeoCare is a nutritionally complete formula developed to provide premature infants with extra calories, protein, vitamins and minerals needed to promote catch-up growth and support development.

Features:

- Reduces the need for caloric and vitamin supplementation. More calories (22 Cal/fl oz) than standard term formulas (20 Cal/fl oz).
- Highly absorbed fat blend, with medium-chain triglycerides (MCToil) to help meet the special digestive needs of premature infants.
- Higher levels of protein, vitamins and minerals per 100 calories to extend the nutritional support initiated in-hospital.
- More calcium and phosphorus for improved bone mineralization.

Ingredients: -D Corn syrup solids, nonfat milk, lactose, whey protein concentrate, soy oil, high-oleic safflower oil, fractionated coconut oil (medium chain triglycerides), coconut oil, potassium citrate, calcium phosphate tribasic, calcium carbonate, ascorbic acid, magnesium chloride, potassium chloride, sodium chloride, taurine, ferrous sulfate, m-inositol, choline chloride, ascorbyl palmitate, L-carnitine, alpha-tocopheryl acetate, zinc sulfate, niacinamide, mixed tocopherols, sodium citrate, calcium pantothenate, cupric sulfate, thiamine chloride hydrochloride, vitamin A palmitate, beta carotene, riboflavin, pyridoxine hydrochloride, folic acid, manganese sulfate, phylloquinone, biotin, sodium selenite, vitamin D3 and cyanocobalamin.

G. Similac Natural Care Low-Iron Human Milk Fortifier Ready To Use, 24 Cal/fl oz.:

Usage: Designed to be mixed with human milk or to be fed alternatively with human milk to low-birth-weight infants.

Ingredients: -D Water, nonfat milk, hydrolyzed cornstarch, lactose, fractionated coconut oil (medium-chain triglycerides), whey protein concentrate, soy oil, coconut oil, calcium phosphate tribasic, potassium citrate, magnesium chloride, sodium citrate, ascorbic acid, calcium carbonate, mono and diglycerides, soy lecithin, carrageenan, choline chloride, m-inositol, taurine, niacinamide, L-carnitine, alpha tocopheryl acetate, zinc sulfate, potassium chloride, calcium pantothenate, ferrous sulfate, cupric sulfate, riboflavin, vitamin A palmitate, thiamine chloride hydrochloride, pyridoxine hydrochloride, biotin, folic acid, manganese sulfate, phylloquinone, vitamin D3, sodium selenite and cyanocobalamin.

Various PUFAs of this invention can be substituted and/or added to the infant formulae described above and to other infant formulae known to those in the art.

II. NUTRITIONAL FORMULATIONS

A. ENSURE®

Usage: ENSURE is a low-residue liquid food designed primarily as an oral nutritional supplement to be used with or between meals or, in appropriate amounts, as a meal replacement. ENSURE is lactose- and gluten-free, and is suitable for use in modified diets, including low-cholesterol diets. Although it is primarily an oral supplement, it can be fed by tube.

Patient Conditions:

- For patients on modified diets
- For elderly patients at nutrition risk
- For patients with involuntary weight loss
- For patients recovering from illness or surgery
- For patients who need a low-residue diet

Ingredients: -D Water, Sugar (Sucrose), Maltodextrin (Corn), Calcium and Sodium Caseinates, High-Oleic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Sulfate, Alpha-Tocopheryl Acetate, Gellan Gum, Niacinamide,

Calcium Pantothenate, Manganese Sulfate, Cupric Sulfate, Vitamin A Palmitate, Thiamine Chloride Hydrochloride, Pyridoxine Hydrochloride, Riboflavin, Folic Acid, Sodium Molybdate, Chromium Chloride, Biotin, Potassium Iodide, Sodium Selenate.

B.ENSURE® BARS:

Usage: ENSURE BARS are complete, balanced nutrition for supplemental use between or with meals. They provide a delicious, nutrient-rich alternative to other snacks. ENSURE BARS contain <1 g lactose/bar, and Chocolate Fudge Brownie flavor is gluten-free. (Honey Graham Crunch flavor contains gluten.)

Patient Conditions:

- For patients who need extra calories, protein, vitamins and minerals.

- Especially useful for people who do not take in enough calories and nutrients.
- For people who have the ability to chew and swallow
- Not to be used by anyone with a peanut allergy or any type of allergy to nuts.

Ingredients: Honey Graham Crunch -- High-Fructose Corn Syrup, Soy Protein Isolate, Brown Sugar, Honey, Maltodextrin (Corn), Crisp Rice (Milled Rice, Sugar [Sucrose], Salt [Sodium Chloride] and Malt), Oat Bran, Partially Hydrogenated Cottonseed and Soy Oils, Soy Polysaccharide, Glycerine, Whey Protein Concentrate, Polydextrose, Fructose, Calcium Caseinate, Cocoa Powder, Artificial Flavors, Canola Oil, High-Oleic Safflower Oil, Nonfat Dry Milk, Whey Powder, Soy Lecithin and Corn Oil. Manufactured in a facility that processes nuts.

Vitamins and Minerals: Calcium Phosphate Tribasic, Potassium Phosphate Dibasic, Magnesium Oxide, Salt (Sodium Chloride), Potassium Chloride, Ascorbic Acid, Ferric Orthophosphate, Alpha-Tocopheryl Acetate, Niacinamide, Zinc Oxide, Calcium Pantothenate, Copper Gluconate, Manganese Sulfate, Riboflavin, Beta Carotene, Pyridoxine Hydrochloride, Thiamine Mononitrate, Folic Acid, Biotin, Chromium Chloride, Potassium Iodide, Sodium Selenate, Sodium Molybdate, Phylloquinone, Vitamin D3 and Cyanocobalamin.

Protein: Honey Graham Crunch - The protein source is a blend of soy protein isolate and milk proteins.

| | |
|---|---|
| Soy protein isolate | 74% |
| Milk proteins | 26% |

Fat: Honey Graham Crunch - The fat source is a blend of partially hydrogenated cottonseed and soybean, canola, high oleic safflower, oils, and soy lecithin.

| | |
|---|---|
| Partially hydrogenated cottonseed and soybean oil | 76% |
| Canola oil | 8% |
| High-oleic safflower oil | 8% |
| Corn oil | 4% |
| Soy lecithin | 4% |

Carbohydrate: Honey Graham Crunch - The carbohydrate source is a combination of high-fructose corn syrup, brown sugar, maltodextrin, honey, crisp rice, glycerine, soy polysaccharide, and oat bran.

| | |
|---|---|
| High-fructose corn syrup | 24% |
| Brown sugar | 21% |
| Maltodextrin | 12% |
| Honey | 11% |
| Crisp rice | 9% |
| Glycerine | 9% |
| Soy Polysaccharide | 7% |
| Oat bran | 7% |

C. ENSURE® HIGH PROTEIN:

Usage: ENSURE HIGH PROTEIN is a concentrated, high-protein liquid food designed for people who require additional calories, protein, vitamins, and minerals in their diets. It can be used as an oral nutritional supplement with or between meals or, in appropriate amounts, as a meal replacement. ENSURE HIGH PROTEIN is lactose- and gluten-free, and is suitable for use by people recovering from general surgery or hip fractures and by patients at risk for pressure ulcers.

Patient Conditions:

- For patients who require additional calories, protein, vitamins, and minerals, such as patients recovering from general surgery or hip fractures, patients at risk for pressure ulcers, and patients on low-cholesterol diets.

Features:

- Low in saturated fat
- Contains 6 g of total fat and < 5 mg of cholesterol per serving
- Rich, creamy taste
- Excellent source of protein, calcium, and other essential vitamins and minerals
- For low-cholesterol diets
- Lactose-free, easily digested

Ingredients:

Vanilla Supreme: -D Water, Sugar (Sucrose), Maltodextrin (Corn), Calcium and Sodium Caseinates, High-Oleic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Suffate, Alpha-Tocopheryl Acetate, Gellan Gum, Niacinamide, Calcium Pantothenate, Manganese Sulfate, Cupric Sulfate, Vitamin A Palmitate, Thiamine Chloride Hydrochloride, Pyridoxine Hydrochloride, Riboflavin, Folic Acid, Sodium Molybdate, Chromium Chloride, Biotin, Potassium Iodide, Sodium Selenate, Phylloquinone, Vitamin D3 and Cyanocobalamin.

Protein:

The protein source is a blend of two high-biologic-value proteins: casein and soy.

| | |
|---|---|
| Sodium and calcium caseinates | 85% |
| Soy protein isolate | 15% |

Fat:

The fat source is a blend of three oils: high-oleic safflower, canola, and soy.

| | |
|---|---|
| High-oleic safflower oil | 40% |
| Canola oil | 30% |
| Soy oil | 30% |

The level of fat in ENSURE HIGH PROTEIN meets American Heart Association (AHA) guidelines. The 6 grams of fat in ENSURE HIGH PROTEIN represent 24% of the total calories, with 2.6% of the fat being from saturated fatty acids and 7.9% from polyunsaturated fatty acids. These values are within the AHA guidelines of < 30% of total calories from fat, < 10% of the calories from saturated fatty acids, and < 10% of total calories from polyunsaturated fatty acids.

Carbohydrate:

ENSURE HIGH PROTEIN contains a combination of maltodextrin and sucrose. The mild sweetness and flavor variety (vanilla supreme, chocolate royal, wild berry, and banana), plus VARI-FLAVORS® Flavor Pacs in pecan, cherry, strawberry, lemon, and orange, help to prevent flavor fatigue and aid in patient compliance.

Vanilla and other nonchocolate flavors:

| | |
|---|---|
| Sucrose | 60% |
| Maltodextrin | 40% |

Chocolate:

| | |
|---|---|
| Sucrose | 70% |
| Maltodextrin | 30% |

D. ENSURE® LIGHT

Usage: ENSURE LIGHT is a low-fat liquid food designed for use as an oral nutritional supplement with or between meals. ENSURE LIGHT is lactose- and gluten-free, and is suitable for use in modified diets, including low-cholesterol diets.

Patient Conditions:

- For normal-weight or overweight patients who need extra nutrition in a supplement that contains 50% less fat and 20% fewer calories than ENSURE.
- For healthy adults who do not eat right and need extra nutrition.

Features:

- Low in fat and saturated fat
- Contains 3 g of total fat per serving and < 5 mg cholesterol
- Rich, creamy taste
- Excellent source of calcium and other essential vitamins and minerals
- For low-cholesterol diets
- Lactose-free, easily digested

Ingredients:

French Vanilla: -D Water, Maltodextrin (Corn), Sugar (Sucrose), Calcium Caseinate, High-Oleic Safflower Oil, Canola Oil, Magnesium Chloride, Sodium Citrate, Potassium Citrate, Potassium Phosphate Dibasic, Magnesium Phosphate Dibasic, Natural and Artificial Flavor, Calcium Phosphate Tribasic, Cellulose Gel, Choline Chloride, Soy Lecithin, Carrageenan, Salt (Sodium Chloride), Ascorbic Acid, Cellulose Gum, Ferrous Sulfate, Alpha-Tocopheryl Acetate, Zinc Sulfate, Niacinamide, Manganese Sulfate, Calcium Pantothenate, Cupric Sulfate, Thiamine Chloride Hydrochloride, Vitamin A Palmitate, Pyridoxine Hydrochloride, Riboflavin, Chromium Chloride, Folic Acid, Sodium Molybdate, Biotin, Potassium Iodide, Sodium Selenate, Phylloquinone, Vitamin D3 and Cyanocobalamin.

Protein:

The protein source is calcium caseinate.

| | |
|---|---|
| Calcium caseinate | 100% |

Fat:

The fat source is a blend of two oils: high-oleic safflower and canola.

| | |
|---|---|
| High-oleic safflower oil | 70% |
| Canola oil | 30% |

The level of fat in ENSURE LIGHT meets American Heart Association (AHA) guidelines. The 3 grams of fat in ENSURE LIGHT represent 13.5% of the.total calories, with 1.4% of the fat being from saturated fatty acids and 2.6% from polyunsaturated fatty acids. These values are within the AHA guidelines of < 30% of total calories from fat, < 10% of the, calories from saturated fatty acids, and < 10% of total calories from polyunsaturated fatty acids.

Carbohydrate:

ENSURE LIGHT contains a combination of maltodextrin and sucrose. The chocolate flavor contains corn syrup as well. The mild sweetness and flavor variety (French vanilla, chocolate supreme, strawberry swirl), plus VARI-FLAVORS® Flavor Pacs in pecan, cherry, strawberry, lemon, and orange, help to prevent flavor fatigue and aid in patient compliance.

Vanilla and other nonchocolate flavors:

| | |
|---|---|
| Sucrose | 51% |
| Maltodextrin | 49% |

Chocolate:

| | |
|---|---|
| Sucrose | 47.0% |
| Corn Syrup | 26.5% |
| Maltodextrin | 26.5% |

Vitamins and Minerals:

An 8-fl-oz serving of ENSURE LIGHT provides at least 25% of the RDIs for 24 key vitamins and minerals.

Caffeine:

Chocolate flavor contains 2.1 mg caffeine/8 fl oz.

E.ENSURE PLUS®

Usage: ENSURE PLUS is a high-calorie, low-residue liquid food for use when extra calories and nutrients, but a normal concentration of protein, are needed. It is designed primarily as an oral nutritional supplement to be used with or between meals or, in appropriate amounts, as a meal replacement. ENSURE PLUS is lactose- and gluten-free. Although it is primarily an oral nutritional supplement, it can be fed by tube.

Patient Conditions:

- For patients who require extra calories and nutrients, but a normal concentration of protein, in a limited volume.
- For patients who need to gain or maintain healthy weight.

Features:

- Rich, creamy taste
- Good source of essential vitamins and minerals

Ingredients:

Vanilla: -D Water, Corn Syrup, Maltodextrin (Corn), Corn Oil, Sodium and Calcium Caseinates, Sugar (Sucrose), Soy Protein Isolate, Magnesium Chloride, Potassium Citrate, Calcium Phosphate Tribasic, Soy Lecithin, Natural and Artificial Flavor, Sodium Citrate, Potassium Chloride, Choline Chloride, Ascorbic Acid, Carrageenan, Zinc Sulfate, Ferrous Sulfate, Alpha-Tocopheryl Acetate, Niacinamide, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D3.

Protein:

The protein source is a blend of two high-biologic-value proteins: casein and soy.

| | |
|---|---|
| Sodium and calcium caseinates | 84% |
| Soy protein isolate | 16% |

Fat:

The fat source is corn oil.

| | |
|---|---|
| Corn oil | 100% |

Carbohydrate:

ENSURE PLUS contains a combination of maltodextrin and sucrose. The mild sweetness and flavor variety (vanilla, chocolate, strawberry, coffee, buffer pecan, and eggnog), plus VARI-FLAVORS® Flavor Pacs in pecan, cherry, strawberry, lemon, and orange, help to prevent flavor fatigue and aid in patient compliance.

Vanilla, strawberry, butter pecan, and coffee flavors:

| | |
|---|---|
| Corn Syrup | 39% |
| Maltodextrin | 38% |
| Sucrose | 23% |

Chocolate and eggnog flavors:

| | |
|---|---|
| Corn Syrup | 36% |
| Maltodextrin | 34% |
| Sucrose | 30% |

Vitamins and Minerals:

An 8-fl-oz serving of ENSURE PLUS provides at least 15% of the RDIs for 25 key Vitamins and minerals.

Caffeine:

Chocolate flavor contains 3.1 mg Caffeine/8 fl oz. Coffee flavor contains a trace amount of caffeine.

F. ENSURE PLUS® HN

Usage: ENSURE PLUS HN is a nutritionally complete high-calorie, high-nitrogen liquid food designed for people with higher calorie and protein needs or limited volume tolerance. It may be used for oral supplementation or for total nutritional support by tube. ENSURE PLUS HN is lactose- and gluten-free.

Patient Conditions:

- For patients with increased calorie and protein needs, such as following surgery or injury.
- For patients with limited volume tolerance and early satiety.

Features:

- For supplemental or total nutrition
- For oral or tube feeding
- 1.5 CaVmL,
- High nitrogen
- Calorically dense

Ingredients:

Vanilla: -D Water, Maltodextrin (Corn), Sodium and Calcium Caseinates, Corn Oil, Sugar (Sucrose), Soy Protein Isolate, Magnesium Chloride, Potassium Citrate, Calcium Phosphate Tribasic, Soy Lecithin, Natural and Artificial Flavor, Sodium Citrate, Choline Chloride, Ascorbic Acid, Taurine, L-Carnitine, Zinc Sulfate, Ferrous Sulfate, Alpha-Tocopheryl Acetate, Niacinamide, Carrageenan, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D3.

G.ENSURE® POWDER:

Usage: ENSURE POWDER (reconstituted with water) is a low-residue liquid food designed primarily as an oral nutritional supplement to be used with or between meals. ENSURE POWDER is lactose- and gluten-free, and is suitable for use in modified diets, including low-cholesterol diets.

Patient Conditions:

- For patients on modified diets
- For elderly patients at nutrition risk
- For patients recovering from illness/surgery
- For patients who need a low-residue diet

Features:

- Convenient, easy to mix
- Low in saturated fat
- Contains 9 g of total fat and < 5 mg of cholesterol per serving
- High in vitamins and minerals
- For low-cholesterol diets
- Lactose-free, easily digested

Ingredients: -D Corn Syrup, Maltodextrin (Corn), Sugar (Sucrose), Corn Oil, Sodium and Calcium Caseinates, Soy Protein Isolate, Artificial Flavor, Potassium Citrate, Magnesium Chloride, Sodium Citrate, Calcium Phosphate Tribasic, Potassium Chloride, Soy Lecithin, Ascorbic Acid, Choline Chloride, Zinc Sulfate, Ferrous Sulfate, Alpha-Tocopheryl Acetate, Niacinamide, Calcium Pantothenate, Manganese Sulfate, Thiamine Chloride Hydrochloride, Cupric Sulfate, Pyridoxine Hydrochloride, Riboflavin, Vitamin A Palmitate, Folic Acid, Biotin, Sodium Molybdate, Chromium Chloride, Potassium Iodide, Sodium Selenate, Phylloquinone, Vitamin D3 and Cyanocobalamin.

Protein:

The protein source is a blend of two high-biologic-value proteins: casein and soy.

| | |
|---|---|
| Sodium and calcium caseinates | 84% |
| Soy protein isolate | 16% |

Fat:

The fat source is corn oil.

| | |
|---|---|
| Corn oil | 100% |

Carbohydrate:

ENSURE POWDER contains a combination of corn syrup, maltodextrin, and sucrose. The mild sweetness of ENSURE POWDER, plus VARI-FLAVORS® Flavor Pacs in pecan, cherry, strawberry, lemon, and orange, helps to prevent flavor fatigue and aid in patient compliance.

Vanilla:

| | |
|---|---|
| Corn Syrup | 35% |
| Maltodextrin | 35% |
| Sucrose | 30% |

H. ENSURE® PUDDING

Usage: ENSURE PUDDING is a nutrient-dense supplement providing balanced nutrition in a nonliquid form to be used with or between meals. It is appropriate for consistency-modified diets (e.g., soft, pureed, or full liquid) or for people with swallowing impairments. ENSURE PUDDING is gluten-free.

Patient Conditions:

- For patients on consistency-modified diets (e.g., soft, pureed, or full liquid)
- For patients with swallowing impairments

Features:

- Rich and creamy, good taste
- Good source of essential vitamins and minerals
- Convenient-needs no refrigeration
- Gluten-free

Nutrient Profile per 5 oz: Calories 250, Protein 10.9%, Total Fat 34.9%, Carbohydrate 54.2%

Ingredients:

Vanilla: -D Nonfat Milk, Water, Sugar (Sucrose), Partially Hydrogenated Soybean Oil, Modified Food Starch, Magnesium Sulfate, Sodium Stearoyl Lactylate, Sodium Phosphate Dibasic, Artificial Flavor, Ascorbic Acid, Zinc Sulfate, Ferrous Sulfate, Alpha-Tocopheryl Acetate, Choline Chloride, Niacinamide, Manganese Sulfate, Calcium Pantothenate, FD&C Yellow #5, Potassium Citrate, Cupric Sulfate, Vitamin A Palmitate, Thiamine Chloride Hydrochloride, Pyridoxine Hydrochloride, Riboflavin, FD&C Yellow #6, Folic Acid, Biotin, Phylloquinone, Vitamin D3 and Cyanocobalamin.

Protein:

The protein source is nonfat milk.

| | |
|---|---|
| Nonfat milk | 100% |

Fat:

The fat source is hydrogenated soybean oil.

| | |
|---|---|
| Hydrogenated soybean oil | 100% |

Carbohydrate:

ENSURE PUDDING contains a combination of sucrose and modified food starch. The mild sweetness and flavor variety (vanilla, chocolate, butterscotch, and tapioca) help prevent flavor fatigue. The product contains 9.2 grams of lactose per serving.

Vanilla and other nonchocolate flavors:

| | |
|---|---|
| Sucrose | 56% |
| Lactose | 27% |
| Modified food starch | 17% |

Chocolate:

| | |
|---|---|
| Sucrose | 58% |
| Lactose | 26% |
| Modified food starch | 16% |

I. ENSURE® WITH FIBER:

Usage: ENSURE WITH FIBER is a fiber-containing, nutritionally complete liquid food designed for people who can benefit from increased dietary fiber and nutrients. ENSURE WITH FIBER is suitable for people who do not require a low-residue diet. It can be fed orally or by tube, and can be used as a nutritional supplement to a regular diet or, in appropriate amounts, as a meal replacement. ENSURE WITH FIBER is lactose- and gluten-free, and is suitable for use in modified diets, including low-cholesterol diets.

Patient Conditions:

- For patients who can benefit from increased dietary fiber and nutrients

Features:

- New advanced formula-low in saturated fat, higher in vitamins and minerals
- Contains 6 g of total fat and < 5 mg of cholesterol per serving
- Rich, creamy taste
- Good source of fiber
- Excellent source of essential vitamins and minerals
- For low-cholesterol diets
- Lactose- and gluten-free

Ingredients:

Vanilla: -D Water; Maltodextrin (Corn), Sugar (Sucrose), Sodium and Calcium Caseinates, Oat Fiber, High-Oleic Safflower Oil, Canola Oil, Soy Protein Isolate, Corn Oil, Soy Fiber, Calcium Phosphate Tribasic, Magnesium Chloride, Potassium Citrate, Cellulose Gel, Soy Lecithin, Potassium Phosphate Dibasic, Sodium Citrate, Natural and Artificial Flavors, Choline Chloride, Magnesium Phosphate, Ascorbic Acid, Cellulose Gum, Potassium Chloride, Carrageenan, Ferrous Sulfate, Alpha-Tocopheryl Acetate, Zinc Sulfate, Niacinamide, Manganese Sulfate, Calcium Pantothenate, Cupric Sulfate, Vitamin A Palmitate, Thiamine Chloride Hydrochloride, Pyridoxine Hydrochloride, Riboflavin, Folic Acid, Chromium Chloride, Biotin, Sodium Molybdate, Potassium Iodide, Sodium Selenate, Phylloquinone, Vitamin D3 and Cyanocobalamin.

Protein:

The protein source is a blend of two high-biologic-value proteins-casein and soy.

| | |
|---|---|
| Sodium and calcium caseinates | 80% |
| Soy protein isolate | 20% |

Fat:

The fat source is a blend of three oils: high-oleic safflower, canola, and corn.

| | |
|---|---|
| High-oleic safflower oil | 40% |
| Canola oil | 40% |
| Corn oil | 20% |

The level of fat in ENSURE WITH FIBER meets American Heart Association (AHA) guidelines. The 6 grams of fat in ENSURE WITH FIBER represent 22% of the total calories, with 2.01 % of the fat being from saturated fatty acids and 6.7% from polyunsaturated fatty acids. These values are within the AHA guidelines of ≤ 30% of total calories from fat, < 10% of the calories from saturated fatty acids, and ≤ 10% of total calories from polyunsaturated fatty acids.

Carbohydrate:

ENSURE WITH FIBER contains a combination of maltodextrin and sucrose. The mild sweetness and flavor variety (vanilla, chocolate, and butter pecan), plus VARI-FLAVORS® Flavor Pacs in pecan, cherry, strawberry, lemon, and orange, help to prevent flavor fatigue and aid in patient compliance.

Vanilla and other nonchocolate flavors:

| | |
|---|---|
| Maltodextrin | 66% |
| Sucrose | 25% |
| Oat Fiber | 7% |

(continued)

| | |
|---|---|
| Soy Fiber | 2% |

Chocolate:

| | |
|---|---|
| Maltodextrin | 55% |
| Sucrose | 36% |
| Oat Fiber | 7% |
| Soy Fiber | 2% |

Fiber:

The fiber blend used in ENSURE WITH FIBER consists of oat fiber and soy polysaccharide. This blend results in approximately 4 grams of total dietary fiber per 8-fl. oz can. The ratio of insoluble to soluble fiber is 95:5.

The various nutritional supplements described above and known to others of skill in the art can be substituted and/or supplemented with the PUFAs produced in accordance with the present invention.

J. Oxepa™ Nutritional Product

Oxepa is a low-carbohydrate, calorically dense, enteral nutritional product designed for the dietary management of patients with or at risk for ARDS. It has a unique combination of ingredients, including a patented oil blend containing eicosapentaenoic acid (EPA from fish oil), $\gamma$-linolenic acid (GLA from borage oil), and elevated antioxidant levels.

Caloric Distribution:

Caloric density is high at 1.5 Cal/mL (355 Cal/8 fl oz), to minimize the volume required to meet energy needs. The distribution of Calories in Oxepa is shown in Table A.

Table A. Caloric Distribution of Oxepa

| | per 8 fl oz. | per liter | % of Cal |
|---|---|---|---|
| Calories | 355 | 1,500 | --- |
| Fat (g) | 22.2 | 93.7 | 55.2 |
| Carbohydrate | (g) | 25 | 105.528.1 |
| Protein (g) | 14.8 | 62.5 | 16.7 |
| Water (g) | 186 | 785 | --- |

Fat:

- Oxepa contains 22.2 g of fat per 8-fl oz serving (93.7 g/L).
- The fat source is an oil blend of 31.8% canola oil, 25% medium-chain triglycerides (MCTs), 20% borage oil, 20% fish oil, and 3.2 % soy lecithin. The typical fatty acid profile of Oxepa is shown in Table B.
- Oxepa provides a balanced amount of polyunsaturated, monounsaturated, and saturated fatty acids, as shown in Table VI.
- Medium-chain trigylcerides (MCTs) -- 25% of the fat blend -- aid gastric emptying because they are absorbed by the intestinal tract without emulsification by bile acids.

The various fatty acid components of Oxepa™ nutritional product can be substituted and/or supplemented with the PUFAs produced in accordance with this invention.

Table B. Typical Fatty Acid Profile

| Fatty Acids | % Total | g/8 fl oz*. | 9/L* |
|---|---|---|---|
| Caproic (6:0) | 0.2 | 0.04 | 0.18 |
| Caprylic (8:0) | 14.69 | 3.1 | 13.07 |
| Capric (10:0) | 11.06 | 2.33 | 9.87 |
| Palmitic (16:0) | 5.59 | 1.18 | 4.98 |
| Palmitoleic | 1.82 | 0.38 | 1.62 |
| Stearic | 1.94 | 0.39 | 1.64 |
| Oleic | 24.44 | 5.16 | 21.75 |
| Linoleic | 16.28 | 3.44 | 14.49 |
| α-Linolenic | 3.47 | 0.73 | 3.09 |
| γ-Linolenic | 4.82 | 1.02 | 4.29 |
| Eicosapentaenoic | 5.11 | 1.08 | 4.55 |
| n-3-Docosapentaenoic | 0.55 | 0.12 | 0.49 |
| Docosahexaenoic | 2.27 | 0.48 | 2.02 |
| Others | 7.55 | 1.52 | 6.72 |

Fatty acids equal approximately 95% of total fat.

Table C. Fat Profile of Oxepa.

| | |
|---|---|
| % of total calories from fat | 55.2 |
| Polyunsaturated fatty acids | 31.44 g/L |
| Monounsaturated fatty acids | 25.53 g/L |
| Saturated fatty acids | 32.38 g/L |
| n-6 to n-3 ratio | 1.75:1 |
| Cholesterol | 9.49 mg/8 fl oz 40.1 mg/L |

Carbohydrate:

- The carbohydrate content is 25.0 g per 8-fl-oz serving (105.5 g/L).
- The carbohydrate sources are 45% maltodextrin (a complex carbohydrate) and 55% sucrose (a simple sugar), both of which are readily digested and absorbed.
- The high-fat and low-carbohydrate content of Oxepa is designed to minimize carbon dioxide (CO2) production. High CO2 levels can complicate weaning in ventilator-dependent patients. The low level of carbohydrate also may be useful for those patients who have developed stress-induced hyperglycemia.
- Oxepa is lactose-free.

Dietary carbohydrate, the amino acids from protein, and the glycerol moiety of fats can be converted to glucose within the body. Throughout this process, the carbohydrate requirements of glucose-dependent tissues (such as the central nervous system and red blood cells) are met. However, a diet free of carbohydrates can lead to ketosis, excessive catabolism of tissue protein, and loss of fluid and electrolytes. These effects can be prevented by daily ingestion of 50 to 100 g of digestible carbohydrate, if caloric intake is adequate. The carbohydrate level in Oxepa is also sufficient to minimize gluconeogenesis, if energy needs are being met.

Protein:

- Oxepa contains 14.8 g of protein per 8-fl-oz serving (62.5 g/L).
- The total calorie/nitrogen ratio (150:1) meets the need of stressed patients.
- Oxepa provides enough protein to promote anabolism and the maintenance of lean body mass without precipitating respiratory problems. High protein intakes are a concern in patients with respiratory insufficiency. Although protein has little effect on CO2 production, a high protein diet will increase ventilatory drive.
- The protein sources of Oxepa are 86.8% sodium caseinate and 13.2% calcium caseinate.
- The amino acid profile of the protein system in Oxepa meets or surpasses the standard for high quality protein set by the National Academy of Sciences.

* Oxepa is gluten-free.

**Table 1**

*Saprolegnia diclina* (ATCC 56851) Desaturase Expression in Baker's Yeast

| CLONE | TYPE OF ENZYME ACTIVITY | % CONVERSION OF SUBSTRATE | |
|---|---|---|---|
| pRSP1 | Δ9 | 0 | (18:0 to 18:1n-9)* |
| (*S. diclina* Δ6 desaturase) | Δ12 | 0 | (18:1 to 18:2n-6) |
| | Δ15 | 0 | (18:2n-6 to 18:3n-3) |
| | Δ6 | 28 | (18:2n-6 to 18:3n-6) |
| | Δ6 | 37 | (18:3n-3 to 18:4n-3) |
| | Δ5 | 0 | (20:3n-6 to 20:4n-6) |
| pRSP3 | Δ9 | 0 | (18:0 to 18:1n-9) |
| (*S. diclina* Δ5 desaturase) | Δ12 | 0 | (18:1 to 18:2n-6) |
| | Δ15 | 0 | (18:2n-6 to 18:3n-3) |
| | Δ6 | 0 | (18:2n-6 to 18:3n-6) |
| | Δ6 | 0 | (18:3n-3 to 18:4n-3) |
| | Δ5 | 27 | (20:3n-6 to 20:4n-6) |

***above endogenous Δ9 activity**

**Table 2**

| Fatty Acid as a Percentage of Total Lipid Extracted from Yeast | | | | | | | | | |
|---|---|---|---|---|---|---|---|---|---|
| **Clone** | **18:1* Present** | **18:2 (n-3) Produced** | **18:2 (n-6) Incorporated** | **18:3 (n-3) Produced** | **18:3 (n-6) Produced** | **18:3 (n-3) Incorporated** | **18:4 (n-3) Produced** | **20:3(n-6) Incorporated** | **20:4 (n-6) Produced** |
| pYX242 (control) | 15.07 | 0 | 11.14 | 0 | 0 | 11.35 | 0 | 11.55 | 0 |
| pRSP1 (Δ6) | 14.41 | 0 | 6.31 | 0 | 2.44 | 7.95 | 4.63 | 13.70 | 0 |
| pRSP3 (Δ5) | 15.34 | 0.08 | 10.72 | 0 | 0 | 10.43 | 0 | 20.69 | 7.74 |

50 μM substrate added
*18:1 is an endogenous fatty acid in yeast Key:

18:1 = Oleic acid
18:2 (n-6) = Linoleic acid
18:3 (n-3) = α-Linolenic acid
18:3 (n-6) = γ-Linolenic acid
18:4 (n-3) = Stearidonic acid
20:3 (n-6) = Dihomo-γ-linolenic acid
20:4 (n-6) = Arachidonic acid

**Table 3**

| Fatty Acid as a Percentage of Total Lipid Extracted from Yeast | | | | | | | | | |
|---|---|---|---|---|---|---|---|---|---|
| Clone | Plasmid in yeast (enzyme) | 18:2 (n-6) Incorporated | 18:3 (n-6) Produced | 20:3 (n-6) Produced | % Conversion | 18:3 (n-3) Incorporated | 18:4 (n-3) Produced | 20:4 (n-3) Produced | % Conversion |
| Control | pYX242 + pYES2 | 6.46 | 0 | 0 | 0 | 13.26 | 0 | 0 | 0 |
| pRSP5 | pRSP1 (Δ6) + pRAE73-A3 (human elongase) | 4.62 | 1.95 | 0.8 | 37.3 | 7.00 | 2.47 | 1.20 | 34.39 |
| pRSP8 | pRSP1 (Δ6) + pRPB2 (M. alpina elongase) | 4.08 | 2.31 | 0.63 | 41 | 5.93 | 2.01 | 0.85 | 32.53 |

50 μM substrate added

Key:

18:2 (n-6) = Linoleic acid
18:3 (n-3) =α-Linolenic acid
18:3 (n-6) = γ-Linolenic acid
18:4 (n-3) = Stearidonic acid
20:3 (n-6) = Dihomo-γ-linolenic acid
20:4 (n-3) = Eicosatetraenoic acid

$$\% \text{ Conversion} = \frac{[\% \text{ Product } 1 + \% \text{ Product } 2]}{[\% \text{ substrate} + \% \text{ Product } 1 + \% \text{ Product } 2]}$$

**Table 4**

| Fatty Acid as a Percentage of Total Lipid Extracted from Yeast | | | | | | | | | |
|---|---|---|---|---|---|---|---|---|---|
| Clone | Plasmid in yeast (enzyme) | 18:3 (n-6) Incorporated | 20:3 (n-6) Produced | 20:4 (n-6) Produced | % Conversion | 18:4 (n-3) Incorporated | 20:4 (n-3) Produced | 20:5 (n-3) Produced | % Conversion |
| Control | pYX242 + pYES2 | 8.17 | 0 | 0 | 0 | 5.61 | 0 | 0 | 0 |
| pRSP7 | pRSP3 (Δ5)+ pRAE73-A3 (human elongase) | 6.25 | 2.30 | 1.63 | 38.6 | 4.12 | 1.98 | 1.56 | 46.2 |
| pRSP10 | pRSP3(Δ5)+ pRPB2 (M. alpina elongase) | 7.00 | 2.07 | 1.35 | 32.82 | 4.66 | 1.50 | 1.61 | 40.02 |

50 μM substrate added

Key:

18:3 (n-6) = γ-Linolenic acid
18:4 (n-3) = Stearidonic Acid
20:3 (n-6) = Dihomo-γ-linolenic acid
20:4 (n-6) = Arachidonic Acid
20:4 (n-3) = Eicosatetraenoic acid
20:5 (n-3) = Eicosapentanoic Acid

$$\% \text{ Conversion} = \frac{[\% \text{ Product 1} + \% \text{ Product 2}]}{[\% \text{ substrate} + \% \text{ Product 1} + \% \text{ Product 2}]}$$

**Table 5 (comparative)**

*Thraustochytrium aureum* (ATCC 34304) Desaturase Expression in Baker's Yeast

| CLONE | TYPE OF ENZYME ACTIVITY | | % CONVERSION OF SUBSTRATE |
|---|---|---|---|
| PRTA4 | Δ9 | 0 | (18:0 to 18:1n-9)* |
| (*T. aureum* Δ5 desaturase) | Δ12 | 0 | (18:1 to 18:2n-6) |
| | Δ15 | 0 | (18:2n-6 to 18:3n-3) |
| | Δ6 | 0 | (18:2n-6 to 18:3n-6) |
| | Δ6 | 0 | (18:3n-3 to 18:4n-3) |
| | Δ5 | 23.7 | (20:3n-6 to 20:4n-6) |
| | Δ17 | 0 | (20:4n-6 to 20:5n-3) |
| | Δ19 | 0 | (22:4n-6 to 22:5n-3) |
| | Δ4 | 0 | (22:4n-6 to 22:5n-6) |
| | Δ4 | 0 | (22:5n-3 to 22:6n-3) |

***above endogenous Δ9 activity**

**Table 6 (comparative)**

| Clone | 18:1* Present | 18:2 Produced | 18:2(n-6) Incorporated | 18:3(n-6) Produced | 18:3(n-6) Produced | 18:3(n-3) Incorporated | 18:4(n-3) Produced | 20:3(n-6) Incorporated | 20:4(n-6) Produced |
|---|---|---|---|---|---|---|---|---|---|
| | Fatty Acid as a Percentage of Total Lipid Extracted from Yeast | | | | | | | | |
| PYX242 (control) | 32.13 | 0 | 8.68 | 0 | 0 | 54.42 | 0 | 4.3 | 0 |
| PRTA4 (Δ5) | 29:67 | 0 | 11.18 | 0 | 0 | 9.93 | 0 | 21.94 | 6.84 |

50 μM substrate added
*18:1 is an endogenous fatty acid in yeast Key:

18:1 =Oleic acid
18:2(n-6) Linoleic acid
18:3(n-3) =α-Linolenic acid
18:3(n-6) =γ-Linolenic acid
18:4(n-3) =Stearidonic acid
20:3 (n-6) =Dihomo-γ-linolenic acid
20:4(n-6) =Arachidonic acid

**Table 7 (comparative)**

| Fatty Acid as a Percentage of Total Lipid Extracted from Yeast | | | | |
|---|---|---|---|---|
| **Clone** | **20:3 Incorporated** | **20:4 Produced** | **22:4 Produced** | **Conversion to products** |
| PYX242/ PYES2 (control) | 41.98 | 0 | 0 | 0 |
| PRTA4(Δ5)/ PRAE73-A3 (human elongase) | 15.59 | 4.2 | 6.28 | 16.7 |

100 μM substrate added
*18:1 is an endogenous fatty acid in yeast Key:

γ-18:3 =γ-Linolenic acid
20:3 =Dihomo-γ-linolenic acid
20:4 =Arachidonic acid
22:4 =Adrenic acid

Table 8 Fatty acid profiles of yeast containing pRAT-2c, pYX242, pRAT-2c/pRAE-73-A3, or pYX242/pYES2, grown in the presence of various fatty acids.

| plasmid 100 μM | pRAT-2c DGLA | pYX242 DGLA | pRAT-2c pRAE-73 LA | pYX242 pYES2 LA | pRAT-2c pRAE-73 ALA | pYX242 pYES2 ALA | pRAT-2c pRAE-73 GLA | pYX242 pYES2 GLA |
|---|---|---|---|---|---|---|---|---|
| | | | | g/100 g Fatty Acid | | | | |
| C18:2n-6 | | | 13.98 | 18.49 | | | | |
| C18:3n-6 | | | | | | | 15.97 | 15.1 |
| C18:3n-3 | | | | | 10.27 | 14.14 | | |
| C18:4n-3 | | | | | | | | |
| C20:2n-6 | | | 0.59 | 0.27 | | | | |
| C20:3n-6 | 33.37 | 32.4 | | | | | 1.25 | |
| C20:4n-6 | 22.98 | | | | | | 0.83 | |
| C20:3n-3 | | | | | 1.58 | 0.25 | | |
| C20:4n-3 | | | | | | | | |
| | | % conversion = [product/(substrate + product)] x 100 | | | | | | |
| elongase | | | 4.0% | 1.4% | 13.3% | 1.7% | 11.5% | |
| Δ5 | 40.8% | | | | | | 39.9% | |

Table 9 Fatty acid profiles of yeast containing pRAT-1a, pYX242, pRAT-1a/pRAE-73-A3, or pYX242/pYES2, grown in the presence of various fatty acids.

| Plasmid 100 μM | pRAT-1a LA | pYX242 LA | pRAT-1a ALA | pYX242 ALA | pRAT-1a EDA | pYX242 EDA | pRAT-1a DGLA | pYX242 DGLA | pRAT-1a pRAE-73 LA | pYX242 pYES2 LA | pRAT-1a pRAE-73 ALA | pYX242 pYES2 ALA |
|---|---|---|---|---|---|---|---|---|---|---|---|---|
| | | | | | | g/100 g Fatty Acid | | | | | | |
| C18:2n-6 | 18.44 | 20.91 | | | | | | | 11.35 | 18.49 | | |
| C18:3n-6 | 2.62 | | | | | | | | 3.49 | | | |
| C18:3n-3 | | | 11.59 | 12.38 | | | | | | | 4.61 | 14.14 |
| C18:4n-3 | | | 3.37 | | | | | | | | 3.69 | |
| C20:2n-6 | | | | | 55.6 | 55.55 | | | 0.21 | 0.27 | | |
| C20:3n-6 | | | | | 2.35 | | 66.37 | 32.4 | 1.1 | | | |
| C20:4n-6 | | | | | | | 0.78 | | | | | |
| C20:3n-3 | | | | | | | | | | | 0.65 | 0.25 |
| C20:4n-3 | | | | | | | | | | | 4.32 | |
| | | | | | | | | | | | | |
| | | % conversion = [product/(substrate + product)] x 100 | | | | | | | | | | |
| Δ6 | 12.4 % | 22.5% | | | | | | | | | | |
| Δ5 | | | | | | | 1.2% | | | | | |
| Δ8 | | | | | 4.0% | | | | | | | |

**Table 10 (comparative)**

| Fatty Acid as a Percentage of Total Lipid Extracted from Yeast | | | | | | | | |
|---|---|---|---|---|---|---|---|---|
| **Clone** | **18:3n-3** | **18:4n-3 Produced** | **20:3n-6** | **20:4n-6 Produced** | **percent conversion** | **20:4n-3** | **20:5n-3 Produced** | **percent conversion** |
| PYX242 (control) | 2.65 | 0 | 72 | 0 | 0 | 11.36 | 0.38 | 3.2 |
| PRIG1 (Δ5) | 2.28 | 0 | 12.17 | 10.12 | 45.4 | 8.43 | 12.59 | 59.75 |

50 uM substrate added Key:

18:3n-3 =αLinolenic acid
18:4n-3 =Stearidonic acid
20:3n-6 =Dihomo-γ-linolenic acid
20:4n-6 =Arachidonic acid
20:4n-3 =n-3 Eicosatetraenoic acid
20:5n-3 =Eicosapentaenoic acid

**percent conversion=** $$\frac{\text{product}}{\text{substrate + product}} \times 100$$

## Claims

1. An isolated nucleic acid comprising or complementary to a nucleotide sequence encoding a polypeptide having desaturase activity, wherein the amino acid sequence of said polypeptide has at least 90% sequence identity to an amino acid sequence selected from the group consisting of SEQ ID NO: 14 and SEQ ID NO: 20.

2. An isolated nucleotide sequence comprising or complementary to at least 90% of the nucleotide sequence selected from the group consisting of SEQ ID NO: 13 and SEQ ID NO: 19.

3. The isolated nucleotide sequence of claim 2 wherein said sequence is selected from the group consisting of SEQ ID NO: 13 and SEQ ID NO: 19.

4. The isolated nucleotide sequence of claim 2 or 3 wherein said sequence encodes a functionally active desaturase which utilizes a Polyunsaturated fatty acid as a substrate.

5. The nucleotide sequence of claim 4 wherein SEQ ID NO: 13 and SEQ ID NO: 19 are derived from *Saprolegnia diclina.*

6. A purified polypeptide encoded by said nucleotide sequence of claim 1, 2 or 3.

7. A purified polypeptide which desaturates polyunsaturated fatty acids at carbon 5 and has at least 90% amino acid identity to an amino acid sequence consisting of SEQ ID NO: 20.

8. A purified polypeptide which desaturates polyunsaturated fatty acids at carbon 6 and has at least 90% amino acid identity to an amino acid sequence consisting of SEQ ID NO: 14.

9. A method of producing a desaturase comprising the steps of:

a) isolating a nucleotide sequence selected from the group consisting of SEQ ID NO: 13 and SEQ ID NO: 19;
b) constructing a vector comprising: i) said isolated nucleotide sequence operably linked to ii) a regulatory sequence;
c) introducing said vector into a host cell for a time and under conditions sufficient for expression of said desaturase.

10. A vector comprising: a) a nucleotide sequence selected from the group consisting of SEQ ID NO: 13 and SEQ ID NO: 19, operably linked to b) a regulatory sequence.

11. A non-human host cell comprising said vector of claim 10.

12. A non-human mammalian cell comprising said vector of claim 10, wherein expression of said nucleotide sequence of said vector results in production of altered levels of AA, EPA, GLA or STA, when said cell is grown in a culture media comprising at least one fatty acid selected from the group consisting of LA, ALA, DGLA and ESP.

13. A plant cell, plant or plant tissue comprising said vector of claim 10, wherein expression of said nucleotide sequence of said vector results in production of a polyunsaturated fatty acid by said plant cell, plant or plant tissue.

14. The plant cell, plant or plant tissue of claim 13 wherein said polyunsaturated fatty acid is selected from the group consisting of AA, EPA, GLA and STA.

15. A transgenic plant comprising said vector of claim 10, wherein expression of said nucleotide sequence of said vector results in production of a polyunsaturated fatty acid in seeds of said transgenic plant.

16. A method for producing a polyunsaturated fatty acid comprising the steps of;

a) isolating a nucleotide sequence consisting of SEQ ID NO: 19;
b) constructing a vector comprising said isolated nucleotide sequence;
c) introducing said vector into a host cell for a time and under conditions sufficient for expression of Δ5- desaturase enzyme; and
d) exposing said expressed Δ5-desaturase enzyme to a substrate polyunsaturated fatty acid in order to convert said substrate to a product polyunsaturated fatty acid.

17. The method according to claim 16, wherein said substrate polyunsaturated fatty acid is DGLA or 20: 4n-3 and said product polyunsaturated fatty acid is AA or EPA, respectively.

18. The method according to claim 16 further comprising the step of exposing said product polyunsaturated fatty acid to an elongase in order to convert said product polyunsaturated fatty acid to another polyunsaturated fatty acid.

19. The method according to claim 18 wherein said product polyunsaturated fatty acid is AA or EPA and said another polyunsaturated fatty acid is adrenic acid or (n-3)-docosapentaenoic acid, respectively.

20. The method of claim 18 further comprising the step of exposing said another polyunsaturated fatty acid to an additional desaturase in order to convert said another polyunsaturated fatty acid to a final polyunsaturated fatty acid.

21. The method of claim 20 wherein said final polyunsaturated fatty acid is (n-6)-docosapentaenoic acid or docosahexaenoic (DHA) acid.

22. A method for producing a polyunsaturated fatty acid comprising the steps of:

a) isolating a nucleotide sequence consisting of SEQ ID NO: 13;
b) constructing a vector comprising said isolated nucleotide sequence;
c) introducing said vector into a host cell for a time and under conditions sufficient for expression of Δ6- desaturase enzyme; and
d) exposing said expressed Δ6-desaturase enzyme to a substrate polyunsaturated fatty acid in order to convert said substrate to a product polyunsaturated fatty acid.

23. The method according to claim 22, wherein said substrate polyunsaturated fatty acid is LA or ALA and said product polyunsaturated fatty acid is GLA or STA, respectively.

24. The method according to claim 22 further comprising the step of exposing said product polyunsaturated fatty acid to an elongase in order to convert said product polyunsaturated fatty acid to another polyunsaturated fatty acid.

25. The method according to claim 24 wherein said product polyunsaturated fatty acid is GLA or STA and said another polyunsaturated fatty acid is DGLA or ETA, respectively.

26. The method of claim 24 further comprising the step of exposing said another polyunsaturated fatty acid to an additional desaturase in order to convert said another polyunsaturated fatty acid to a final polyunsaturated fatty acid.

27. The method of claim 26 wherein said final polyunsaturated fatty acid is AA or EPA.

## Patentansprüche

1. Eine isolierte Nukleinsäure, die eine Nukleotidsequenz umfasst oder zu einer Nukleotidsequenz komplementär ist, die ein Polypeptid codiert, das Desaturaseaktivität hat, worin die Aminosäuresequenz des Polypeptids mindestens 90% Sequenzidentität mit einer Aminosäuresequenz hat, die gewählt ist aus der Gruppe bestehend aus Sequenzidentifikationsnr. 14 und Sequenzidentifikationsnr. 20.

2. Eine isolierte Nukleotidsequenz, die mindestens 90% der Nukleotidsequenz umfasst oder zur Nukleotidsequenz komplementär ist, die gewählt ist aus der Gruppe bestehend aus Sequenzidentifikationsnr. 13 und Sequenzidentifikationsnr. 19.

3. Die isolierte Nukleotidsequenz gemäß Anspruch 2, worin die Sequenz gewählt ist aus der Gruppe bestehend aus Sequenzidentifikationsnr. 13 und Sequenzidentifikationsnr. 19.

4. Die isolierte Nukleotidsequenz gemäß Anspruch 2 oder 3, worin die Sequenz eine funktionell aktive Desaturase codiert, die eine mehrfach ungesättigte Fettsäure als Substrat nutzt.

5. Die Nukleotidsequenz gemäß Anspruch 4, worin Sequenzidentifikationsnr. 13 und Sequenzidentifikationsnr. 19 von *Saprolegnia diclina* abgeleitet sind.

6. Ein gereinigtes Polypeptid, codiert durch die Nukleotidsequenz gemäß Anspruch 1, 2 oder 3.

7. Ein gereinigtes Polypeptid, das mehrfach ungesättigte Fettsäuren am Kohlenstoff 5 entsättigt und mindestens 90% Aminosäureidentität mit einer Aminosäuresequenz hat, die aus Sequenzidentifikationsnr. 20 besteht.

8. Ein gereinigtes Polypeptid, das mehrfach ungesättigte Fettsäuren am Kohlenstoff 6 entsättigt und mindestens 90% Aminosäureidentität mit einer Aminosäuresequenz hat, die aus Sequenzidentifikationsnr. 14 besteht.

9. Ein Verfahren zur Herstellung einer Desaturase, das folgende Schritte umfasst:

   a) Isolieren einer Nukleotidsequenz, die gewählt ist aus der Gruppe bestehend aus Sequenzidentifikationsnr. 13 und Sequenzidentifikationsnr. 19,
   b) Konstruktion eines Vektors, der Folgendes umfasst: i) die isolierte Nukleotidsequenz, operativ verknüpft mit ii) einer regulatorischen Sequenz,
   c) Einführen des Vektors in eine Wirtszelle über einen Zeitraum und unter Bedingungen, die zur Expression der Desaturase ausreichen.

10. Ein Vektor, der Folgendes umfasst: a) eine Nukleotidsequenz, gewählt aus der Gruppe bestehend aus Sequenzidentifikationsnr. 13 und Sequenzidentifikationsnr. 19, operativ verknüpft mit b) einer regulatorischen Sequenz.

11. Eine nicht menschliche Wirtszelle, die den Vektor gemäß Anspruch 10 umfasst.

12. Eine nicht menschliche Säugetierzelle, die den Vektor gemäß Anspruch 10 umfasst, worin die Expression der Nukleotidsequenz des Vektors zu der Produktion veränderter Mengen von AA, EPA, GLA oder STA führt, wenn die Zelle in einem Kulturmedium gezüchtet wird, das mindestens eine Fettsäure, gewählt aus der Gruppe bestehend aus LA, ALA, DGLA und ESP, umfasst.

**13.** Eine Pflanzenzelle, Pflanze oder ein Pflanzengewebe, die/das den Vektor gemäß Anspruch 10 umfasst, worin die Expression der Nukleotidsequenz des Vektors zu der Produktion einer mehrfach ungesättigten Fettsäure durch die Pflanzenzelle, Pflanze oder das Pflanzengewebe führt.

**14.** Die Pflanzenzelle, Pflanze oder das Pflanzengewebe gemäß Anspruch 13, worin die mehrfach ungesättigte Fettsäure gewählt ist aus der Gruppe bestehend aus AA, EPA, GLA und STA.

**15.** Eine transgene Pflanze, die den Vektor gemäß Anspruch 10 umfasst, worin die Expression der Nukleotidsequenz des Vektors zu der Produktion einer mehrfach ungesättigten Fettsäure in Samen der transgenen Pflanze führt.

**16.** Ein Verfahren zur Herstellung einer mehrfach ungesättigten Fettsäure, das folgende Schritte umfasst:

a) Isolieren einer Nukleotidsequenz, die aus Sequenzidentifikationsnr. 19 besteht,
b) Konstruktion eines Vektors, der die isolierte Nukleotidsequenz umfasst,
c) Einführen des Vektors in eine Wirtszelle für einen Zeitraum und unter Bedingungen, die zur Expression von $\Delta$5-Desaturaseenzym ausreichen, und
d) Aussetzen des exprimierten $\Delta$5-Desaturaseenzyms gegenüber einer mehrfach ungesättigten Substrat-Fettsäure, um das Substrat in eine mehrfach ungesättigte Produkt-Fettsäure zu umwandeln.

**17.** Das Verfahren gemäß Anspruch 16, worin die mehrfach ungesättigte Substrat-Fettsäure DGLA oder 20: 4n-3 ist und die mehrfach ungesättigte Produkt-Fettsäure AA beziehungsweise EPA ist.

**18.** Das Verfahren gemäß Anspruch 16, das weiter den Schritt des Aussetzens der mehrfach ungesättigten Produkt-Fettsäure gegenüber einer Elongase umfasst, um die mehrfach ungesättigte Produkt-Fettsäure in eine andere mehrfach ungesättigte Fettsäure zu umwandeln.

**19.** Das Verfahren gemäß Anspruch 18, worin die mehrfach ungesättigte Produkt-Fettsäure AA oder EPA ist und die andere mehrfach ungesättigte Fettsäure Adrensäure beziehungsweise (n-3)-Docosapentaensäure ist.

**20.** Das Verfahren gemäß Anspruch 18, das weiter den Schritt des Aussetzens der anderen mehrfach ungesättigten Fettsäure gegenüber einer weiteren Desaturase umfasst, um die andere mehrfach ungesättigte Fettsäure in eine endgültige mehrfach ungesättigte Fettsäure zu umwandeln.

**21.** Das Verfahren gemäß Anspruch 20, worin die endgültige mehrfach ungesättigte Fettsäure (n-6)-Docosapentaensäure oder Docosahexaensäure (DHA-Säure) ist.

**22.** Ein Verfahren zur Herstellung einer mehrfach ungesättigten Fettsäure, das folgende Schritte umfasst:

a) Isolation einer Nukleotidsequenz, die aus Sequenzidentifikationsnr. 13 besteht,
b) Konstruktion eines Vektors, der die isolierte Nukleotidsequenz umfasst,
c) Einführen des Vektors in eine Wirtszelle für einen Zeitraum und unter Bedingungen, die zur Expression von $\Delta$6-Desaturaseenzym ausreichen, und
d) Aussetzen des exprimierten $\Delta$6-Desaturaseenzyms gegenüber einer mehrfach ungesättigten Substrat-Fettsäure, um das Substrat in eine mehrfach ungesättigte Produkt-Fettsäure zu umwandeln.

**23.** Das Verfahren gemäß Anspruch 22, worin die mehrfach ungesättigte Substrat-Fettsäure LA oder ALA ist und die mehrfach ungesättigte Produkt-Fettsäure GLA beziehungsweise STA ist.

**24.** Das Verfahren gemäß Anspruch 22, das weiter den Schritt des Aussetzens der mehrfach ungesättigten Produkt-Fettsäure gegenüber einer Elongase umfasst, um die mehrfach ungesättigte Produkt-Fettsäure in eine andere mehrfach ungesättigte Fettsäure zu umwandeln.

**25.** Das Verfahren gemäß Anspruch 24, worin die mehrfach ungesättigte Produkt-Fettsäure GLA oder STA ist und die andere mehrfach ungesättigte Fettsäure DGLA beziehungsweise ETA ist.

26. Das Verfahren gemäß Anspruch 24, das weiter den Schritt des Aussetzens der anderen mehrfach ungesättigten Fettsäure gegenüber einer weiteren Desaturase umfasst, um die andere mehrfach ungesättigte Fettsäure in eine endgültige mehrfach ungesättigte Fettsäure zu umwandeln.

27. Das Verfahren gemäß Anspruch 26, worin die endgültige mehrfach ungesättigte Fettsäure AA oder EPA ist.

**Revendications**

1. Acide nucléique isolé comprenant ou complémentaire d'une séquence nucléotidique codant un polypeptide ayant une activité désaturase, où la séquence d'aminoacide dudit polypeptide a au moins 90 % d'identité de séquence avec une séquence d'aminoacides choisie dans le groupe consistant en SEQ ID NO:14 et SEQ ID NO:20.

2. Séquence nucléotidique isolée comprenant ou complémentaire d'au moins 90 % de la séquence nucléotidique choisie dans le groupe consistant en SEQ ID NO:13 et SEQ ID NO:19.

3. Séquence nucléotidique isolée selon la revendication 2 où ladite séquence est choisie dans le groupe consistant en SEQ ID NO:13 et SEQ ID NO:19.

4. Séquence nucléotidique isolée selon la revendication 2 ou 3 où ladite séquence code une désaturase fonctionnellement active qui utilise un acide gras polyinsaturé comme substrat.

5. Séquence nucléotidique selon la revendication 4 où SEQ ID NO:13 et SEQ ID NO:19 sont dérivés de *Saprolegnia diclina.*

6. Polypeptide purifié codé par ladite séquence nucléotidique selon la revendication 1, 2 ou 3.

7. Polypeptide purifié qui désature les acides gras polyinsaturés au carbone 5 et a au moins 90 % d'identité d'aminoacides avec une séquence d'aminoacide consistant en SEQ ID NO:20.

8. Polypeptide purifié qui désature les acides gras polyinsaturés au carbone 6 et a au moins 90 % d'identité d'aminoacides avec une séquence d'aminoacides consistant en SEQ ID NO:14.

9. Procédé de production d'une désaturase comprenant les étapes de :

   a) isoler une séquence nucléotidique choisie dans le groupe consistant en SEQ ID NO:13 et SEQ ID NO:19 ;
   b) construire un vecteur comprenant : i) ladite séquence nucléotidique isolée liée de manière fonctionnelle à ii) une séquence régulatrice ;
   c) introduire ledit vecteur dans une cellule hôte pendant une durée et dans des conditions suffisantes pour l'expression de ladite désaturase.

10. Vecteur comprenant : a) une séquence nucléotidique choisie dans le groupe consistant en SEQ ID NO:13 et SEQ ID NO:19, liée de manière fonctionnelle à b) une séquence régulatrice.

11. Cellule hôte non humaine comprenant ledit vecteur selon la revendication 10.

12. Cellule de mammifère non humain comprenant ledit vecteur selon la revendication 10, où l'expression de ladite séquence nucléotidique dudit vecteur conduit à la production de niveaux modifiés de AA, EPA, GLA ou STA, quand ladite cellule est cultivée dans un milieu de culture comprenant au moins un acide gras choisi dans le groupe consistant en LA, ALA, DGLA et ESP.

13. Cellule végétale, plante ou tissu végétal comprenant ledit vecteur selon la revendication 10, où l'expression de ladite séquence nucléotidique dudit vecteur conduit à la production d'un acide gras polyinsaturé par ladite cellule végétale, ladite plante ou ledit tissu végétal.

14. Cellule végétale, plante ou tissu végétal selon la revendication 13 où ledit acide gras polyinsaturé est choisi dans le groupe consistant en AA, EPA, GLA et STA.

**15.** Plante transgénique comprenant ledit vecteur selon la revendication 10, où l'expression de ladite séquence nucléotidique dudit vecteur conduit à la production d'un acide gras polyinsaturé dans les graines de ladite plante transgénique.

**16.** Procédé pour produire un acide gras polyinsaturé comprenant les étapes de :

a) isoler une séquence nucléotidique consistant en SEQ ID NO:19 ;
b) construire un vecteur comprenant ladite séquence nucléotidique isolée ;
c) introduire ledit vecteur dans une cellule hôte pendant une durée et dans des conditions suffisantes pour l'expression d'une enzyme Δ5-désaturase ; et
d) exposer ladite enzyme Δ5-désaturase exprimée à un acide gras polyinsaturé formant substrat pour convertir ledit substrat en un acide gras polyinsaturé formant produit.

**17.** Procédé selon la revendication 16 où ledit acide gras polyinsaturé formant substrat est DGLA ou 20 : 4n-3 et ledit acide gras polyinsaturé formant produit est AA ou EPA, respectivement.

**18.** Procédé selon la revendication 16 comprenant en outre l'étape d'exposition dudit acide gras polyinsaturé formant produit à une élongase pour convertir ledit acide gras polyinsaturé formant produit en un autre acide gras polyinsaturé.

**19.** Procédé selon la revendication 18 où ledit acide gras polyinsaturé formant produit est AA ou EPA et ledit autre acide gras polyinsaturé est l'acide adrénique ou l'acide (n-3)-docosapentaénoïque, respectivement.

**20.** Procédé selon la revendication 18 comprenant en outre l'étape d'exposition dudit autre acide gras polyinsaturé à une désaturase supplémentaire pour convertir ledit autre acide gras polyinsaturé en un acide gras polyinsaturé final.

**21.** Procédé selon la revendication 20 où ledit acide gras polyinsaturé final est l'acide (n-6)-docosapentaénoïque ou l'acide docosahexaénoïque (DHA).

**22.** Procédé pour produire un acide gras polyinsaturé comprenant les étapes de :

a) isoler une séquence nucléotidique consistant en SEQ ID NO:13 ;
b) construire un vecteur comprenant ladite séquence nucléotidique isolée ;
c) introduire ledit vecteur dans une cellule hôte pendant une durée et dans des conditions suffisantes pour l'expression d'une enzyme Δ6-désaturase ; et
d) exposer ladite enzyme Δ6-désaturase exprimée à un acide gras polyinsaturé formant substrat pour convertir ledit substrat en un acide gras polyinsaturé formant produit.

**23.** Procédé selon la revendication 22 où ledit acide gras polyinsaturé formant substrat est LA ou ALA et ledit acide gras polyinsaturé formant produit est GLA ou STA, respectivement.

**24.** Procédé selon la revendication 22 comprenant en outre l'étape d'exposition dudit acide gras polyinsaturé formant produit à une élongase pour convertir ledit acide gras polyinsaturé formant produit en un autre acide gras polyinsaturé.

**25.** Procédé selon la revendication 24 où ledit acide gras polyinsaturé formant produit est GLA ou STA et ledit autre acide gras polyinsaturé est DGLA OU ETA, respectivement.

**26.** Procédé selon la revendication 24 comprenant en outre l'étape d'exposition dudit autre acide gras polyinsaturé à une désaturase supplémentaire pour convertir ledit autre acide gras polyinsaturé en un acide gras polyinsaturé final.

**27.** Procédé selon la revendication 26 où ledit acide gras polyinsaturé final est AA ou EPA.

**Figure 1**
Fatty Acid Biosynthetic Pathway

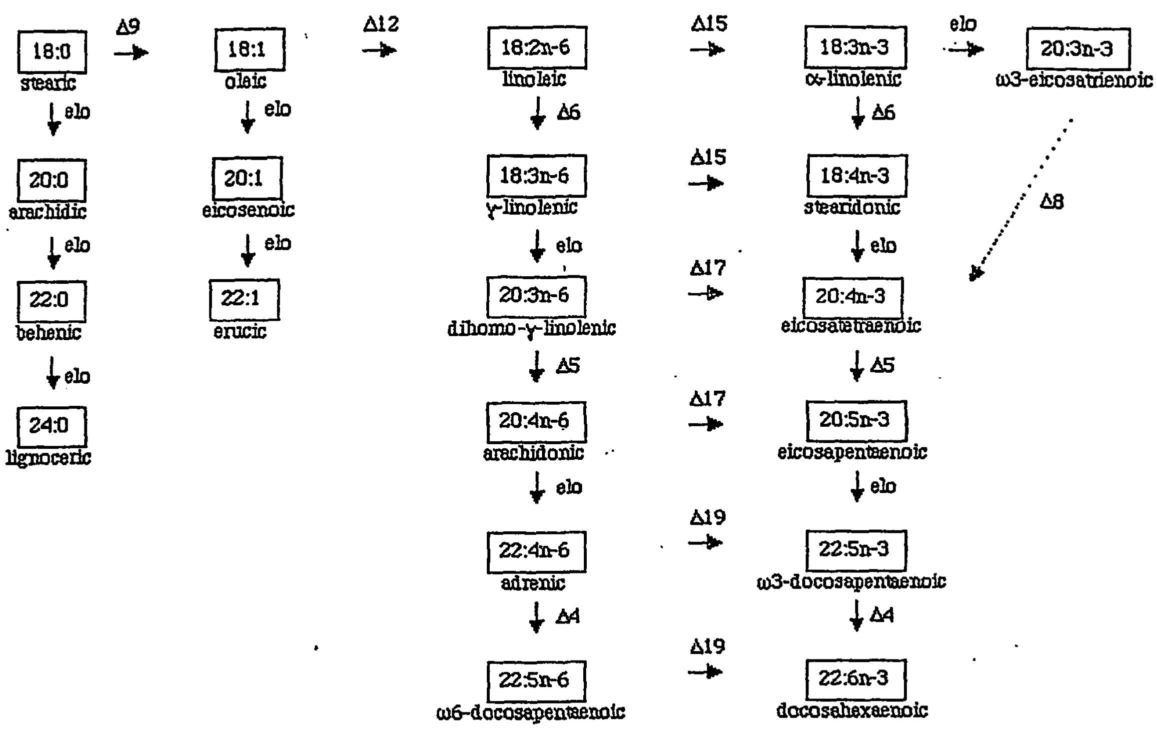

## Figure 2

*Gene Sequence of Delta 6- Desaturase from Saprolegnia diclina* (ATCC 56851)

```
1     ATGGTCCAGG GGCAAAAGGC CGAGAAGATC TCGTGGGCGA CCATCCGTGA
51    GCACAACCGC CAAGACAACG CGTGGATCGT GATCCACCAC AAGGTGTACG
101   ACATCTCGGC CTTTGAGGAC CACCCGGGCG GCGTCGTCAT GTTCACGCAG
151   GCCGGCGAAG ACGCGACCGA TGCGTTCGCT GTCTTCCACC CGAGCTCGGC
201   GCTCAAGCTC CTCGAGCAGT ACTACGTCGG CGACGTCGAC CAGTCGACGG
251   CGGCCGTCGA CACGTCGATC TCGGACGAGG TCAAGAAGAG CCAGTCGGAC
301   TTCATTGCGT CGTACCGCAA GCTGCGCCTT GAAGTCAAGC GCCTCGGCTT
351   GTACGACTCG AGCAAGCTCT ACTACCTCTA CAAGTGCGCC TCGACGCTGA
401   GCATTGCGCT TGTGTCGGCG GCCATTTGCC TCCACTTTGA CTCGACGGCC
451   ATGTACATGG TCGCGGCTGT CATCCTTGGC CTCTTTTACC AGCAGTGCGG
501   CTGGCTCGCC CATGACTTTC TGCACCACCA AGTGTTTGAG AACCACTTGT
551   TTGGCGACCT CGTCGGCGTC ATGGTCGGCA ACCTCTGGCA GGGCTTCTCG
601   GTGCAGTGGT GGAAGAACAA GCACAACACG CACCATGCGA TCCCCAACCT
651   CCACGCGACG CCCGAGATCG CCTTCCACGG CGACCCGGAC ATTGACACGA
701   TGCCGATTCT CGCGTGGTCG CTCAAGATGG CGCAGCACGC GGTCGACTCG
751   CCCGTCGGGC TCTTCTTCAT GCGCTACCAA GCGTACCTGT ACTTTCCCAT
801   CTTGCTCTTT GCGCGTATCT CGTGGGTGAT CCAGTCGGCC ATGTACGCCT
851   TCTACAACGT TGGGCCCGGC GGCACCTTTG ACAAGGTCCA GTACCCGCTG
901   CTCGAGCGCG CCGGCCTCCT CCTCTACTAC GGCTGGAACC TCGGCCTTGT
951   GTACGCAGCC AACATGTCGC TGCTCCAAGC GGCTGCGTTC CTCTTTGTGA
1001  GCCAGGCGTC GTGCGGCCTC TTCCTCGCGA TGGTCTTTAG CGTCGGCCAC
1051  AACGGCATGG AGGTCTTTGA CAAGGACAGC AAGCCCGATT TTTGGAAGCT
1101  GCAAGTGCTC TCGACGCGCA ACGTGACGTC GTCGCTCTGG ATCGACTGGT
1151  TCATGGGCGG CCTCAACTAC CAGATCGACC ACCACTTGTT CCCGATGGTG
1201  CCCCGGCACA ACCTCCCGGC GCTCAACGTG CTCGTCAAGT CGCTCTGCAA
1251  GCAGTACGAC ATCCCATACC ACGAGACGGG CTTCATCGCG GGCATGGCCG
1301  AGGTCGTCGT GCACCTCGAG CGCATCTCGA TCGAGTTCTT CAAGGAGTTT
1351  CCCGCCATGT AA
```

## Figure 3

Amino Acid Sequence of Delta 6- Desaturase from *Saprolegnia diclina* (ATCC 56851)

```
1    MVQGQKAEKI SWATIREHNR QDNAWIVIHH KVYDISAFED HPGGVVMFTQ
51   AGEDATDAFA VFHPSSALKL LEQYYVGDVD QSTAAVDTSI SDEVKKSQSD
101  FIASYRKLRL EVKRLGLYDS SKLYYLYKCA STLSIALVSA AICLHFDSTA
151  MYMVAAVIIG LFYQQCGWLA HDFLHHQVFE NHLFGDLVGV MVGNLWQGFS
201  VQWWKNKHNT HHAIPNLHAT PEIAFHGDPD IDTMPILAWS LKMAQHAVDS
251  PVGLFFMRYQ AYLYFPILLF ARISWVIQSA MYAFYNVGPG GTFDKVQYPL
301  LERAGLLLYY GWNLGLVYAA NMSLLQAAAF LFVSQASCGL FLAMVFSVGH
351  NGMEVFDKDS KPDFWKLQVL STRNVTSSLW IDWFMGGLNY QIDHHLFPMV
401  PRHNLPALNV LVKSLCKQYD IPYHETGFIA GMAEVVVHLE RISIEFFKEF
451  PAM*
```

## Figure 4

Gene Sequence of Delta 5- Desaturase from *Saprolegnia diclina* (ATCC 56851)

```
   1  ATGGCCCCGC AGACGGAGCT CCGCCAGCGC CACGCCGCCG TCGCCGAGAC
  51  GCCGGTGGCC GGCAAGAAGG CCTTTACATG GCAGGAGGTC GCGCAGCACA
 101  ACACGGCGGC CTCGGCCTGG ATCATTATCC GCGGCAAGGT CTACGACGTG
 151  ACCGAGTGGG CCAACAAGCA CCCCGGCGGC CGCGAGATGG TGCTGCTGCA
 201  CGCCGGTCGC GAGGCCACCG ACACGTTCGA CTCGTACCAC CCGTTCAGCG
 251  ACAAGGCCGA GTCGATCTTG AACAAGTATG AGATTGGCAC GTTCACGGGC
 301  CCGTCCGAGT TTCCGACCTT CAAGCCGGAC ACGGGCTTCT ACAAGGAGTG
 351  CCGCAAGCGC GTTGGCGAGT ACTTCAAGAA GAACAACCTC CATCCGCAGG
 401  ACGGCTTCCC GGGCCTCTGG CGCATGATGG TCGTGTTTGC GGTCGCCGGC
 451  CTCGCCTTGT ACGGCATGCA CTTTTCGACT ATCTTTGCGC TGCAGCTCGC
 501  GGCCGCGGCG CTCTTTGGCG TCTGCCAGGC GCTGCCGCTG CTCCACGTCA
 551  TGCACGACTC GTCGCACGCG TCGTACACCA ACATGCCGTT CTTCCATTAC
 601  GTCGTCGGCC GCTTTGCCAT GGACTGGTTT GCCGGCGGCT CGATGGTGTC
 651  ATGGCTCAAC CAGCACGTCG TGGGCCACCA CATCTACACG AACGTCGCGG
 701  GCTCGGACCC GGATCTTCCG GTCAACATGG ACGGCGACAT CCGCCGCATC
 751  GTGAACCGCC AGGTGTTCCA GCCCATGTAC GCATTCCAGC ACATCTACCT
 801  TCCGCCGCTC TATGGCGTGC TTGGCCTCAA GTTCCGCATC CAGGACTTCA
 851  CCGACACGTT CGGCTCGCAC ACGAACGGCC CGATCCGCGT CAACCCGCAC
 901  GCGCTCTCGA CGTGGATGGC CATGATCAGC TCCAAGTCGT TCTGGGCCTT
 951  CTACCGCGTG TACCTTCCGC TTGCCGTGCT CCAGATGCCC ATCAAGACGT
1001  ACCTTGCGAT CTTCTTCCTC GCCGAGTTTG TCACGGGCTG GTACCTCGCG
1051  TTCAACTTCC AAGTAAGCCA TGTCTCGACC GAGTGCGGCT ACCCATGCGG
1101  CGACGAGGCC AAGATGGCGC TCCAGGACGA GTGGGCAGTC TCGCAGGTCA
1151  AGACGTCGGT CGACTACGCC CATGGCTCGT GGATGACGAC GTTCCTTGCC
1201  GGCGCGCTCA ACTACCAGGT CGTGCACCAC TTGTTCCCCA GCGTGTCGCA
1251  GTACCACTAC CCGGCGATCG CGCCCATCAT CGTCGACGTC TGCAAGGAGT
1301  ACAACATCAA GTACGCCATC TTGCCGGACT TTACGGCGGC GTTCGTTGCC
1351  CACTTGAAGC ACCTCCGCAA CATGGGCCAG CAGGGCATCG CCGCCACGAT
1401  CCACATGGGC TAA
```

## Figure 5

Amino Acid Sequence of Delta 5- Desaturase from *Saprolegnia diclina* (ATCC 56851)

```
1     MAPQTELRQR HAAVAETPVA GKKAFTWQEV AQHNTAASAW IIIRGKVYDV
51    TEWANKHPGG REMVLLHAGR EATDTFDSYH PFSDKAESIL NKYEIGTFTG
101   PSEFPTFKPD TGFYKECRKR VGEYFKKNNL HPQDGFPGLW RMMVVFAVAG
151   LALYGMHFST IFALQLAAAA LFGVCQALPL LHVMHDSSHA SYTNMPFFHY
201   VVGRFAMDWF AGGSMVSWLN QHVVGHHIYT NVAGSDPDLP VNMDGDIRRI
251   VNRQVFQPMY AFQHIYLPPL YGVLGLKFRI QDFTDTFGSH TNGPIRVNPH
301   ALSTWMAMIS SKSFWAFYRV YLPLAVLQMP IKTYLAIFFL AEFVTGWYLA
351   FNFQVSHVST ECGYPCGDEA KMALQDEWAV SQVKTSVDYA HGSWMTTFLA
401   GALNYQVVHH LFPSVSQYHY PAIAPIIVDV CKEYNIKYAI LPDFTAAFVA
451   HLKHLRNMGQ QGIAATIHMG *
```

**Figure 6** (comparative)

Gene Sequence of Delta 5- Desaturase from *Thraustochytrium aureum* (ATCC 34304)

```
1     ATGGGACGCG GCGGCGAAGG TCAGGTGAAC AGCGCGCAGG TGGCACAAGG
51    CGGTGCGGGA ACGCGAAAGA CGATCCTGAT CGAGGGCGAG GTCTACGATG
101   TCACCAACTT TAGGCACCCC GGCGGGTCGA TCATCAAGTT TCTCACGACC
151   GACGGCACCG AGGCTGTGGA CGCGACGAAC GCGTTTCGCG AGTTTCACTG
201   CCGGTCGGGC AAGGCGGAAA AGTACCTCAA GAGCCTGCCC AAGCTCGGCG
251   CGCCGAGCAA GATGAAGTTT GACGCCAAGG AGCAGGCCCG GCGCGACGCG
301   ATCACGCGAG ACTACGTCAA GCTGCGCGAG GAGATGGTGG CCGAGGGCCT
351   CTTCAAGCCC GCGCCCCTCC ACATTGTCTA CAGGTTTGCG GAGATCGCAG
401   CCCTGTTCGC GGCCTCGTTC TACCTGTTTT CGATGCGCGG AAACGTGTTC
451   GCCACGCTCG CGGCCATCGC AGTCGGGGGC ATCGCGCAGG GCCGCTGCGG
501   CTGGCTCATG CACGAGTGCG GACACTTCTC GATGACCGGG TACATCCCGC
551   TTGACGTGCG CCTGCAGGAG CTGGTGTACG GCGTGGGGTG CTCGATGTCG
601   GCGAGCTGGT GGCGCGTTCA GCACAACAAG CACCACGCGA CCCCGCAGAA
651   ACTCAAGCAC GACGTCGACC TCGACACCCT GCCGCTCGTT GCGTTCAACG
701   AGAAGATCGC CGCCAAGGTG CGCCCCGGCT CGTTCCAGGC CAAGTGGCTC
751   TCGGCGCAGG CGTACATTTT TGCGCCGGTG TCCTGCTTCC TGGTTGGTCT
801   CTTCTGGACC CTGTTTCTGC ACCCGCGCCA CATGCCGCGC ACGAGCCACT
851   TTGCTGAGAT GGCCGCCGTC GCGGTGCGCG TCGTGGGCTG GGCGGCGCTC
901   ATGCACTCGT TCGGGTACAG CGGGAGCGAC TCGTTCGGTC TCTACATGGC
951   CACCTTTGGC TTTGGCTGCA CCTACATCTT CACCAACTTT GCGGTCAGCC
1001  ACACGCACCT CGACGTCACC GAGCCGGACG AGTTCCTGCA CTGGGTCGAG
1051  TACGCCGCGC TGCACACGAC CAACGTGTCC AACGACTCGT GGTTCATCAC
1101  CTGGTGGATG TCGTACCTCA ACTTTCAGAT CGAGCACCAC CTCTTTCCGT
1151  CGCTGCCCCA GCTCAACGCC CCGCGCGTCG CCCCGCGCGT CCGCGCCCTC
1201  TTCGAGAAGC ACGGCATGGC TTACGACGAG CGCCCGTACC TTACCGCGCT
1251  TGGCGACACG TTTGCCAACC TGCACGCCGT GGGCCAAAAC GCGGGCCAGG
1301  CGGCGGCCAA GGCCGCTTAG
```

**Figure 7** (comparative)

Amino Acid Sequence of Delta 5- Desaturase from *Thraustochytrium aureum* (ATCC 34304)

```
  1  MSRGGEGQVN SAQVAQGGAS TRKTILIEGE VYDVTNFRHP GGSIIKFLTT
 51  DGTEAVDATN AFREFHCRSG KAEKYLKSLP KLGAPSKMKF DAKEQARRDA
101  ITRDYVKLRE EMVAEGLFKP APLHIVYRFA EIAALFAASF YLFSMRGNVF
151  ATLAAIAVGG IAQGRCGWLM HECGHFSMTG YIPLDVRLQE LVYGVGCSMS
201  ASWWRVQHNK HHATPQKLKH DVDLDTLPLV AFNEKIAAKV RPGSFQAKWL
251  SAQAYIFAPV SCFLVGLFWT LFLHPRHMPR TSHFAEMAAV AVRVVGWAAL
301  MHSFGYSGSD SFGLYMATFG FGCTYIFTNF AVSHTHLDVT EPDEFLHWVE
351  YAALHTTNVS NDSWFITWWM SYLNFQIEHH LFPSLPQLNA PRVAPRVRAL
401  FEKHGMAYDE RPYLTALGDT FANLHAVGQN ASQAAAKAA
```

Figure 8 (comparative)

```
   1  GAATTCACCA TGGGTCGCGG AGCACAGGGA GAGCCAAGGC AGGCCACAGA
  51  GCTGAAGAGC AGCCCAAGTG AGCAGCGTAA GGTGTTGCTC ATTGACGGGC
 101  AGCTGTACGA TGCAACCAAC TTCAGGCATC CTGGTGGCTC CATCATCAAA
 151  TATTTGTGCA CCGATGGCAA GGAGGTAGTT GATGCAACCG AAGCGTACAA
 201  GGAGTTCCAC TGCAGATCCT CGAAGGCGGT CAAGTACCTC AACTCCCTGC
 251  CAAAGATCGA CGGCCCAATC AAGTACAAAT ACGACGCAAA GGAGCAGGCT
 301  CGCCATGACA AACTCACGAG GGAGTATGTA GCTCTCCGCG AACAGCTCGT
 351  CAAGGAGGGA TACTTTGACC CCAGCCCGCT CCACATTATC TACAGATGCG
 401  CCGAGTTGGC AGCCATGTTC GCTCTCTCGT TCTACCTTTT CTCCTTCAAG
 451  GGTAACGTCA TGGCCACTAT TGCTGCCATC GTGATTGGGG GGTGCGTGCA
 501  GGGTCGTTGT GGGTGGCTCA TGCATGAAGC TGGCCACTAC AGCATGACCG
 551  GAAACATCCC TGTTGACTTG CGCCTTCAAG AGTTTTTGTA CGGAATTGGG
 601  TGTGGCATGA GCGGGGCTTG GTGGAGAAGC CAGCACAACA AGCACCACGC
 651  CACCCCCCAA AAGCTCAAGC ATGACGTTGA TTTGGACACT CTTCCTCTTG
 701  TCGCCTGGAA CGAGAAAATT GCCCGTCGCG TCAAGCCAGG TAGCTTCCAG
 751  GCAAAGTGGC TTCATCTCCA GGGATACATC TTTGCCCCAG TCTCCTGCCT
 801  TCTCGTTGGT CTCTTCTGGA CTTTGTACTT GCATCCTCGC CACATGATCC
 851  GCACCAAGCG CAACTTCGAG ATATTTTCTG TCGCTCTGCG CTACGTATGC
 901  TGGTTCTCGC TTCTTTTGAG CATGGGCTAC ACTGTCGGAG AGTCTCTGGG
 951  TCTCTATGTG CTTACTTTTG GACTTGGCTG TACCTACATC TTTACGCATT
1001  TTGCTGTAAG CCACACCCAC TTGCCAGTGT CCGAGGAGGA CGAGTACCTG
1051  CACTGGGTCG AGTACGCTGC GCTGCACACC ACGAACGTTG CCATCGACTC
1101  GTACGTTGTC ACCTGGCTGA TGAGCTACCT CAACTTTCAG ATCGAGCACC
1151  ACTTGTTCCC TTGCTGCCCG CAGTTCCGCC ACCCTGCAAT CTCTTCTCGC
1201  GTCAAGAAAC TTTTCGAGGA CAATGGTCTG GTATACGACG CCCGCTCATA
1251  CGTCCAGGCG CTCAAGGATA CCTTCGGCAA CCTACACGAA GTGGGCGTCA
1301  ACGCTGGCCA AGCTGCCAAG AGCGAGTAAG ATCTCGAG
```

**Figure 9** (comparative)

```
  1 MGRGAQGEPR QATELKSSPS EQRKVLLIDG QLYDATNFRH PGGSIIKYLC
 51 TDGKEVVDAT EAYKEFHCRS SKAVKYLNSL PKIDGPIKYK YDAKEQARHD
101 KLTREYVALR EQLVKEGYFD PSPLHIIYRC AELAAMFALS FYLFSFKGNV
151 MATIAAIVIG GCVQGRCGWL MHEAGHYSMT GNIPVDLRLQ EFLYGIGCGM
201 SGAWWRSQHN KHHATPQKLK HDVDLDTLPL VAWNEKIARR VKPGSFQAKW
251 LHLQGYIFAP VSCLLVGLFW TLYLHPRHMI RTKRNFEIFS VALRYVCWFS
301 LLLSMGYTVG ESLGLYVLTF GLGCTYIFTH FAVSHTHLPV SEEDEYLHWV
351 EYAALHTTNV AIDSYVVTWL MSYLNFQIEH HLFPCCPQFR HPAISSRVKK
401 LFEDNGLVYD ARSYVQALKD TFGNLHEVGV NAGQAAKSE
```

Figure 10 (comparative)

```
   1  CCATGGGCCG CGGCGGCGAG AAAAGCGAGG TGGACCAGGT GCAGCCACAA
  51  AAGACCGAGC AGCTCCAGAA GGCCAAGTGG GAGGATGTTG TTCGCATCAA
 101  TGGAGTCGAA TACGACGTCA CGGACTATCT CAGAAAACAC CCTGGTGGCA
 151  GCGTGATCAA GTACGGGCTT GCCAACACCG GCGCTGATGC CACGTCCCTC
 201  TTTGAAGCGT TCCACATGCG CTCAAAGAAG GCTCAGATGG TGCTCAAGTC
 251  TCTCCCAAAG CGTGCTCCGG TCCTCGAGAT CCAGCCAAAC CAGCTTCCAG
 301  AGGAGCAGAC CAAGGAGGCG GAGATGCTGC GTGATTTTAA AAAATTTGAG
 351  GATGAGATTC GCCGGGATGG ATTGATGGAA CCTTCCTTCT GGCATCGCGC
 401  TTACAGATTA TCAGAGCTTG TAGGTATGTT CACGCTCGGC CTCTACCTCT
 451  TCTCGTTAAA CACTCCTCTG TCTATTGCTG CTGGTGTCCT CGTCCACGGT
 501  CTCTTTGGTG CATTCTGTGG ATGGTGCCAG CATGAGGCAG GCCACGGCTC
 551  CTTTTTTTAC AGCCTTTGGT GGGGCAAGCG TGTACAGGCC ATGTTGATCG
 601  GGTTTGGTCT AGGAACATCC GGCGACATGT GGAACATGAT GCACAACAAG
 651  CATCATGCTG CCACCCAAAA GGTTCATCAC GACCTTGACA TTGACACAAC
 701  TCCTTTTGTA GCTTTCTTCA ACACTGCATT TGAGAAAAAC AGATGGAAGG
 751  GCTTTTCCAA GGCTTGGGTC CGCTTTCAGG CTTTCACGTT CATTCCTGTC
 801  ACCAGCGGCA TGATCGTCAT GCTGTTCTGG CTGTTTTTTC TCCACCCTCG
 851  CCGCGTCGTT CAAAAGAAGA ACTTTGAGGA GGGTTTTTGG ATGCTGTCGA
 901  GCCACATTGT GCGCACCTAT CTCTTCCACC TTGTGACCGG CTGGGAGAGC
 951  CTCGCTGCAT GCTACCTTGT TGGGTATTGG GCGTGCATGT GGGTGTCCGG
1001  TATGTATTTG TTTGGCCACT TTTCGCTCTC CCACACTCAT ATGGACATTG
1051  TGGAGGCGGA CGTGCATAAG AACTGGGTCA GGTACGCTGT TGACCACACT
1101  GTTGACATCA GCCCATCCAA CCCGCTCGTG TGCTGGGTCA TGGGTTACCT
1151  CAACATGCAG ACCATCCACC ACTTGTGGCC TGCCATGCCC CAGTACCACC
1201  AGGTCGAGGT CTCACGCCGC TTTGCCATCT TCGCCAAAAA ACACGGCCTC
1251  AACTACCGCG TCGTCTCTTA CTTTGAGGCT TGGCGCCTGA TGCTCCAAAA
1301  TCTTGCTGAC GTCGGTTCCC ACTACCATGA GAACGGTGTC AAGCGCGCCC
1351  CAAAGAAAGC CAAGGCGCAG TAGAAAGCTA T
```

**Figure 11** (comparative)

```
  1 MGRGGEKSEV DQVQPQKTEQ LQKAKWEDVV RINGVEYDVT DYLRKHPGGS
 51 VIKYGLANTG ADATSLFEAF HMRSKKAQMV LKSLPKRAPV LEIQPNQLPE
101 EQTKEAEMLR DFKKFEDEIR RDGLMEPSFW HRAYRLSELV GMFTLGLYLF
151 SLNTPLSIAA GVLVHGLFGA FCGWCQHEAG HGSFFYSLWW GKRVQAMLIG
201 FGLGTSGDMW NMMHNKHHAA TQKVHHDLDI DTTPFVAFFN TAFEKNRWKG
251 FSKAWVRFQA FTFIPVTSGM IVMLFWLFFL HPRRVVQKKN FEEGFWMLSS
301 HIVRTYLFHL VTGWESLAAC YLVGYWACMW VSGMYLFGHF SLSHTHMDIV
351 EADVHKNWVR YAVDHTVDIS PSNPLVCWVM GYLNMQTIHH LWPAMPQYHQ
401 VEVSRRFAIF AKKHGLNYRV VSYFEAWRLM LQNLADVGSH YHENGVKRAP
451 KKAKAQ
```

**Figure 12** (comparative)

```
pRAT-2a    1  MGRGAQGEPRQATELKSSPSEQRKVLLIDGQLYDATNFRHPGGSI  45
pRAT-2c    1  MGRGAQGEPRQATELKSSPSEQRKVLLIDGQLYDATNFRHPGGSI  45

pRAT-2a   46  IKYLCTDGKEVVDATEAYKEFHCRSSKADKYLNSLPKIDGPIKYK  90
pRAT-2c   46  IKYLCTDGKEVVDATEAYKEFHCRSSKAVKYLNSLPKIDGPIKYK  90

pRAT-2a   91  YDAKEQARHDKLTREYVALREQLVKEGYFDPSPLHIIYRCAELAA  135
pRAT-2c   91  YDAKEQARHDKLTREYVALREQLVKEGYFDPSPLHIIYRCAELAA  135

pRAT-2a  136  MFALSFYLFSFKGNVVATIAAIVIGGCVQGRCGWLMHEAGHYSMT  180
pRAT-2c  136  MFALSFYLFSFKGNVMATIAAIVIGGCVQGRCGWLMHEAGHYSMT  180

pRAT-2a  181  GNIPVDLRLQEFLYGIGCGMSGAWWRRQHNKHHATPQKLKHDVDL  225
pRAT-2c  181  GNIPVDLRLQEFLYGIGCGMSGAWWRSQHNKHHATPQKLKHDVDL  225

pRAT-2a  226  DTLPLVAWNEKIARRVKPGSFQAKWPHLQGYIFAPVSCLLVGLFW  270
pRAT-2c  226  DTLPLVAWNEKIARRVKPGSFQAKWLHLQGYIFAPVSCLLVGLFW  270

pRAT-2a  271  TLYLHPRHMIRTKRNFEIFSVALRYVCWFSLLLSMGYTVGESLGL  315
pRAT-2c  271  TLYLHPRHMIRTKRNFEIFSVALRYVCWFSLLLSMGYTVGESLGL  315

pRAT-2a  316  YVLTFGLGCTYIFTHFAVSHTHLPVSEEDEYLHWVEYAALHTTNV  360
pRAT-2c  316  YVLTFGLGCTYIFTHFAVSHTHLPVSEEDEYLHWVEYAALHTTNV  360

pRAT-2a  361  AIDSYVVTWLMSYLNFQIEHHLFPCCPQFRHPAISSRVKKLFEDN  405
pRAT-2c  361  AIDSYVVTWLMSYLNFQIEHHLFPCCPQFRHPAISSRVKKLFEDN  405

pRAT-2a  406  GLVYDARSYVQALKDTFGNLHEVGVNAGQAAKSE            439
pRAT-2c  406  GLVYDARSYVQALKDTFGNLHEVGVNAGQAAKSE            439
```

**Figure 13** (comparative)

```
pRAT-1a    1  M G R G G E K S E V D Q V Q P Q K T E Q L Q K A K W E D V V R I N G V E Y D V T D Y L R  44
pRAT-1b    1  M G R G G E K S E V D Q V Q P Q K T E Q L Q K A K W E D V V R I N G V E Y D V T D Y L R  44

pRAT-1a   45  K H P G G S V I K Y G L A N T G A D A T S L F E A F H M R S K K A Q M V L K S L P K R A  88
pRAT-1b   45  K H P G G S V I K Y G L A N T G A D A T S L F E A F H M R S K K A Q M V L K S L P K R A  88

pRAT-1a   89  P V L E I Q P N Q L P E E Q T K E A E M L R D F K K F E D E I R R D G L M E P S F W H R  132
pRAT-1b   89  P V L E I Q P N Q L P E E Q T K E A E M L R D F K K F E D E I R R D G L M E P S F W H R  132

pRAT-1a  133  A Y R L S E L V G M F T L G L Y L F S L N T P L S I A A G V L V H G L F G A F C G W C Q  176
pRAT-1b  133  A Y R L S E L V G M F T L G L Y L F S L N T P L S I A A G V L V H G L F G A F C G W C Q  176

pRAT-1a  177  H E A G H G S F F Y S L W W G K R V Q A M L I G F G L G T S G D M W N M M H N K H H A A  220
pRAT-1b  177  H E A G H G S F F Y S L W W G K R V Q A M L I G F G L G T S G D M W N V M H N K H H A A  220

pRAT-1a  221  T Q K V H H D L D I D T T P F V A F F N T A F E K N R W K G F S K A W V R F Q A F T F I  264
pRAT-1b  221  T Q K V H H D L D I D T T P F V A F F N T A F E K N R W K G F S K A W V R F Q A F T F I  264

pRAT-1a  265  P V T S G M I V M L F W L F F L H P R R V V Q K K N F E E G F W M L S S H I V R T Y L F  308
pRAT-1b  265  P V T S G M I V M L F W L F F L H P R R V V Q K K N F E E G F W M L S S H I V R T Y L F  308

pRAT-1a  309  H L V T G W E S L A A C Y L V G Y W A C M W V S G M Y L F G H F S L S H T H M D I V E A  352
pRAT-1b  309  H L V T G W E S L A A C Y L V G Y W A C M W V S G M Y L F G H F S L S H T H M D I V E A  352

pRAT-1a  353  D V H K N W V R Y A V D H T V D I S P S N P L V C W V M G Y L N M Q T I H H L W P A M P  396
pRAT-1b  353  D V H K N W V R Y A V D H T V D I S P S N P L V C W V M G Y L N M Q T I H H L W P A M P  396

pRAT-1a  397  Q Y H Q V E V S R R F A I F A K K H G L N Y R V V S Y F E A W R L M L Q N L A D V G S H  440
pRAT-1b  397  Q Y H Q V E V S R R F A I F A K K H G L N Y R V V S Y F E A W R L M L Q N L A D V G S H  440

pRAT-1a  441  Y H E N G V K R A P K K A K A Q  456
pRAT-1b  441  Y H E N G V K R A P K K A K A Q  456
```

# Figure 14 (comparative)

| | | | | | |
|---|---|---|---|---|---|
| 1 | ATGGTGGCAG | GCAAATCAGG | CGCTGCGGCG | CACGTGACTC | ACAGCTCGAC |
| 51 | ATTGCCCCGT | GAGTACCATG | GCGCGACCAA | CGACTCGCGC | TCTGAGGCGG |
| 101 | CCGACGTCAC | CGTCTCTAGC | ATCGATGCTG | AAAAGGAGAT | GATCATCAAC |
| 151 | GGCCGCGTGT | ATGACGTGTC | GTCATTTGTG | AAGCGGCACC | CAGGTGGCTC |
| 201 | GGTGATCAAG | TTCCAGCTGG | GCGCCGACGC | GAGCGACGCG | TACAACAACT |
| 251 | TTCACGTCCG | CTCCAAGAAG | GCGGACAAGA | TGCTGTATTC | GCTCCCGTCC |
| 301 | CGGCCGGCCG | AGGCCGGCTA | CGCCCAGGAC | GACATCTCCC | GCGACTTTGA |
| 351 | GAAGCTGCGC | CTCGAGCTGA | AGGAGGAGGG | CTACTTCGAG | CCCAACCTGG |
| 401 | TGCACGTGAG | CTACAGGTGT | GTGGAGGTTC | TTGCCATGTA | CTGGGCTGGC |
| 451 | GTCCAGCTCA | TCTGGTCCGG | GTACTGGTTC | CTCGGCGCGA | TCGTGGCCGG |
| 501 | CATTGCGCAG | GGCCGCTGCG | GCTGGCTCCA | GCATGAGGGT | GGGCACTACT |
| 551 | CGCTCACCGG | CAACATCAAG | ATCGACCGGC | ATCTGCAGAT | GGCCATCTAT |
| 601 | GGGCTTGGCT | GCGGCATGTC | GGGCTGCTAC | TGGCGCAACC | AGCACAACAA |
| 651 | GCACCACGCC | ACGCCGCAGA | AGCTCGGGAC | CGACCCCGAC | CTGCAGACGA |
| 701 | TGCCGCTGGT | GGCCTTCCAC | AAGATCGTCG | GCGCCAAGGC | GCGAGGCAAG |
| 751 | GGCAAGGCGT | GGCTGGCGTG | GCAGGCGCCG | CTCTTCTTTG | GCGGGATCAT |
| 801 | CTGCTCGCTC | GTCTCTTTCG | GCTGGCAGTT | CGTGCTCCAC | CCCAACCACG |
| 851 | CGCTGCGCGT | GCACAATCAC | CTGGAGCTCG | CGTACATGGG | CCTGCGGTAC |
| 901 | GTGCTGTGGC | ACCTGGCCTT | TGGCCACCTC | GGGCTGCTGA | GCTCGCTCCG |
| 951 | CCTGTACGCC | TTTTACGTGG | CCGTGGGCGG | CACCTACATC | TTCACCAACT |
| 1001 | TCGCCGTCTC | GCACACCCAC | AAGGACGTCG | TCCCGCCCAC | CAAGCACATC |
| 1051 | TCGTGGGCAC | TCTACTCGGC | CAACCACACG | ACCAACTGCT | CCGACTCGCC |
| 1101 | CTTTGTCAAC | TGGTGGATGG | CCTACCTCAA | CTTCCAGATC | GAGCACCACC |
| 1151 | TCTTCCCGTC | GATGCCGCAG | TACAACCACC | CCAAGATCGC | CCCGCGGGTG |
| 1201 | CGCGCGCTCT | TCGAGAAGCA | CGGGGTCGAG | TATGACGTCC | GGCCATACCT |
| 1251 | GGAGTGTTTT | CGGGTCACGT | ACGTCAACCT | GCTCGCCGTA | GGCAACCCGG |
| 1301 | AGCACTCCTA | CCACGAGCAC | ACGCACTAG | | |

**Figure 15** (comparative)

```
  1  MVAGKSGAAA HVTHSSTLPR EYHGATNDSR SEAADVTVSS IDAEKEMIIN
 51  GRVYDVSSFV KRHPGGSVIK FQLGADASDA YNNFHVRSKK ADKMLYSLPS
101  RPAEAGYAQD DISRDFEKLR LELKEEGYFE PNLVHVSYRC VEVLAMYWAG
151  VQLIWSGYWF LGAIVAGIAQ GRCGWLQHEG GHYSLTGNIK IDRHLQMAIY
201  GLGCGMSGCY WRNQHNKHHA TPQKLGTDPD LQTMPLVAFH KIVGAKARGK
251  GKAWLAWQAP LFFGGIICSL VSFGWQFVLH PNHALRVHNH LELAYMGLRY
301  VLWHLAFGHL GLLSSLRLYA FYVAVGGTYI FTNFAVSHTH KDVVPPTKHI
351  SWALYSANHT TNCSDSPFVN WWMAYLNFQI EHHLFPSMPQ YNHPKIAPRV
401  RALFEKHGVE YDVRPYLECF RVTYVNLLAV GNPEHSYHEH TH
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9613591 A
- US 5552306 A
- WO 9411516 A
- US 5443974 A
- WO 9311245 A
- US 5912120 A
- US 5107065 A
- US 5231020 A
- US 4945050 A
- EP 50424 A
- EP 84796 A
- EP 258017 A
- EP 237362 A
- EP 201184 A
- US 4683202 A, Mullis
- US 4582788 A, Erlich
- US 4683194 A, Saiki
- US 5750176 A
- US 5700671 A
- US 5463174 A
- US 4943674 A
- US 5106739 A
- US 5175095 A
- US 5420034 A
- US 5188958 A
- US 5589379 A
- WO 9524494 A, Prieto
- US 5004863 A
- US 5159135 A
- US 5518908 A
- US 5569834 A
- US 5416011 A
- US 5631152 A
- US 5196198 A
- US 4826877 A
- US 4666701 A
- US 4758592 A
- US 5116871 A


### Non-patent literature cited in the description


- *The Faseb Journal,* A532
- *Experimental Biology,* 18 April 1998, vol. 98
- **Smith ; Waterman.** *Appl. Math.,* 1981, vol. 2, 482
- **Needleman ; Wunsch.** *J. Mol. Biol.,* 1970, vol. 48, 443
- **Pearson ; Lipman.** *Proc. Natl. Acad. Sci. (USA),* 1988, vol. 85, 2444
- **Higgins et al.** *CABIOS,* 1989, 5L151-153
- **Altschul et al.** *Nucleic Acids Research,* 1997, vol. 25, 3389-3402
- **Sambrook et al.** Molecular Cloning: A Laboratory Manual. Cold Spring Harbor Laboratory Press, 1989
- **Okamuro ; Goldberg.** *Biochemistry of Plants,* 1989, vol. 15, 1-82
- **Turner, R. ; Foster, G. D.** *Molecular Biotechnology,* 1995, vol. 3, 225
- **Ingelbrecht et al.** *Plant Cell,* 1989, vol. 1, 671-680
- **Klein et al.** *Nature (London),* 1996, vol. 327, 70-73
- **Ishida Y. et al.** *Nature Biotech.,* 1996, vol. 14, 745-750
- **Sambrook, J. ; Fritsch, E.F. ; Maniatis, T.** Molecular Cloning: A Laboratory Manual. Cold Spring Harbor Laboratory Press, 1989
- **Mullis et al.** *Cold Spring Harbor Symp. Quant. Biol.,* 1986, vol. 51, 263-273
- **Jones et al.** *EMBO J.,* 1985, vol. 4, 2411-2418
- **De Almeida et al.** *Mol. Gen. Genetics,* 1989, vol. 218, 78-86
- Molecular Cloning: A Laboratory Manual. Cold Spring Harbor Laboratory Press, 1989, vol. 1-3
- **Schnieke et al.** *Science,* 1997, vol. 278, 2130-2133
- **Weissbach ; Weissbach.** Methods for Plant Molecular Biology. Academic Press, Inc, 1988
- **McCabe.** *BiolTechnology,* 1988, vol. 6, 923
- **Christou et al.** *Plant Physiol,* 1988, vol. 87, 671-674
- **Cheng et al.** *Plant Cell Rep.,* 1996, vol. 15, 653-657
- **McKently et al.** *Plant Cell Rep.,* 1995, vol. 14, 699-703
- **Grant et al.** *Plant Cell Rep.,* 1995, vol. 15, 254-258
- **Bytebier et al.** *Proc. Natl. Acad. Sci. (USA),* 1987, vol. 84, 5354

- **Wan ; Lemaux.** *Plant Physiol,* 1994, vol. 104, 37
- **Rhodes et al.** *Science,* 1988, vol. 240, 204
- **Gordon-Kamm et al.** *Plant Cell,* 1990, vol. 2, 603-618
- **Fromm et al.** *BiolTechnology,* 1990, vol. 8, 833
- **Koziel et al.** *BiolTechnology,* 1993, vol. 11, 194
- **Armstrong et al.** *Crop Science,* 1995, vol. 35, 550-557
- **Somers et al.** *BiolTechnology,* 1992, vol. 10 (15), 89
- **Horn et al.** *Plant Cell Rep.,* 1988, vol. 7, 469
- **Toriyama et al.** *TheorAppl. Genet.,* 1986, vol. 205, 34
- **Part et al.** *Plant Mol. Biol.,* 1996, vol. 32, 1135-1148
- **Abedinia et al.** *Aust. J. Plant Physiol.,* 1997, vol. 24, 133-141
- **Zhang ; Wu.** *Theor. Appl. Genet.,* 1988, vol. 76, 835
- **Zhang et al.** *Plant Cell Rep.,* 1988, vol. 7, 379
- **Battraw ; Hall.** *Plant Sci.,* 1992, vol. 86, 191-202
- **Christou et al.** *Bio/Technology,* 1991, vol. 9, 957
- **De la Pena et al.** *Nature,* 1987, vol. 325, 274
- **Bower ; Birch.** *Plant J.,* 1992, vol. 2, 409
- **Wang et al.** *BiolTechnology,* 1992, vol. 10, 691
- **Vasil et al.** *Bio/Technology,* 1992, vol. 10, 667
- **Marcotte et al.** *Nature,* 1988, vol. 335, 454-457
- **Marcotte et al.** *Plant Cell,* 1989, vol. 1, 523-532
- **McCarty et al.** *Cell,* 1991, vol. 66, 895-905
- **Hattori et al.** *Genes Dev.,* 1992, vol. 6, 609-618
- **Goff et al.** *EMBO J.,* 1990, vol. 9, 2517-2522
- **Maliga et al.** Methods in Plant Molecular Biology. Cold Spring Harbor Press, 1995
- **Sambrook et al.** Molecular Cloning: A Laboratory Manual. Cold Spring Harbor Press, 1989
- **Birren et al.** Genome Analysis: Detecting Genes. Cold Spring Harbor, 1998
- **Birren et al.** Genome Analysis: Analyzing DNA. Cold Spring Harbor, 1998
- Plant Molecular Biology: A Laboratory Manual. Springer, 1997
- **Horrobin et al.** *Am. J. Clin. Nutr.,* vol. 57, 732S-737S
- **Brenner et al.** *Adv. Exp. Med. Biol.,* 1976, vol. 83, 85-101
- **Hoge et al.** *Exp. Mycology,* 1982, vol. 6, 225-232
- **Okuley et al.** *The Plant Cell,* 1994, vol. 6, 147-158